# EUROPEAN PATENT SPECIFICATION

(11) **EP 1 874 818 B1**
(45) Date of publication and mention of the grant of the patent: **13.04.2011**
(21) Application number: 06750860.6
(22) Date of filing: 20.04.2006
(51) Int. Cl.: C07K 16/22, G01N 33/68, G01N 33/577

(54) **TGF BETA 1 SPECIFIC ANTIBODIES**
TGF-BETA-1-SPEZIFISCHE ANTIKÖRPER
ANTICORPS SPÉCIFIQUES DE TGF BETA 1

(30) Priority: 22.04.2005 US 674082 P
(43) Date of publication of application: 09.01.2008
(73) Proprietor: ELI LILLY AND COMPANY, Indianapolis, IN 46285 (US)
(72) Inventor: DAVIES, Julian, San Diego, California 92109 (US); DICKINSON, Craig, Duane, San Diego, California 92129 (US); HUANG, Lihua, Carmel, Indiana 46032 (US); JONES, Bryan, Edward, Carmel, Indiana 46032 (US); MARQUIS, David, Matthew, Encinitas, California 92024 (US); ROWLINSON, Scott, William, Zionsville, Indiana 46077 (US); TANG, Ying, San Diego, California 92129 (US); VAILLANCOURT, Peter, Edward, Del Mar, California 92014 (US); WATKINS, Jeffry, Dean, Encinitas, California 92024 (US)
(74) Representative: Ingham, Stephen H.
(86) International application number: PCT/US2006/014943
(87) International publication number: WO 2006/116002

(56) References cited:
- WO-A-00/66631
- WO-A2-2005/010049
- "Monoclonal anti-TGF-b1 antibody" R&D SYSTEM ORDERING INFORMATION, no. mab2401, 29 January 2003 (2003-01-29), XP002313281
- LUCAS C ET AL: "THE AUTOCRINE PRODUCTION OF TRANSFORMING GROWTH FACTOR-BETA1 DURING LUMPHOCYTE ACTIVATION A STUDY WITH A MONOCLONAL ANTIBODY-BASED ELISA" JOURNAL OF IMMUNOLOGY, THE WILLIAMS AND WILKINS CO. BALTIMORE, US, vol. 145, 1 September 1990 (1990-09-01), pages 1415-1422, XP000917385 ISSN: 0022-1767
- SHEHATA MEDHAT ET AL: "TGF-beta1 induces bone marrow reticulin fibrosis in hairy cell leukemia." JOURNAL OF CLINICAL INVESTIGATION, vol. 113, no. 5, March 2004 (2004-03), pages 676-685, XP002406208 ISSN: 0021-9738
- FLANDERS K C ET AL: "ANTIBODIES TO PEPTIDE DETERMINANTS IN TRANSFORMING GROWTH FACTOR BETA AND THEIR APPLICATIONS" BIOCHEMISTRY, AMERICAN CHEMICAL SOCIETY. EASTON, PA, US, vol. 27, 1988, pages 739-746, XP002044723 ISSN: 0006-2960
- CHENG JINGFEI ET AL: "Transforming growth factor-beta signal transduction and progressive renal disease." EXPERIMENTAL BIOLOGY AND MEDICINE (MAYWOOD), vol. 227, no. 11, December 2002 (2002-12), pages 943-956, XP002406209 ISSN: 1535-3702

## Description

### FIELD OF THE INVENTION

The invention generally relates to compositions related to TGF Beta 1 proteins. In particular, it provides purified binding compositions, and related reagents useful, e.g., in the diagnosis, treatment, and prevention of cell proliferative, autoimmune/inflammatory, cardiovascular, and fibrotic disorders, and in the assessment of the effects of exogenous compounds on the expression of nucleic acid and amino acid sequences of such proteins.

### BACKGROUND OF THE INVENTION

The first member of the transforming growth factor-beta (TGF-Beta) superfamily of secreted polypeptide factors, TGF-Beta 1, was discovered approximately twenty years ago. Subsequently, the family has grown considerably and now comprises over thirty vertebrate members and a dozen or so structurally and functionally related proteins in invertebrates such as worms and flies (see, e.g., Attisano & Lee-Hoeflich, 2001 Gen. Biol. 2, review 30101; Moustakas, et al., 2001 J. Cell Sci. 114:4359; Wrana, 2000 Cell 100:189). Members of the TGF Beta family control many cellular functions, and their activity is critical for regulating numerous developmental and homeostatic processes. Experiments have revealed a conserved TGF-Beta signaling pathway exists among vertebrates, wormy, and flies.

One member of this family, TGF Beta 1, is involved in a variety of cellular processes such as, for example, cell proliferation and differentiation, migration, differentiation, apoptosis, embryonic development, extracellular matrix formation, bone development, wound healing, hematopoiesis, and immune and inflammatory responses (see, e.g., Roberts, & Spom 1990 Peptide Growth Factors and Their Receptors, pp. 419-72, Springer-Verlag, Heidelberg, Germany; Massagué, J. 1998 Annu. Rev. Biochem 67:753).

Additionally, preclinical and clinical data indicate that TGF-Beta 1 is a major contributor to matrix protein deposition in interstitial fibrosis, and is involved in the initiation and progression of a number of associated fibrotic disease states, including renal fibrosis - which is common to all forms of chronic renal disease (CRD). The extent of renal fibrosis positively correlates with progression to chronic renal failure (CRF, also known as end-stage renal disease (ESRD)), and can result in death, chronic dialysis, or renal transplantation.

TGF Beta is associated with CRF through complex and diverse events that impact the majority of cells of the kidney (Bottinger, 2002, J. Am. Soc. Nephrol. 13:2600). These events ultimately result in both tubulointerstitial fibrosis and glomerulosclerosis leading to loss of nephron function and ultimately chronic renal failure. Of the three TGF-Beta isoforms, TGF-Beta 1 appears to predominant in mediating the progression of chronic renal disease, not only as being the most predominantly expressed isoform, but also as both TGF-Beta 2 and -Beta 3 appear to mediate their effects through up-regulation of TGF-Beta 1 expression (Yu, 2003, Kid. Int. 64, 844). Consequently, to prevent the deleterious effects of disorders such as CRD, there is a need to modulate TGF Beta 1 expression.

International Publication No. WO2005/010049 discloses monodonal antibodies specific for TGF-Beta 1 are TGF-Beta 2 or TGF-Beta 3, and which neutralize TGF-Beta 1.

Accordingly, discovery of new TGF Beta 1 binding compositions satisfies a need in the art by providing compositions that are useful in the diagnosis, prevention, and treatment of fibrotic disorders such as chronic renal disease.

### SUMMARY OF THE INVENTION

The invention is based in part upon the discovery of binding compositions with improved properties that specifically and/or selectively bind mature TGF Beta 1 over mature TGF Beta 2 and/or mature TGF Beta 3 and which neutralize mature TGF Beta 1.

The present invention provides an antibody or an antigen-binding fragment thereof or an antigen-binding fragment thereof according to claim 1.

The present invention further provides a pharmaceutical composition comprising an antibody or an antigen-binding fragment thereof of the invention and a pharmaceutically acceptable carrier.

The present invention also provides an antibody or an antigen-binding fragment thereof of the invention for use in the treatment of a fibrotic disease in a human subject.

The present invention additionally provides an antibody or an antigen-binding fragment thereof of the invention for use in the treatment of a chronic renal disease in a human subject.

The present invention additionally provides an antibody or an antigen-binding fragment thereof of the invention for use in combination therapy for treating a fibrotic disease in a human subject, wherein said antibody or antigen-binding fragment thereof is administered in combination with an ACE inhibitor.

The present invention additionally provides an antibody or an antigen-binding fragment thereof of the invention for use in combination therapy for treating a chronic renal disease in a human subject, wherein said antibody or antigen-binding fragment thereof is to be administered in combination with an ACE inhibitor.

The present invention additionally provides an antibody or an antigen-binding fragment thereof of the invention for use in the treatment of cancer.

### DETAINED DESCRIPTION OF THE PREFERRED EMBODIMENTS

### I. General

It is to be understood that this invention is not limited to the particular compositions, methods, and techniques described herein, as such compositions, methods, and techniques may, of course, vary. It is also to be understood that the terminology used herein is to describe particular embodiments only, and is not intended to limit the scope of the invention, which is only limited by the appended claims.

As used herein, including the appended claims, singular forms of words such as "a," "an," and "the" include, e.g., their corresponding plural referents unless the context clearly dictates otherwise. Thus, for example, reference to "an organism" includes, e.g., one or more different organisms, reference to "a cell" includes, e.g., one or more of such cells, and reference to "a method" include, e.g., reference to equivalent steps and methods known to a person of ordinary skill in the art, and so forth.

Unless otherwise defined, all technical and scientific terms used herein have the same meaning as commonly understood by a person of ordinary skill in the art to which this invention belongs. Although methods and materials similar or equivalent to those described herein can be used to practice or test the invention, suitable methods, and materials are described below. All publications, patent applications, patents, and other references discussed herein are provided solely for their disclosure before the filing date of the present application. Nothing herein is to be construed as an admission that the invention is not entitled to antedate any such disclosure by virtue of its prior invention. All publications, patent applications, patents, and other references mentioned herein are incorporated by reference in their entirety for the teachings for which they are cited (as the context clearly dictates), including all figures, drawings, pictures, graphs, hyperlinks, and other form of browser-executable code.

### II. Definitions

A **"binding composition"** is a molecular entity with selective and/or specific binding affinity for at least one other molecular entity or binding partner. Typically, the association will be in a naturally physiologically relevant interaction, either covalently or non-covalent, and may include members of a multi-protein complex, including, without limitation, carrier compounds, dimerization or multimerization partners. A binding composition can be naturally derived (e.g., isolated and/or purified) or synthetically produced, either in whole or in part. Typically, a binding composition has at least one region such as, by way of non-limiting example, a surface area, a shape (such as, e.g., a cavity, cleft, crevice, or protrusion), a molecular arrangement, or a spatial configuration, that specifically and/or selectively "fits with," "binds to," or is "complementary with" a particular spatial and/or polar organization of an area or region on a binding partner. Thus, for example, when a binding composition is sufficiently proximate to a potential binding partner, the binding composition and partner will specifically and/or selectively bind each other. Non-limiting examples of a binding composition paired with a binding partner include: antigen-antibody, and hapten-binding site. Non-limiting examples of antibody binding compositions include: antibodies, diabodies, triabodies, tetrabodies, minibodies, Fab fragments (including, such as, e.g., dimeric or trimeric Fabs), Fv fragments, scFv fragments, F(ab)2 fragments, etc. (see, e.g., Hudson & Souriau 2003 Nature Medicine 9:129-34 for non-limiting examples of antibody binding compositions encompassed by the invention). A monoclonal antibody binding composition is monoclonal in the sense that it is derived from a population of substantially homogeneous antibodies (i.e., the individual antibodies comprising the population are substantially identical (e.g., they may be derived from a clone of a single cell type)). However, this is not meant to limit it to a particular origin. Such an antibody may readily be produced in cells that commonly do not produce antibodies, such as CHO, NSO, or COS cells. In addition, such an antibody may be produced in other types of cells, especially mammalian and even plant cells, by genetically engineering such cells to express and assemble the polypeptide light and heavy chains forming the antibody product. In addition, such chains can be chemically synthesized but, since they would be specific for a given antigenic determinant, would still constitute a "monoclonal" antibody within the spirit in which that term is used here. Thus, as used herein, the term monoclonal antibody is intended to denote more the specificity and purity of the antibody molecules rather than the mere mechanism used for production of said antibodies.

A **"binding site"** is a specific region, area, or configuration of a molecular entity that takes part in the specific and/or selective binding with another molecular entity. A non-limiting example of a binding site is the contiguous amino acid sequence comprising a complementary determining region (CDR) of an antibody. In one embodiment, a binding site of the invention comprises sequence having the formula shown in Table 1. In another non-limiting embodiment, a binding site comprises a combination of the sequences of Table 1. Another non-limiting example is a binding site formed from the three-dimensional configuration and spatial organization of the amino acid sequences comprising the six CDR loops of the heavy and light variable chains at the rim of the eight-stranded beta barrel of a Fab fragment (see, e.g., Chothia & Lesk, 1987 J. Mol. Biol. 196:901-17). A disclosed CDR of the invention can be incorporated/embedded with a framework or molecular structure such as, for example, as done in CDR grafting. In one embodiment, the structure for carrying a CDR of the invention will generally be of an antibody heavy or light chain sequence or substantial portion thereof in which the CDR is located at a location corresponding to the CDR of naturally occurring VH and VL antibody variable domains encoded by rearranged immunoglobulin genes. The structures and locations of immunoglobulin variable domains may be determined by reference to (Kabat, et al, 1987 Sequences of Proteins of Immunological Interest. 4th Ed. US Department of Health and Human Services, and updates thereof, now available on the Internet (http://immuno.b-me.nwu.edu)). CDRs are generally as defined by Kabat but may also be informed by the definitions of Chothia (J. Mol. Biol. 196:901-17) and/or MacCallum (J. Mol. Biol. 262:732-45). Those skilled in the art can routinuely determine which residues comprise a particular CDR given the variable region amino acid sequence within which it is embedded. Additionally, in CDR grafting residues of the loop defined by Chothia adjacent the Kabat VH CDR1 may be grafted.

In one embodiment, CDR sequences of the invention may be introduced into a repertoire of variable domains lacking CDR regions, using recombinant DNA technology. For example, Marks et al (1992 BioTechnology 10:779-83) describe methods of producing repertoires of antibody variable domains in which consensus primers directed at or adjacent to the 5' end of the variable domain area are used in conjunction with consensus primers to the third framework region of human VH genes to provide a repertoire of VH variable domains lacking a CDR3. Marks et al further describe how this repertoire may be combined with a CDR3 of a particular antibody (such as described herein). Using analogous techniques, the CDR3 sequences of the present invention may be shuffled with repertoires of VH or VL domains lacking a CDR3, and the shuffled complete VH or VL domains combined with a cognate VL or VH domain to provide specific binding composition members of the invention. Alternatively, the CDR3 sequences can be combined with other CDRs of the invention using a similar strategy. The repertoire may then be displayed in a suitable host system such as the phage display system of W092/01047 so that suitable specific binding members may be selected.

### Binding Composition:TGF Beta 1 Complex

The term **"binding composition:TGF Beta 1 complex**", as used herein, refers to a complex of a binding composition and TGF Beta 1 protein formed by specific and/or selective binding of the binding composition to a TGF Beta 1 protein. In a preferred embodiment, the TGF Beta 1 referred to throughout is a "mature" primate TGF Beta 1 protein. In a more preferred embodiment, the TGF Beta 1 referred to throughout is a mature human TGF Beta 1 protein (see, e.g., NCBI accession No: P01137, which describes the amino acid sequence of the human transforming growth factor beta 1 precursor (TGF-beta 1) and its mature portion). In each instance, the TGF Beta 1 referred to throughout is TGF Beta 1 in its mature, biologically active form [(TGF Beta 1 is released from cells as an inactive 'latent' complex, comprising a TGF Beta 1 homodimer in a non-covalent complex with two prosegments, to which one of several TGF latent Beta 1 binding proteins is often linked (see, e.g., Annes, et al., 2003 J. Cell Science 116:217-24; Miyazono, et al., 1993 Growth Factors 8:11-22; Munger, et al. 1997 Kidney Int. 51:1376-82; Oklu & Hesketh 2000 Biochem. J. 352:601-10). This latent TGF Beta 1 complex represents an important safeguard against 'inadvertent' activation, and may stabilize and target latent TGF beta 1 to the extracellular matrix, where it is sequestered (Taipale, et al. 1998 Adv. Cancer Res. 75:87-134). The extracellular matrix thus acts as a reservoir from which TGF-Beta 1 can readily be activated without the need for new cell synthesis. The secretion of TGF Beta 1 as a latent complex necessitates the existence of a regulated activation process, which is most probably mediated through the activities of proteases that preferentially degrade the TGF Beta 1 prosegments and thereby release the highly stable, mature, active TGF-Beta 1 dimer form.)].

Specific binding of the binding composition means that the binding composition has a binding site that recognizes a region of TGF Beta 1, typically in its native active conformation. For example, antibodies raised to a TGF Beta 1 and recognizing an epitope of TGF Beta 1 are capable of forming a binding composition:TGF Beta 1 complex by specific binding. Typically, the formation of a binding composition:TGF Beta 1 protein complex allows the measurement of TGF Beta 1 in a biological sample, e.g., a mixture with other proteins and biologics. An epitope of a binding composition of the invention can be determined using techniques described herein or in the art (see, e.g., G. Tribbick 2002 Journal of Immunological Methods 267:27-35; Woods & Hamuro 2001 Journal of Cellular Biochemistry Supplement 37:89-98) and/or as determined by competitive binding as described herein. In a preferred embodiment, an epitope of a binding composition of the invention comprises the amino acid residues YYVGRK of SEQ ID NO: 1; in another embodiment; an epitope of a binding compositions of the invention comprises the amino acid residues YYVGRK of SEQ ID NO: 1 and YSKV of SEQ ID NO: 1; in an additional embodiment, an epitope of a binding composition of the invention comprises at least 1, 2, 3, 4, 5, or 6 residues (continguous or non-contiguous) from YYVGRK of SEQ ID NO: 1 and/or at least 1, 2, 3, or 4 residues (continguous or non-contiguous) from YSKV of SEQ ID NO:1 (such an embodiment is encompassed to include any and all combinations thereof such as, e.g. without limitation: YYVGRK and KV of SEQ ID NO:1; or YVGRK and Y and KV of SEQ ID NO:1 (all such combinations are available merely by using a computer algorithm and well known mathematical formulas for permutaions and combinations). In a still further preferred embodiment, an epitope of the invention is defined functionally, for example, by the ability of a binding composition of the invention to prevent formation of a subsequent binding complex by competing binding compositions for the same antigen such as, e.g., TGF Beta 1 (such competitive binding is described herein).

The term **"specific binding"** as used herein refers to the situation in which one member of a specific binding pair will not show significant binding to molecules other than its specific binding partner(s). The term is also applicable where e.g. an antigen-binding domain is specific for a particular epitope that is carried by a number of antigens, in which case the specific binding member carrying the antigen-binding domain will be able to bind to the various antigens carrying the epitope. Accordingly, a binding composition specific for mature TGF Beta 1 "will not show any significant binding to molecules other than its specific binding partner(s)" that is, mature TGF Beta 1.

The phrases **"specifically binds"** or **"specifically immunoreactive with",** with respect to a binding composition, refers to a binding reaction that is determinative of the presence of the binding composition in the presence of a heterogeneous population of proteins and other biological components. Thus, under designated immunoassay conditions, the specified binding composition binds a specific protein and does not significantly bind other proteins present in the sample. Specific binding to a binding composition under such conditions may require a binding composition that is selected for its specificity for a particular protein. For example, a binding composition, such as an antibody, can be raised to the human active dimer form of the TGF Beta 1, whose mature amino acid sequence monomer depicted in [SEQ ID NO:1 and subsequently selected to obtain antibodies specifically immunoreactive with that TGF Beta 1 protein and not with other proteins. Such a binding composition could differentiate proteins highly homologous to the human TGF Beta 1 protein, e.g., such as, for example, other human TGF Beta isoforms (e.g., such as, human TGF Beta 2, or human TGF Beta 3). In a preferred embodiment, the specificity of a binding composition of the invention for mature TGF Beta 1 is equal to or greater than 1.5-, 2.0-, 2.5-, 3.0-, 3.5-, 4.0-, 4.5-, 5.0-, 5.5-, 6.0-, 7.0-, 7.5-, 8.0-, 8.5-, 9.0-, 9.5-, 10-, 15-, 20-, 30-, 40-, 50-, 60-, 70-, 80-, 85-, 90-, 95-, 100-, 125-, 150-, 200-, 300-, 400-, 500-, 600-, 700-, 800-900-, 1000-, 1500-, 2000-, 2500-, 3000-, 3500, 4000-, 4500-, 5000-, 5500-, 6000-, 6500-, 7000-, 7500-, 8000-8500-, 9000-, 9500-, or 10,000-fold relative to its specificity for mature TGF Beta 2 and/or mature TGF Beta 3. In another preferred embodiment, the specificity of an invention composition can have a particular specificity value for human TGF Beta 2 from the above list and a different specificity value for human TGF Beta 3 such as, for example, a specificity for mature TGF Beta 1 that is equal or greater than 100-fold in its specifity relative to mature TGF Beta 2 and equal or greater than 900-fold in its specifity relative to mature TGF Beta 3. Any such combinations are encompassed.

A binding composition of the invention preferably neutralizes TGF Beta 1 and has a dissociation constant (Kd) for TGF Beta 1 preferably of less than about: 40pM, 35pM, 30pM, 25pM, 20pM, 15pM, 14pM, 13pM, 12pM, 11pM, or 10pM; more preferably less than about: 10pM, 9.9pM, 9.8pM, 9.7pM, 9.6pM, 9.5pM, 9.4pM, 9.3pM, 9.2pM, 9.1pM 9.0pM, 8.9pM, 8.8pM, 8.7pM, 8.6pM, 8.5pM, 8.4pM, 8.3pM, 8.2pM, 8.1pM 8.0pM, 7.9pM, 7.8pM, 7.7pM, 7.6pM, 7.5pM, 7.4pM, 7.3pM, 7.2pM, 7.1pM 7.0pM, 6.9pM, 6.8pM, 6.7pM, 6.6pM, 6.5pM, 6.4pM, 6.3pM, 6.2pM, 6.1pM, 6.0pM, 5.9pM, 5.8pM, 5.7pM, 5.6pM, 5.5pM, 5.4pM, 5.3pM, 5.2pM, 5.1pM or 5.0pM; even more preferably of less than about 5.0pM, 4.9pM, 4.8pM, 4.7pM, 4.6pM, 4.5pM, 4.4pM, 4.3pM, 4.2pM, 4.1pM 4.0pM, 3.9pM, 3.8pM, 3.7pM, 3.6pM, 3.5pM, 3.4pM, 3.3pM, 3.2pM, 3.1pM 3.0pM 2.9pM, 2.8pM, 2.7pM, 2.6pM, 2.5pM, 2:4pM, 2.3pM, 2.2pM, 2.1pM 2.0pM, 1.9pM, 1.8pom, 1.7pM, 1.6pM, 1.5pM, 1.4pM, 1.3pM, 1.2pM, 1.1pM, 1.0pM; and even far more preferably 9.9pM, 9.8pM, 9.7pM, 9.6pM, 9.5pM, 9.4pM, 9.3pM, 9.2pM, 9.1pM 9.0pM, 8.9pM, 8.8pM, 8.7pM, 8.6pM, 8.5pM, 8.4pM, 8.3pM, 8.2pM, 8.1pM 8.0pM, 7.9pM, 7.8pM, 7.7pM, 7.6pM, 7.5pM, 7.4pM, 7.3pM, 7.2pM, 7.1pM 7.0pM, 6.9pM, 6.8pM, 6.7pM, 6.6pM, 6.5pM, 6.4pM, 6.3pM, 6.2pM, 6.1pM, 6.0pM, 5.9pM, 5.8pM, 5.7pM, 5.6pM, 5.5pM, 5.4pM, 5.3pM, 5.2pM, 5.1pM, 5.0pM, 5.0nM, 4.9pM, 4.8pM, 4.7pM, 4.6pM, 4.5pM, 4.4pM, 4.3pM, 4.2pM, 4.1 pM 4.0pM, 3.9pM, 3.8pM, 3.7pM, 3.6pM, 3.5pM, 3.4pM, 3.3pM, 3.2pM, 3.1pM 3.0pM 2.9pM, 2.8pM, 2.7pM, 2.6pM, 2.5pM, 2.4pM, 2.3pM, 2.2pM, 2.1pM 2.0pM, 1.9pM, 1.8pM, 1.7pM, 1.6pM, 1.5pM, 1.4pM, 1.3pM, 1.2pM, 1.1pM, 1.0pM, 0.9pM, 0.8pM, 0.7pM, 0.6pM, 0.5pM, 0.4pM, 0.3pM, 0.2pM, 0.1pM, or 0.01pM. The dissociation constant (Kd) of a binding composition can be determined using any art method, for example, by BIACore™, adapting the method of Karlsson et al., 1991 J. Immunol. Methods 145, 299-340. For other descriptions, see Jonsson, et al. 1993 Ann. Biol. Clin. 51:19-26; Jonsson, et al. 1991 Biotechniques 11:620-7; Johnsson, et al. 1995 J. Mol. Recognit. 8:125-31; and Johnnson, et al. 1991 Anal. Biochem. 198:268-77.

The term "kₒₙ", as used herein is intended to refer to the association or on rate constant, or specific reaction rate, of the forward, or complex-forming, reaction, measured in units: M-1sec-1. The term "k_{off}", as used herein, is intended to refer to the dissociation or off rate constant, or specific reaction rate, for dissociation of an antibody from the antibody/antigen complex, measured in units: 1/second. The term "Kd," as used herein, is intended to refer to the dissociation constant of a particular antibody-antigen interaction. It is calculated by the formula: k_{off}/kₒₙ = Kd. The affinity of a binding composition is often correlated with a lower k_{off} more so than a higher kₒₙ, however, not being bound by theory, both improved k_{off} and kₒₙ, embodiments are encompassed. In a more preferred embodiment, binding compositions of the present invention are high potency antibodies or fragments thereof, generally exhibiting low k_{off} values. In even a more preferable embodiment, the kₒₙ rate of a Fab embodiment of the invention has at least a 1.5-, 1.6-, 1.7-, 1.8-, 1.9-, 2.0-, 2.1-, 2.2-, 2.3-, or 2.4-fold improved kₒₙ rate than a comparable Fab such as, e.g., the mAb2471 described in International Publication No. W02005/010049. In another embodiment, a composition of the invention has an improved k_{off} rate that is at least 10, 20, 30, 40, 50, 60, 70, 80, 90, 100, 125, 130, 150, 195, 200, 225, 250, 260, 270, 280, 290, or 300 times an improvement over a comparable binding composition (e.g., such as a Fab or mAb).

Preferably, an antibody binding composition specifically and/or selectively binds TGF Beta 1 as compared to TGF Beta 2 and/or TGF Beta 3; more preferably, an antibody binding composition specifically and/or selectively binds human TGF Beta 1 as compared to human TGF Beta 2 and/or human TGF Beta 3. Preferably, such an antibody has less than about 20% cross-reactivity with TGF Beta 2 and/or TGF Beta 3 (as measured by the ratio of the dissociation constants), more preferably less than about 15% cross-reactivity, and even more preferably has less than about 10% cross-reactivity; and even more preferably less than about 9, 8, 7, 6, 5, 4, 3, 2, or 1% cross-reactivity.

Further, an antibody binding composition preferably recognizes the active but not the latent form of TGF Beta 1; more preferably the active, but not the latent form of human TGF Beta 1.

### Neutralization

The term **"neutralize"** or **"antagonize"** with respect to a binding composition refers to the situation in which the specific and/or selective binding of a binding composition to another molecular entity results in the abrogation or inhibition of the biological effector function of the molecular entity bound by the binding composition. With respect to TGF Beta 1, the term "neutralize" or "antagonize" is intended to refer to a binding composition whose binding to, or interaction with, TGF Beta 1 results in inhibition of a biological activity induced by TGF Beta 1. Inhibition of TGF Beta 1 biological activity can be assessed by measuring one or more *in vitro* or *in vivo* indicators of TGF Beta 1 biological activity including for example, without limit, inhibition of receptor binding, inhibition of fibrosis, inhibition of chemotaxis, or inhibition of signal transduction in a TGF Beta 1 binding assay (see, e.g., EP 0 945 464 for non-limiting examples of encompassed neutralization assays). In a non-limiting embodiment, the ability of a binding composition to neutralize or antagonize TGF Beta 1 activity is assessed by use of the assay as described in an Example herein.

The **neutralizing activity** of a binding composition, such as, for example, an antibody embodiment can be tested by art-accepted method. In a non-limiting example, testing is carried out by adopting/modifying the TGF assay of Randall et al (1993) J. Immunol Methods 164, 61-67. This assay is based on the ability of TGF Beta 1 and TGF Beta 2 to inhibit the interleukin-5 (IL-5) induced proliferation of the erythroleukaemia cell line TF1 and on the ability to reverse the TGF Beta inhibition with TGF Beta specific binding compositions. The assay is reported to be rapid, reproducible, and sensitive to less than 500 fg/ml of TGF-beta 1, and 5-10 pg/ml TGF-beta 2. The assay is also reported to be 100-1000-fold less sensitive to other inhibitory molecules such as interferon-beta, interferon-gamma, and TNF-alpha. The assay is also reported to be capable of being made specific for TGF-beta 1 or TGF-beta 2 by including specific neutralizing antibodies for TGF-beta 1 or TGF-beta 2 and to recognize all the readily available recombinant molecular species of these molecules as well as the natural proteins produced from human and bovine platelets and to detect TGF-beta in serum samples.

Other assays, reported herein or art-known, are also encompassed. For example, the rat anti-Thy1.1 model is a well-established model of mesangioproliferative glomerulonephritis (see, e.g., Morita, et al., 1998 Am J Kidney Dis 31:559-73; Bagchus, et al., 1986 Lab. Invest. 55:680-7 and Yamamoto & Wilson 1987 Kidney Int. 32:514-25) in which injection of an antibody directed against the Thy antigen located on the surface of mesangial cells induces mesangiolysis followed by a phase of over compensatory proliferation of mesangial cells resulting in elevated levels of urinary protein (proteinuria). The anti-Thy1.1 nephritis model resembles human IgA nephritis or Henoch-Schonlein purpura in many aspects (O'Donoghue, et al., 1991 J Clin Invest 88:1522-30) and it has been used to test potentially therapeutic approaches to kidney disease by determining the ability of test compositions to effect dose-related decreases in proteinuria (see, e.g., Burg, et al., 1997 Lab Invest 76:505-16; Johnson, et al., 1995 Kidney Int 47:62-9). In a preferred embodiment, a binding composition of the invention decreases proteinuria in such a model by greater than or equal to 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, or 80%. Further encompassed are embodiments having a range of decreased proteinuria (in such a model) between any two such indicated values, which range may or may not include either or both end-point numeric values (e.g., a range >12% and ≤42%).

Additionally, one can adopt either the db/db diabetic mouse model assay of Sharma (see, e.g., 2000 PNAS 97:8015-20), demonstrating that chronic inhibition of the biologic actions of TGF-beta in the kidney effectively prevents renal failure resulting from diabetes u.c. or, one can adopt or the Sharma STZ diabetic mouse model (1996 Diabetes 45, 522-30) to test a binding composition's ability to ameliorate or prevent diabetic nephropathy. Further, Ueberham et al., 2003 Hepatology 37(5):1067-78, describe a tetracycline-regulated gene expression system in a double-transgenic mouse model of liver fibrosis where the expression of TGF-Beta 1 is regulated by the addition or removal of doxycycline hydrochloride to drinking water thus permitting switching TGF-Beta 1 expression on or off at will. Increasing TGF-Beta 1 expression in the liver of such animals leads to fibrotic disease states that are reversible by switching off TGF Beta 1 expression - even after liver mass has been reduced 59%. Use of this model permits one to evaluate the effects of binding compositions of the invention on inhibiting TGF Beta 1 biological functions by comparing a binding composition of the invention with the effects of doxycycline hydrochloride switching off TGF Beta 1 expression. In a particular embodiment using an HT-2 cell proliferation assay (as described herein) applicants preferably encompass binding composition embodiments with an IC₅₀ ≥ at least a: 50-, 60-, 70-, 80-, 90-, 100-, 105-, 125-, 150-, 175-, 200-, 225-, 250-, 275-, 300-, 325-, 350-, 375-, 400-, 425-, or 450-fold improvement compared to an IC₅₀ value, obtained using a similar binding composition, for example, such as mAb2471 (described in International Publication No. WO2005/010049) in the same or under similar assay conditions. Further encompassed are embodiments having a range of IC₅₀ improvement (as defined above) of two such values that may or may not include either or both end points (e.g., an IC₅₀ improvement in the range of >80 and ≤ 100-fold improvement over, e.g., mAb2471).

### Antibodies

**Antibody binding compositions** of the invention include, e.g., without limitation, polyclonal, monoclonal, multispecific, human, humanized, or chimeric antibodies, single chain antibodies, Fab fragments, F(ab') fragments, fragments produced by a Fab expression library, anti-idiotypic (anti-Id) antibodies (including, e.g., anti-Id antibodies to antibodies of the invention), and an epitope-binding fragment of any of the above.

The term **"antibody"** as used herein, refers to immunoglobulin compositions and immunologically active portions of immunoglobulin compositions, e.g., a binding composition molecule that contains a binding site that specifically and/or selectively binds an antigen. An immunoglobulin composition of the invention can be of any type (e.g., IgG, IgE, IgM, IgD, IgA, and IgA), class (e.g., IgG1, IgG2, IgG3, IgG4, IgA1, and IgA2) or a subclass of an immunoglobulin molecule. Preferably an antibody is a human antigen-binding antibody fragment of the invention such as, e.g., without limitation, Fab, Fab' and F(ab')2, Fc, single-chain Fvs (scFv), single-chain antibodies, disulfide-linked Fvs (sdFv), and fragments comprising either a VL or VH domain. Antigen-binding antibody fragments, including single-chain antibodies, may comprise the variable region(s) alone or in combination with the entirety or a portion of the following: a hinge region, a CH1, a CH2, or a CH3 domain or combinations thereof. Also included in the invention is, e.g., without limitation, an antigen-binding fragment that also can comprise any combination of variable region(s) with a hinge region, e.g., such as a CH1, CH2, or a CH3 domain or combinations thereof. An antibody of the invention may be from any animal origin including birds and mammals. Preferably, the antibodies are human, primate murine (e.g., mouse and rat), donkey, rabbit, goat, guinea pig, camel, horse, or chicken.

As used herein, the phrase **"human antibodies"** includes, e.g., without limitation, antibodies having an amino acid sequence of a human immunoglobulin including, e.g., without limitation, an antibody isolated from a human immunoglobulin library (such as, e.g., a human germ-line library) or from an animal transgenic for one or more human immunoglobulins and that do not express endogenous immunoglobulins, as described herein or, as taught, e.g., in U.S. Patent No. 5,939,598.

A binding composition may be monospecific, bispecific, trispecific, or having greater multispecificity. Multispecific antibodies may be specific for different epitopes of a target protein, polypeptide (or fragment thereof) or may be specific for both a TGF Beta 1 as well as for a heterologous epitope, such as a heterologous TGF Beta isoform or solid support material (see, e.g., WO 2093/17715; WO 92/08802; WO 91/00360; WO 92/05793; Tutt, et al. (1991) J. Immunol. 147:60-69; U.S. Patent Nos. 4,474,893; 4,714,681; 4,925,648; 5,573,920; or 5,601,819; or Kostelny, et al. (1992) J. Immunol. 148:1547-1553.

A binding composition may be described or specified in terms of an epitope(s) or portion(s) of a TGF Beta 1 protein (or fragment thereof) that it recognizes or selectively and/or specifically binds. An epitope(s) or polypeptide portion(s) may be specified as described herein, e.g., by N-terminal and C-terminal positions, by size in contiguous amino acid residues, or as listed in an accompanying Table and/or Figure, or as described herein. Additionally, an antibody that specifically binds an epitope, polypeptide, protein, or fragment of a polypeptide or protein, may also be specifically excluded from this invention. For instance, Applicants reserve the right to proviso out any antibody that specifically binds an epitope, polypeptide, protein, or fragment of a polypeptide or protein. Accordingly, the invention can encompass a first (or other) antibody that specifically binds a polypeptide or protein, or fragment thereof, and, at the same time, it can exclude a second (or other) antibody that may also selectively bind the same protein or polypeptide, or fragment thereof, e.g., by binding a different epitope. In a preferred embodiment, Applicants proviso out a binding composition which specifically and/or selectively binds TGF Beta 1 isoform over TGF Beta 2 and/or TGF Beta 3 and which neutralizes TGF Beta 1 comprising at least one binding site comprising at least: four contiguous amino acids from QQWNGNPPA [SEQ ID NO: 126]; at least six contiguous amino acids from QQWDSNPPA [SEQ ID NO: 127]; at least five contiguous amino acids from YIYPYNGDTGYNQKFKS [SEQ ID NO: 128] (wherein one of said at least five contiguous amino acids is D); or at least five contiguous amino acids from GYTFTDYTMH [SEQ ID NO: 129].

Antibodies of the invention may also be described or specified in terms of their cross-reactivity. Antibodies that do not bind any other analog, ortholog, paralog, or homologue of a target protein, polypeptide (or fragment thereof) are included.

A binding composition may also be characterized or specified in terms of its binding affinity to a protein or polypeptide (fragment thereof), or epitope. In another embodiment, a preferred binding affinity of a binding composition, e.g., an antibody or antibody binding fragment, includes, e.g., a binding affinity (using an assay described herein) that demonstrates a dissociation constant or Kd of less than (or in a range between two numbers defined below, which range may or may not include either or both end points): 10⁻¹¹ M, 5 X 10⁻¹² M, 4.9 X 10⁻¹² M, 4.8 X 10⁻¹²M, 4.7 X 10⁻¹²M, 4.6 X 10⁻¹²M, 4.5 X 10⁻¹²M, 4.4 X 10⁻¹²M, 4.3 X 10⁻¹²M, 4.2 X 10⁻¹²M, 4.1 X 10⁻¹²M, 4.0 X 10⁻¹²M, 3.9 X 10⁻¹²M,3 . 8 X 10⁻¹²M, 3.7 X 10⁻¹²M, 3.6 X 10⁻¹²M, 3.5 X 10⁻¹²M, 3.4 X 10⁻¹²M, 3.3 X 10⁻¹²M 3.2X 10⁻¹²M, 3.1 X 10⁻¹²M, 3.0 X 10⁻¹²M, 2.9 X 10⁻¹²M , 2.8 X 10⁻¹² M, 2.7 X 10⁻¹²M, 2.6 X 10⁻¹²M, 2.5 X 10⁻¹²M, 2.4 X 10⁻¹²M, 2.3 X 10⁻¹²M, 2.2 X 10⁻¹²M, 2.1 X 10⁻¹²M, 2.0 X 10⁻¹²M, 1.9 X 10⁻¹²M, 1.8 X 10⁻¹²M, 1.7 X 10⁻¹²M, 1.6 X 10⁻¹²M, 1.5 X 10⁻¹²M, 1.4 X 10⁻¹²M, 1.3 X 10⁻¹²M, 1.2 X 10⁻¹²M, 1.1 X 10⁻¹²M, 1.0 X 10⁻¹²M, 0.9 X 10⁻¹²M, 0.8 X 10⁻¹²M, 0.7 X 10⁻¹²M, 0.6 X 10⁻¹²M,0.5 X 10⁻¹²M, 0.4 X 10⁻¹²M, 0.3 X 10⁻¹²M, 0.2 X 10⁻¹²M, 0.1 X 10⁻¹²M, 10⁻¹²M, 5 X 10⁻¹³M, 10⁻¹³M, 5 X 10⁻¹⁴M, 10⁻¹⁴M, 5 X 10⁻¹⁵M, or 10⁻¹⁵M.

Antibodies of the invention may act as antagonists of TGF Beta 1 (or a fragment thereof). For example, an antibody or binding composition of invention can disrupt, e.g., an interaction, either partially or completely, of TGF Beta 1 with a cognate receptor/ligand. Preferably, antibodies of the invention bind an antigenic epitope of TGF Beta 1, or a portion thereof.

Likewise encompassed by the invention, are antibodies that bind a ligand and prevent it binding to a receptor (e.g., by steric hindrance). Similarly encompassed are ligand-binding antibodies that inhibit receptor activation without inhibiting receptor binding.

Antibodies of the invention may be used, e.g., without limitation, to purify, detect, or target a TGF Beta 1 (or fragment thereof) for, e.g., *in vitro* and/or i*n vivo* diagnostic and therapeutic methods. For example, the antibodies have use in immunoassays for qualitatively and/or quantitatively measuring levels of TGF Beta 1 (or fragment thereof) of the invention in a biological sample (see, e.g., Harlow & Lane, Using Antibodies: A Laboratory Manual, (Cold Spring Harbor Laboratory Press, 1999).

A binding composition may be used either alone or in combination with other compositions. Furthermore, a binding composition such as an antibody may be recombinantly fused to a heterologous polypeptide at the N- or C-terminus, or chemically conjugated (including covalently and non-covalently conjugations) to a polypeptide or other compositions. For example, antibodies of the invention may be recombinantly fused or conjugated to molecules useful as labels in detection assays and effector molecules such as heterologous polypeptides, drugs, radionuclides, or toxins (see, e.g., WO 92/08495; WO 91/14438; WO 89/12624; U.S. Patent No. 5,314,995; and EP 396,387).

A binding composition includes, e.g., derivatives that are modified, e.g., by the covalent attachment of any type of molecule to, e.g., an antibody such that the covalent attachment does not prevent the antibody from generating an anti-idiotypic response. For example, an antibody derivative includes without limitation antibodies modified, e.g., by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, e.g., but not limited to, specific chemical cleavage, acetylation, formulation, metabolic synthesis of tunicamycin, etc. Additionally, a derivative may contain one or more non-classical amino acids. A binding composition may be generated by any suitable known art method. For example, monoclonal antibodies can be prepared using any art known technique such as, e.g., Harlow & Lane, Antibodies: A Laboratory Manual, (Cold Spring Harbor Laboratory Press, 1988); Harlow & Lane, Using Antibodies: A Laboratory Manual, (Cold Spring Harbor Laboratory Press, 1999); Breitling & Dübel 1999 Recombinant Antibodies (John Wiley & Sons, New York); H. Zola, Monoclonal Antibodies (Garland Press); and James W. Goding, Monoclonal Antibodies: Principles and Practice (Academic Press).

The term **"monoclonal antibody"** as used herein is not limited to antibodies produced by a particular technique, (e.g., hybridoma technology). Methods for producing and screening for specific antibodies using hybridoma technology are routine and known in the art. The term "monoclonal antibody" refers to an antibody derived from a single clone, including any eukaryotic, prokaryotic, or phage clone, and not the method by which it is produced.

**Antibody fragments** that recognize specific epitopes may be generated by known techniques. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the Light chain. For example, a binding composition can also be generated using various phage display methods known in the art in which functional antibody domains are displayed on the surface of phage particles, which carry a polynucleotide sequence encoding them.

In a particular embodiment, a phage display method is used to display antigen-binding domains expressed from a repertoire or combinatorial antibody library (e.g., human or murine). Phage that express an antigen binding domain that binds an antigen of interest can be selected or identified with antigen, e.g., using labeled antigen or antigen bound or captured to a solid surface or bead. After phage selection, antibody coding regions from a phage are isolated and used to generate whole antibodies, including human antibodies, or any other desired antigen binding fragment, and expressed in any desired host, including mammalian cells, insect cells, plant cells, yeast, and bacteria, e.g., as described herein or in the literature.

Techniques to recombinantly produce Fab, Fab' and F(ab')2 fragments can also be employed using art known methods such as, e.g., WO 92/122324; Mullinax, et al., BioTechniques 1992 12(6):864-9; and Sawai, et al., 1995 AJRI 34:26-34; and Better, et al., 1988 Science 240:1041-3. Examples of producing single-chain Fvs and antibodies include, e.g., U.S. Patents 4,946,778 and 5,258,498; Huston, et al., 1991 Methods in Enzymology 203:46-88; Shu, et al., 1993 PNAS USA 90:7995-9; and Skerra, et al., 1988 Science 240: 1038-40. For some uses, including *in vivo* use of antibodies in humans and *in vitro* detection assays, it may be preferable to use humanized, or human antibodies produced by art known techniques.

**Humanized antibodies** herein are binding compositions that bind a desired antigen having one or more complementarity determining regions (CDRs) as described herein embedded within a framework region that is less likely to create a detrimental immunogenicity reaction upon *in vivo* introduction to a human host's system (e.g., after parenteral administration). Often, CDR donor regions are appropriately embedded within human framework regions to improve antigen binding and/or reduce immunogenicity. Useful framework regions can be identified using art known methods, e.g., by (1) modeling the interactions of a CDR and framework residues to identify framework residues important for antigen binding; (2) by sequence comparison to identify unusual framework residues at particular positions (see, e.g., U.S. Patent No. 5,585,089, Riechmann, et al., Nature 332:323 (1988); or (3) empirically.

Variable heavy and light chain complementary determining regions (CDRs) of particular monoclonal antibody binding composition embodiments of the invention are shown below in Table 1(a & b). The CDR regions are indicated using standard amino acid single letter code and standard CDR numbering, (i.e., with the increasing numerical value of a CDR corresponding with its increasing proximity to the constant domain of a typical IgG heavy or light chain structure; e.g., VH CDR3 is more proximal to the CH1 domain than VH CDR1).

Specific CDR embodiments are represented generically using amino acid formulae to describe a genus of CDRs (again, using standard single letter amino acid code with substitutable amino acid residues indicated by the letter "X" and their residue placement within a particular CDR indicated by a numeric subscript whose value increases from lowest (amino-most) to highest (carboxy-most) residue in the CDR (e.g., X₁ in VHCDR2 is the most amino residue of the CDR while the carboxy-most substitutable residue is X₆). Using these generic formulae, one of ordinary skill in the art is capable of determining all CDR embodiments possible at each designated position in a variable heavy or light chain domain (V_{L} or V_{H}) embodiment encompassed by the invention. Any particular instantiation of a possible amino acid sequence is determined merely by generating all possible substitutions at every particular X residue within the particular CDR formula using the amino acid code. All such embodiments are encompassed herein.)).

The information in the following tables is provided for information only and does not form part of the invention.

**Table 1a CDR Heavy Chain Formula of Binding Compositions**

| **Heavy Chain CDRs** | | |
|---|---|---|
| **CDR1** | **CDR2** | **CDR3** |
| GYX₁FX₂DYNX₃X₄ * | X₁X₂YPYDGX₃TGX₄NX₅K₆KS ** | GYRX₁X₂X₃Y*** |
| **[SEQ ID NO: 2]** | **[SEQ ID NO: 3]** | **[SEQ ID NO: 4]** |

| | | |
|---|---|---|
| • * For VHCDR1: X₁ is either T or D; X₂ is either T, E, or F; X₃ is either M, I, L, or V; and X₄ is either H, V, or A. • **For VHCDR2: X₁ is either Y, Q, or S; X₂ is either I, or V; X₃ is either D, or E; X₄ is either Y, T, H, or L; X₅ is either Q, K, P, or S; and X₆ is either F or Y. • *** For VHCDR3: X₁ is either W or A; X₂ is either F or L; and X₃ is either A, E, or Y. | | |

**Table 1b CDR Light Chain Formula of Binding Compositions**

| **Light Chain CDRs** | | |
|---|---|---|
| **CDR1** | **CDR2** | **CDR3** |
| X₁AX₂X₃X₄VX₅ YMH * | ATSNX₁AX₂ ** | X₁QWDX₂X₃X₄PA *** |
| **[SEQ ID NO: 5]** | **[SEQ ID NO: 6]** | **[SEQ ID NO: 7]** |

| | | |
|---|---|---|
| • * For VLCDR1: X₁ is either R, Y, E, or Q; X₂ is either S or T; X₃ is either S, V, or A; X₄ is either S or L; X₅ is either S, P, L, or Y. • **For VLCDR2: X₁, is either L, N, or P; and X₂ is either S, K, Y, L, M, F, E, Q, R, or H. • ***For VLCDR3: X₁ is either Q or S; X₂ is either L, D, or P; X₃ is either N or R; and X₄ is either P, F, Y, or R. | | |

Further, encompassed are antibody binding compositions using CDRs encompassed herein that are embedded (in appropriate orientation) or carried within human antibody framework regions to enable the resulting binding composition to specifically and/or selectively bind mature TGF Beta 1 over mature TGF Beta 2 and/or mature TGF Beta 3 and to neutralize mature TGF Beta 1. Art known techniques can be used to embed or place particular CDRs within appropriate frameworks.

In a preferred embodiment, the HCVR FR1 framework comprises QVQLVQSGAEVKKPGASVKVSCKAS **[SEQ ID NO: 8];** the HCVR FR2 framework comprises WVRQAPGQGLEWMG **[SEQ ID NO: 9];** the HCVR FR3 framework comprises RVTMTTDTSTSTAYMELRSLRSDDTAVYYCAR **[SEQ ID NO: 10];** and the HCVR FR4 framework comprises WGQGTLVTVSS **[SEQ ID NO: 11].** In another preferred embodiment, the LCVR FR1 framework comprises DIQMTQSPSSLSASVGDRVTITC **[SEQ ID NO: 12];** the LCVR FR2 framework comprises a sequence selected from: **[SEQ ID NO: 13,14 & 18],** the LCVR FR3 framework comprises GVPSRFSGSGSGTDFTLTISSLQPEDFATYYC [SEQ ID NO: 37]; and the LCVR FR4 framework comprises FGQGTKLEIK [SEQ ID NO: 38]. Also disclosed are framework regions which may contain alterations, deletions, additions, substitutions, or any combination thereof. Moreover, frameworks in which 1, 2, 3, 4, 5, 6, 7, 8, 9, or 10 amino acids are substituted, deleted, or added in any combination are also disclosed.

In one embodiment, a preferred heavy chain constant region for use in embedding antibody binding composition CDRs of the invention includes, for example, an IgG constant region. In a more preferred embodiment, the IgG constant region is an IgG1 constant region or an IgG4 constant region as shown below:
IgG1 **[SEQ ID NO: 39]**: or
IgG4 **[SEQ ID NO: 40]**

A preferred light chain constant region sequence of the invention is the kappa chain constant region shown below:

In another preferred embodiment, antibody, binding compositions contain the IgG1 Heavy chain constant region or the IgG4 Heavy chain constant region and the kappa Light chain constant region.

The following embodiment is provided for information only and is not part of the invention. Using the information provided herein, one of ordinary skill could create a mAb embodiment, for example, such as No. 46P-L1-6, which would have a Light Chain comprising: where:
the LCVR FR1 framework = DIQMTQSPSSLSASVGDRVTITC;
the VL CDR1 = EASSSVSYMH of the formula X₁AX₂X₃X₄VX₅ YMH, where X1 is either R, Y, E, or Q; X2 is either S or T; X3 is either S, V, or A; X4 is either S or L; and X5 is either S, P, L, or Y;
the LCVR FR2 framework = WYQQKPGKAPKPLIY;
the VL CDR2 = ATSNLAS of the formula ATSNX1AX₂, where X₁ is either L, N, or P; and X₂ is either S, K, Y, L, M, F, E, Q, R, or H;
the LCVR FR3 framework = GVPSRFSGSGSGTDFTLTISSLQPEDFATYYC;
the VL CDR3 = QQWDLNPPA of the formula X₁QWDX₂X₃X₄PA, where X₁ is either Q or S; X₂ is either L, D, or P; X₃ is either N or R; and X₄ is either P, F, Y, or R;
the LCVR FR4 framework = FGQGTKLEIK; and
the Light Chain constant region = and a Heavy chain comprising : where: the HCVR FR1 framework = QVQLVQSGAEVKKPGASVKVSCKAS;
the VH CDR1 = GYTFTDYNMH of the formula GYX₁FX₂DYNX₃X₄ ; X₁ is either T or D; X₂ is either T, E, or F; X₃ is either M, I, L, or V; and X₄ is either H, V, or A;
the HCVR FR2 framework = WVRQAPGQGLEWMG;
the VH CDR2 = YIYPYDGDTGYNQKFKS of the formula X₁X₂YPYDGX₃TGX4NX₅KX₆KS ; X₁ is either Y, Q, or S; X₂ is either I, or V; X₃ is either D, or E; X₄ is either Y, T, H, or L; X₅ is either Q, K, P, or S; and X₆ is either F or Y;
the HCVR FR3 framework = RVTMTTDTSTSTAYMELRSLRSDDTAVYYCAR;
the VH CDR3 = GYRWFAY of the formula GYRX₁X₂X₃Y ; where X₁ is either W or A; X₂ is either F or L; and X₃ is either A, E, or Y;
the HCVR FR4 framework = wGQGTLVTVSS; and
the Heavy Chain constant region =

### Polynucleotides Encoding Antibodies.

Nucleic acid molecules comprising polynucleotide sequences that encode a binding composition (or fragment thereof), or polypeptide sequence of the invention may be obtained, and the nucleotide sequence of the polynucleotides determined by any art known method, for example, if the polypeptide sequence (e.g., of an antibody or fragment thereof) is known, a polynucleotide encoding the polypeptide can be determined simply by using the degeneracy of the genetic code in any computer algorithm and the resulting sequence information can be used to assemble, for example, chemically synthesized oligonucleotides (e.g., as described in Kutmeier, et al., (1994) BioTechniques 17:242), which, briefly described, involves synthesizing overlapping oligonucleotides containing portions of the sequence encoding the polypeptide sequence, annealing, and ligating those oligonucleotides, then, amplifying the ligated oligonucleotides using a polymerase chain reaction.

Alternatively, a polynucleotide encoding a polypeptide sequence of the invention can be generated from nucleic acid of any suitable source. If a clone containing a nucleic acid molecule encoding a particular antibody is not available, but, however, the sequence of the antibody molecule is known, then a nucleic acid encoding the immunoglobulin may be chemically synthesized or obtained from a suitable source. For example the source may be an antibody cDNA library, or a cDNA library generated from poly A+ RNA, isolated from any tissue or cell expressing the antibody of interest, such as, e.g., a hybridoma cells selected to express an antibody of the invention by PCR amplification using synthetic primers hybridizable to the 3' and 5' ends of a polynucleotide sequence of interest or by cloning using an oligonucleotide probe specific for the particular gene sequence to identify, e.g., a cDNA clone from a cDNA library that encodes the antibody, a nucleic acid molecule for the antibody can be generated.

Amplified nucleic acids may be cloned into replicable cloning vectors using any art known method. Once the nucleotide and corresponding amino acid sequence of the antibody are determined, the nucleotide sequence of the antibody may be manipulated using any known art method, e.g., recombinant DNA techniques, site directed mutagenesis, PCR, etc. to generate antibodies having a different amino acid sequence to create amino acid substitutions, deletions, and/or insertions (see, e.g., Sambrook, et al., and Ausubel, et al., eds., cur. ed., Current Protocols in Molecular Biology, John Wiley & Sons, NY).

In a specific embodiment, the amino acid sequence of the heavy and/or light chain variable domains may be inspected to identify the sequences of complementarity determining regions (CDRs) by known methods, e.g., by comparing known amino acid sequences of other heavy and light chain variable regions to determine regions of sequence hypervariability. Using routine recombinant DNA techniques, one or more CDRs described herein may be inserted within suitable framework regions, e.g., into human framework regions to reduce immunogenicity. Framework regions may be naturally occurring or consensus framework regions (or as taught herein) and are preferably human framework regions (for a listing of human framework regions see, e.g., Chothia, et al. 1998 J. Mol. Biol. 278: 457-79). Generally speaking, to identify residues within the human frameworks likely to influence the integrity of the antigen binding site, and therefore antigen binding, both donor and selected human acceptor sequences can be aligned to several sequence templates derived from antibody repertoires. 'Invariant residues' (Kabat et al., 1991) and 'key residues' (Chothia et al., 1989) are identified, and canonical-class assignments of the donor antigen binding loops L1-L3, H1 and H2, respectively, are determined by screening the sequence against sequence templates (Martin & Thornton, 1996 Mol. Biol. 263:800-15 at http://www.bioinf. org.uk/). Furthermore, residues at the VH/VL interface (Chothia et al., 1985) and residues known to be structurally conserved at core sites (Chothia et al., 1998), are compared with corresponding donor and acceptor residues. Non-matching donor and acceptor framework residues at these sites are analyzed based on information from other antibodies of known structure from the Protein Data Bank (Berman, et al., 2000 Nucl. Acids Res. 28(1):235-42). Selection of the human frameworks to use as templates for humanization of foreign V regions can define subsequent decisions regarding which residues to humanize. Choosing homologous templates from antibodies with known crystal structure, from germline, non-germline, or consensus sequences derived from available data bases are options (for a review, see Routledge et al. (Routledge, et al., 1993 in Protein Engineering of Antibody Molecules for Prophylactic and Therapeutic Applications in Man (Clark, M., ed) pp. 14-44, Academic Titles, Nottingham, UK; and B. Lo, 2004 Antibody Engineering: Methods and Protocols, Humana Press)). Another strategy to humanize antibodies is to choose the closest human germline sequence (Tomlinson et al., 1992) as the framework to receive the donor CDRs. This germline approach is based on the same rationale as the best-fit strategy, but only germline sequences are searched in the databases (see, e.g.,V BASE, which is a comprehensive directory of all human germline variable region sequences compiled from over a thousand published sequences, including those in the current releases of the Genbank and EMBL data libraries. The V BASE database has been developed over several years at the MRC Center for Protein Engineering (Cambridge, UK) as an extension of work on the sequencing and mapping of human antibody genes and as a user friendly tool for their analysis). The germline framework method is a very useful because a human germline sequence does not present somatic hypermutations that are potentially immunogenic. CDRs can also be grafted or embedded into human frameworks using a consensus human germline strategy, in which one of the human subgroups is used as the framework (see, e.g., Presta et al., 1993 J. Immunol 151:2623-32; Couto et al., 1994 Hybridoma, 13:215-9; Couto et al., 1995 Cancer Res. (Suppl.), 55, 5973s-7s; Werther et al., 1996 J. Immunol., 157:4986-95; O'Connor et al., 1998 Protein Engng 11:321-8).

Preferably, the polynucleotide generated by the combination of the framework regions and CDRs encodes an antibody (or fragment thereof) that specifically and/or selectively binds TGF Beta 1 (or epitope thereof). Preferably, as discussed herein, one or more amino acid substitutions may be made within the framework regions to improves binding of the antibody to its antigen.

Additionally, such methods may be used to make amino acid substitutions or deletions of one or more variable-region, cysteine residues participating in an intrachain disulfide bond to generate antibody molecules lacking one or more intrachain disulfide bonds. Other alterations to the polynucleotide are encompassed by the invention and within the skill of an ordinary artisan, e.g., such as a molecular biologist.

Alternatively, techniques can be adapted to produce single chain antibodies (see, e.g., U.S. Patent No. 4,946,778; Bird, Science 242:423-42 (1988); Huston, et al., PNAS 85:5879-5883 (1988); and Ward, et al., Nature 334:544-54 (1989)). Single chain antibodies are formed by linking the heavy and light chain fragments of the Fv region via an ammo acid bridge, resulting in a single chain polypeptide. Techniques for the assembly of functional Fv fragments in *E*. *coli* may also be used (Skewa, et al. (1988) Science 242: 1038-1041).

### Polypeptide Fragments

The invention also encompasses fragments of a binding composition. A "polypeptide fragment or segment" encompasses an amino acid sequence that is a portion of a sequence described herein or a portion of a polypeptide sequence of a SEQ ID NO:. Protein and/or polypeptide fragments or segments may be "free-standing," or they may comprise part of a larger polypeptide or protein, of which the fragment or segment forms a portion or region, e.g., a single continuous region of a SEQ ID NO: herein connected in a fusion protein.

Preferably, a polypeptide segment can have a length of contiguous amino acids that is at least about: 7, 8, 9, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 110, 120, 130, 140, or 150 contiguous amino acids in length. In this context "about" includes, e.g., the specifically recited ranges or values described herein, and it also encompasses values that differ from these recited values by several amino acid residues (e.g., plus or minus 5, plus or minus 4, plus or minus 3, plus or minus 2, or; plus or minus 1 amino acid residues), at either or both ends of the fragment. Polynucleotides encoding such a polypeptide fragment are also encompassed by the invention.

Moreover, the invention encompasses proteins or polypeptides comprising a plurality of said amino acid segments or fragments, e.g., nonoverlapping, segments of a specified length. Typically, a plurality will be at least two, more usually at least three, and preferably at least: four, five, six, seven, eight, nine, or more. While minimum lengths of a segment are provided, maximum lengths of various sizes are also encompassed for any specific plurality of segments, e.g., a plurality of three segments in *toto* could have one segment of length 7 contiguous amino acids, and two additional nonoverlapping segments, each of which has a length of 12.

Also preferred are polypeptide fragments or segments (and their corresponding polynucleotide fragments) that characterize structural or functional domains, such as, fragments, or combinations thereof, that comprise e.g., complementary determining regions (CDRs such as VL or VH: CDR1, CDR2, or CDR3), variable regions (e.g., of the heavy or light chains, VL or VH), framework regions (FH1, 2, 3, or 4), D or J regions, constant domains (e.g., such as, C_{L}, C_{H}1, C_{H}2 or C_{H}3), hinge regions, Fc gamma receptor binding regions, alpha-helix, and alpha-helix forming regions, beta-sheet, and beta-sheet-forming regions, turn, and turn-forming regions, coil, and coil-forming regions, hydrophilic regions, hydrophobic regions, alpha amphipathic regions, beta amphipathic regions, flexible regions, loop regions, hairpin domains, beta-alpa-beta motifs, helix bundles, alpha/beta barrels, up and down beta barrels, jelly roll or Swiss roll motifs, transmembrane domains, surface-forming regions, substrate binding regions, transmembrane regions, linkers, immunogenic regions, epitopic regions, and high antigenic index regions. Moreover, polynucleotides encoding these domains are also encompassed.

Other preferred polypeptide segments are biologically active fragments. Biologically active fragments are those exhibiting activity similar, but not necessarily identical, to an activity of a binding composition polypeptide (or fragment thereof) such as, e.g., Fab, Fv, scFv, or F(ab)2. Polynucleotides encoding these polypeptide fragments are also encompassed by the invention.

Preferably, the polynucleotide fragments encode a polypeptide that demonstrates a functional activity. The phrase **"functional activity"** encompasses a polypeptide segment that can accomplish one or more known functional activities. Such functional activities include, e.g., without limitation, biological activity, antigenicity [ability to bind (or compete with a polypeptide for binding) to an antibody binding composition to a polypeptide of the invention], immunogenicity (ability to generate antibody that binds to a polypeptide of the invention), ability to form multimers with a polypeptide of the invention, and the ability to bind to a receptor or ligand of a polypeptide described herein.

The functional activity of a polypeptide (including fragments, variants, derivatives, and analogs thereof) can be assayed by various methods. For example, where one is assaying for the ability to bind or compete with a full-length polypeptide of the invention for binding to an antibody of a polypeptide of the invention, various immunoassays known in the art can be used, including, e.g., without limitation, competitive and non-competitive assay systems using techniques such as radioimmunoassays, ELISA (enzyme linked immunosorbent assay), "sandwich" immunoassays, immuno-radiometric assays, gel diffusion precipitation reactions, immuno-diffusion assays, in situ immunoassays (using colloidal gold, enzyme or radioisotope labels, for example), western blots, precipitation reactions, agglutination assays (e.g., gel agglutination assays, hemagglutination assays, complement fixation assays, immunofluorescence assays, protein A assays, and immuno-electrophoresis assays, etc.).

In another embodiment, antibody binding is accomplished by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the invention.

In another embodiment, where a ligand is identified, or the ability of a polypeptide fragment, variant or derivative of the invention to multimerize is being evaluated, binding can be assayed, e.g., by using reducing and non-reducing gel chromatography, protein affinity chromatography, and affinity blotting (see generally, Phizicky, et al. (1995) Microbial. Rev. 59:94-123). In another embodiment, physiological correlates of binding of a polypeptide to its substrates (signal transduction) can be assayed with common techniques. In addition, assays described herein (see, e.g., the "Examples" section of the application), or otherwise known in the art, can routinely be applied to measure the ability of a binding composition (its fragments, variants derivatives and analogs thereof) to modulate a related biological activity (either *in vitro* or *in vivo*) of TGF Beta 1.

### Methods of Producing Antibodies

An antibody binding composition can be produced using any known art method, in particular, by chemical synthesis or preferably, by recombinant expression techniques.

Recombinant expression of a binding composition, or fragment, derivative or analog thereof, (e.g., a heavy or light chain of an antibody of the invention or a single chain antibody of the invention), requires construction of an expression vector containing a polynucleotide sequence that encodes the antibody. Once a polynucleotide sequence encoding an antibody molecule or a heavy or light chain of an antibody, or portion thereof (preferably containing the heavy or light chain variable domain), of the invention has been obtained, the vector for the production of the antibody molecule may be produced by know recombinant DNA technology techniques.

Methods known in the art can be used to construct expression vectors containing binding composition coding sequences and appropriate transcriptional and translational control signals. These methods include, e.g., without limitation, *in vitro* recombinant DNA techniques, synthetic techniques, and *in vivo* genetic recombination. The invention, thus, encompasses replicable vectors comprising nucleotide sequence encoding an antibody of the invention (or fragment thereof), or a heavy or light chain thereof, or a heavy or light chain variable domain, or a CDR operably-linked to a promoter. Such vectors may include, e.g., the nucleotide sequence encoding the constant region of the antibody molecule (see, e.g., WO 86/05807; WO 89/01036; or U.S. Patent No. 5,122,464) and the variable domain of the antibody may be cloned into such a vector for expression of the entire heavy or light chaim or any portion thereof.

Generally speaking, an expression vector is transferred to a host cell by conventional techniques and the transfected cells are then cultured to produce an antibody or portion thereof. Thus, the invention also encompasses, e.g., host cells containing a polynucleotide encoding an antibody of the invention, or a heavy or light chain thereof, or a single chain antibody of the invention, operably linked to a heterologous promoter. In preferred embodiments for the expression of double-chained antibodies, vectors encoding both the heavy and light chains may be co-expressed in a host cell for expression of the entire immunoglobulin molecule, as detailed herein or known in the art.

A variety of host-expression vector systems may be utilized to express antibody molecules of the invention. Such host-expression systems represent vehicles by which any coding sequence of interest may be produced and subsequently purified. However, when transformed or transfected with an appropriate nucleotide coding sequence, host-expression system cells may also represent an antibody molecule of the invention *in situ.* These cells include, e.g., without limitation, microorganisms such as bacteria (e.g., *E*. *coli, B. subtilis*) transformed with recombinant bacteriophage DNA, plasmid DNA, or cosmid DNA expression vectors containing antibody coding sequences; yeast (e.g., *Saccharomyces, Pichia*) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, 293, 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter).

Preferably, bacterial cells such as *Escherichia coli,* and more preferably, eukaryotic cells are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking, et al. (1986) Gene 45:101; Cockett, et al. (1990) Bio/Technology 8:2).

In bacterial systems, a number of expression vectors may be advantageously selected depending upon the intended use of the expressed antibody molecule. For example, when a large quantity of protein is to be produced, like, e.g., for the generation of pharmaceutical compositions of an antibody molecule, then vectors that direct the expression of high levels of fusion protein products, which are readily purified may be desirable. Such vectors include, e.g., without limitation, the *E. coli* expression vector pUR278 (Ruther, et al., EMBO J. 2: 1791 (1983)), in which the antibody coding sequence may be ligated individually into the vector in frame with the lac Z coding region so that a fusion protein is produced; pIN vectors (Inouye and Inouye, Nucleic Acids Res. 13:3 101-3 109 (1985); Van Heeke and Schuster, J. Biol. Chem. 24:5503-5509 (1989)); and the like. The pGEX vectors may also be used to express foreign polypeptides as fusion proteins with glutathione S-transferase (GST).

In general, such fusion proteins are soluble and can easily be purified from lysed cells by adsorption and binding to matrix glutathione-agarose beads followed by elution in the presence of free glutathione. The pGEX vectors are designed to include, e.g., thrombin, or factor Xa protease cleavage sites so that the cloned target gene product can be released from a GST moiety. One insect system used as a vector to express a foreign gene, is the *Autographa californica* nuclear polyhedrosis virus (AcNPV) system. The AcNPV virus grows in *Spodopteru frugiperda* cells. The antibody coding sequence may be cloned individually into non-essential regions (e.g., the polyhedrin gene) of the virus and placed under control of an AcNPV promoter (e.g., the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome using *in vitro* or *in vivo* recombination. Insertion in a non-essential region of the viral genome (e.g., region E1 or E3) results in a recombinant virus that is viable and capable of expressing the antibody molecule in infected hosts (see, e.g., Logan & Shenk, 1984 PNAS 8 1:355-9). Specific initiation signals may be required for efficient translation of inserted antibody coding sequences. These signals include, e.g., the ATG initiation codon, and adjacent sequences. Furthermore, the initiation codon must be in phase with the reading frame of the desired coding sequence to ensure proper translation of the entire insert. These exogenous translational control signals and initiation codons can be of a variety of origins, both natural and synthetic.

The efficiency of expression may be enhanced by the inclusion of appropriate transcription enhancer elements, transcription terminators, etc. (see, e.g., Bittner, et al., 1987 Methods in Enzymol. 153:5 1-4). In addition, a host cell strain may be chosen that modulates the expression of the inserted sequence, or modifies and processes the gene product in the specific fashion desired. Such modifications (e.g., glycosylation) and processing (e.g., cleavage) of protein products may be important for the function of a protein.

Different host cells have characteristic and specific mechanisms for the post-translational processing and modification of proteins and gene products. Appropriate cell lines or host systems can be chosen to ensure the correct modification and processing of a foreign protein that is expressed. To this end, eukaryotic host cells that possess the cellular machinery for proper processing of the primary transcript, glycosylation, and phosphorylation may be used. Such mammalian host cells include, e.g., without limitation, CHO, VERY, BHK, HeLa, COS, MDCK, 293, 3T3, W138, and, in particular, breast cancer cell lines such as, e.g., BT483, Hs578T, HTB2, BT20 and T47D, and normal mammary gland cell line such as, e.g., CRL7030 and Hs578Bst.

The invention encompasses antibodies recombinantly fused or chemically conjugated (including both covalent and non-covalent conjugations) to a polypeptide (or portion thereof, preferably comprising at least: 10, 20, 30, 40, 50, 60, 70, 80, 90, or 100 contiguous amino acids of the polypeptide) to generate fusion proteins. The fusion does not necessarily need to be direct, but may occur through linker sequences.

Antibodies fused or conjugated to a polypeptide may also be used in *in vitro* immunoassays and in purification methods using known art methods (see e.g., Harbor, et al., *supra,* and WO 9312 1232; EP 439,095; Naramura et al. (1994) Immunol. Lett. 39:91-9; U.S. Patent No. 5,474,981; Gillies, et al. 1992 PNAS 89:1428-32; Fell, et al. 1991 J. Immunol. 146:2446-52).

The invention further includes compositions comprising a polypeptide (or fragment thereof) fused or conjugated to an antibody domain other than a variable region. For example, a polypeptide of the invention (or fragment thereof) may be fused or conjugated to an antibody constant region, D or J region, or an Fc region, or portion thereof. The antibody portion that is fused to a polypeptide of the invention (or fragment thereof) may comprise a constant region, a hinge region, a CH1 domain, a CH2 domain, and/or a CH3 domain or any combination of whole domains or portions thereof. A polypeptide (or fragment thereof) may also be fused or conjugated to an antibody portion described herein to form multimers. For example, Fc portions fused to a polypeptide of the invention (or fragment thereof) can form dimers through disulfide bonding between the Fc portions. Higher multimeric forms can be made by fusing the polypeptides to portions of IgA and IgM. Methods for fusing or conjugating a polypeptide of the invention (or fragment thereof) to an antibody portion are known (see, e.g., U.S. Patent Nos. 5,336,603; 5,622,929; 5,359,046; 5,349,053; 5,447,851; 5,112,946; EP 307,434; EP 367,166; WO 96/04388; WO9106,570; Ashkenazi, et al. (1991) PNAS 88: 10535-10539; Zheng, et al. (1995) J. Immunol. 154:5590-5600; and Vie, et al. (1992) PNAS 89: 11337-11341).

As discussed herein, a polypeptide, polypeptide fragment, may be fused or conjugated to an antibody portion described herein or known in the art to increase the *in* vivo half-life. Further, a polypeptide, polypeptide fragment, may be fused or conjugated to an antibody portion to facilitate purification. One example uses chimeric proteins comprising the first two domains of the human CD4-polypeptide and various domains of the constant regions of the heavy or light chains of mammalian immunoglobulins. (see, e.g., EP 394,827; Traunecker, et al. (1988) Nature 33 1:84-86).

In many cases, the Fc part of a fusion protein is beneficial in therapy and diagnosis, and thus can result in, e.g., improved pharmacokinetic properties (see, e.g., EP A232, 262). Alternatively, deleting the Fc part after the fusion protein has been expressed, detected, and purified, can be favored. For example, the Fc portion may hinder therapy and diagnosis if the fusion protein is used as an antigen for immunizations. In drug discovery, e.g., human proteins, such as hIL-5, have been fused with Fc portions for the purpose of high-throughput screening assays to identify antagonists of hIL-5 (see, e.g., Bennett, et al. (1995) J. Molecular Recognition 8:52-58; Johanson, et al. (1995) J. Biol. Chem. 270:9459-9471).

Moreover, a binding composition (or fragment thereof) can be fused to marker sequences, such as a peptide to facilitate purification. In preferred embodiments, the marker amino acid sequence is a hexa-histidine peptide, such as the tag provided in a pQE vector (QIAGEN, Inc., Chatsworth, CA), among others, many of which are commercially available. Hexa-histidine provides for convenient purification of a fusion protein (Gentz, et al. (1989) PNAS 86:821-824). Other peptide tags useful for purification include, e.g., the "HA" tag, which corresponds to an epitope derived from the influenza hemagglutinin protein (Wilson, et al. (1984) Cell 37:767) and the "flag" tag.

The invention further encompasses antibodies or fragments thereof conjugated to a diagnostic or therapeutic agent. The antibodies can be used diagnostically to, for-example, monitor the development or progression of a tumor as part of a clinical testing procedure to determine the efficacy of a given treatment regimen. Detection can be facilitated by coupling the antibody to a detectable substance. Examples of detectable substances include, e.g., various enzymes, prosthetic groups, fluorescent materials, luminescent materials, bioluminescent materials, radioactive materials, positron emitting metals using various positron emission tomographies, and nonradioactive paramagnetic metal ions. The detectable substance may be coupled or conjugated either directly to the antibody (or fragment thereof) or indirectly, through an intermediate (such as, e.g., an art known linker) using established techniques (see, e.g., U.S. Patent No. 4,741,900 for metal ions that can be conjugated to antibodies for use as diagnostics according to the invention). Examples of suitable enzymes include, e.g., without limitation, horseradish peroxidase, alkaline phosphatase, beta-galactosidase, or acetylcholinesterase; examples of suitable prosthetic group complexes include, e.g., without limit, streptavidin/biotin and avidin/biotin; examples of suitable fluorescent materials include, e.g:, without limitation, umbelliferone, fluorescein, fluorescein isothiocyanate, rhodamine, dichlorotriazinylamine fluorescein, dansyl chloride, or phycoerythrin; an example of a luminescent material includes, e.g., without limitation, luminol; examples of bioluminescent materials include, e.g., without limitation, luciferase, luciferin, and aequorin; and examples of a suitable radioactive material includes, e.g., I¹²⁵, I¹³¹, I¹¹¹ or Tc⁹⁹.

A binding composition of the invention can also be attached to solid support, which are particularly useful for immunoassays or purification of a target antigen. Such solid supports include, e.g., without limitation, glass, cellulose, poly-acrylamide, nylon, polystyrene, polyvinyl chloride, or polypropylene. Techniques for conjugating a therapeutic moiety to an antibody are known, see, e.g., Amon, et al., "Monoclonal Antibodies For Immunotargeting Of Drugs In Cancer Therapy", in Monoclonal Antibodies And Cancer Therapy, Reisfeld, et al. (eds.), pp. 243-56 (Alan R. Liss, Inc.1985); Hellstrom, et al., "Antibodies For Drug Delivery", in Controlled Drug Delivery (2nd Ed.), Robinson, et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera, et al. (eds.), pp. 475-506 (1985); "Analysis, Results, and Future Prospective of the Therapeutic Use Of Radiolabeled Antibody in Cancer Therapy", in Monoclonal Antibodies for Cancer Detection and Therapy, Baldwin, et al. (eds.), pp. 303-16 (Academic Press 1985), and Thorpe, et al., "The Preparation and Cytotoxic Properties of Antibody-Toxin Conjugates," Immunol. Rev. 62: 119-58 (1982).

### B. Immunoassays

A particular protein such as TGF Beta 1 can be measured by a variety of immunoassay methods including, e.g., without limitation, competitive and non-competitive assay systems using techniques such as, e.g., without limitation, western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, and protein A immunoassays. For a review of immunological and immunoassay procedures in general, see Stites and Terr (eds.) (1991) Basic and Clinical Immunology (7th ed.). Moreover, the immunoassays of the invention can be performed in many configuration, which are reviewed extensively in Maggio (ed.) (1980) Enzyme Immunoassay CRC Press, Boca Raton, Florida; Gosling J P 2000 Immunoassays: A Practical Approach (Practical Approach Series) Oxford Univ Press; Diamandis & Christopoulus, 1996 Immunoassay Academic Press, San Diego, CA; Tijan (1985) "Practice and Theory of Enzyme Immunoassays," Laboratory Techniques in Biochemistry and Molecular Biology, Elsevier Science Publishers B.V., Amsterdam; Wild, D. (Ed.), 2001 The Immunoassay Handbook (2nd edition) Nature Pub Group; James T. Wu, 2000 Quantitative Immunoassay: A Practical Guide for Assay Establishment, Troubleshooting, and Clinical Application. Amer Assn for Clinical Chemistry, Brousseau & Beaudet (Eds.) Manual of Immunological Methods CRC Press Boca Raton, Florida; and Harlow and Lane Antibodies, A Laboratory Manual, *supra.* See also Chan (ed.) (1987) Immunoassay: A Practical Guide Academic Press, Orlando, FL; Price and Newman (eds.) (1991) Principles and Practice of Immunoassays Stockton Press, NY; and Ngo (ed.) (1988) Non-isotopic Immunoassays Plenum Press, NY.

Immunoassays for measurement can be performed by a variety of art-known methods. In brief, immunoassays to measure the protein can be either competitive or noncompetitive binding assays. In competitive binding assays, the sample to be analyzed competes with a labeled analyte for specific binding sites on a capture agent bound to a solid surface. Preferably, the capture agent is an antibody specifically reactive with a TGF Beta 1 protein as described herein. The concentration of labeled analyte bound to the capture agent is inversely proportional to the amount of free analyte present in the sample.

In a competitive binding immunoassay, the target protein present in the sample competes with labeled protein for binding to a specific binding composition, for example, a binding composition, such as an antibody, that is specifically and/or selectively reactive with the target protein. The binding composition may be bound to a solid surface to effect separation of bound-labeled protein from the unbound-labeled protein. Alternately, the competitive binding assay may be conducted in liquid phase and a variety of techniques known in the art may be used to separate the bound-labeled protein from the unbound-labeled protein. Following separation, the amount of bound labeled protein is determined. The amount of protein present in the sample is inversely proportional to the amount of labeled protein binding.

Alternatively, a homogeneous immunoassay may be performed in which a separation step is not needed. In these immunoassays, the label on the protein is altered by the binding of the protein to its specific binding composition. This alteration in the labeled protein results in a decrease or increase in the signal emitted by label, so that measurement of the label at the end of the immunoassay allows for detection or quantitation of the protein.

Competitive assays are also particularly useful, where the cells are contacted and incubated with a labeled binding partner or antibody having known binding affinity to the protein, such as ¹²⁵I-antibody, and a test sample whose binding affinity to the binding composition is being measured. The bound and free-labeled binding compositions are then separated to assess the degree of protein binding. The amount of test compound bound is inversely proportional to the amount of labeled binding partner binding to the known source. Any one of numerous techniques can be used to separate bound from free protein to assess the degree of protein binding. This separation step could typically involve a procedure such as adhesion to filters followed by washing, adhesion to plastic followed by washing, or centrifugation of the cell membranes. Viable cells could also be used to screen for the effects of drugs on a TGF beta protein mediated function (e.g., second messenger levels, such as, e.g., cell proliferation; inositol phosphate pool changes, transcription using a luciferase-type assay; and others). Some detection methods allow for elimination of a separation step, e.g., a proximity-sensitive detection system.

Qualitative or quantitative analysis of proteins may also be determined by a variety of noncompetitive immunoassay methods. For example, a two-site, solid phase sandwich immunoassay may be used. In this type of assay, a binding composition for the protein, for example an antibody, is attached to a solid support. A second protein-binding composition, which may also be an antibody, and which binds the protein at a different site, is labeled. After binding at both sites on the protein has occurred, the unbound-labeled binding composition is removed and the amount of labeled binding composition bound to the solid phase is measured. The amount of labeled binding composition bound is directly proportional to the amount of protein in the sample.

The immunoassay formats described above employ labeled assay components. The label may be coupled directly or indirectly to the desired component of the assay according to methods well known in the art. A variety of labels and methods may be used. Traditionally, a radioactive label incorporating ³H, ¹²⁵I, ³⁵S, ¹⁴C or ³²P was used. Non-radioactive labels include proteins, which bind to labeled antibodies, fluorophores, chemiluminescent agents, enzymes, and antibodies, which can serve as specific-binding pair members for a labeled protein. The choice of label depends on sensitivity required, ease of conjugation with the compound, stability requirements, and available instrumentation. For a review of various labeling or signal producing systems, which may be used, see U.S. Patent No. 4,391,904.

Antibody binding compositions reactive with a particular protein can also be measured by a variety of immunoassay methods. For a review of immunological and immunoassay procedures applicable to the measurement of antibodies by immunoassay techniques, see Stites and Terr (eds.) Basic and Clinical Immunology (7th ed.) *supra;* Maggio (ed.) Enzyme Immunoassay, supra; and Harlow and Lane Using Antibodies, A Laboratory Manual, *supra.*

In brief, immunoassays to measure antisera reactive with the targeted protein can be either competitive or noncompetitive binding assays. In competitive binding assays, the sample analyte competes with a labeled analyte for specific binding sites on a capture agent bound to a solid surface. Preferably, the capture agent is a purified recombinant protein. Other sources of proteins, including isolated or partially purified naturally occurring protein, may also be used. Noncompetitive assays include sandwich assays, in which the sample analyte is bound between two analyte-specific binding reagents. One of the binding compositions is used as a capture agent and is bound to a solid surface. The second binding composition is labeled and is used to measure or detect the resultant complex by visual or instrument means. A number of combinations of capture agent and labeled binding composition can be used. A variety of different immunoassay formats, separation techniques, and labels can be used similar to those described above for the measurement of a protein.

The ability of the antibody of interest to immunoprecipitate a particular antigen can be assessed by, e.g., Western blot analysis. One of skill in the art would be knowledgeable as to the parameters are modifiable to increase binding of an antibody to an antigen and to decrease background (e.g., by pre-clearing the cell lysate with sepharose beads). Further discussion of immunoprecipitation protocols can be found in, e.g., Ausubel et al, eds., 1994, Current Protocols in Molecular Biology, Vol. 1, John Wiley & Sons, Inc., New York.

An ELISA assay comprises preparing an antigen, coating the well of a 96 well microtiter-plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs, the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. An ordinary artisan can determine without undue experimentation what parameters to adjust, e.g., to increase signal as well as what other variations for an ELISA should be used (see, e.g., Ausubel, et al., eds., 1994, Current Protocols in Molecular Biology, Vol. 1, John Wiley & Sons, Inc., New York).

The binding affinity of an antibody to an antigen and the on- and off-rate of an antibody-antigen interaction can be determined by, e.g., using a competitive binding assay. One non-limiting example is a radioimmunoassay comprising incubating labeled antigen (e.g., using ³H or ¹²⁵I) with an antibody of interest in the presence of increasing amounts of unlabeled antigen, and then detecting the amount of antibody bound to the labeled antigen. The affinity of the antibody of interest for a particular antigen and the binding off-rates can be determined from the data by, e.g., Scatchard plot analysis. Competition with a second antibody can also be determined using, e.g., radioimmunoassays. In this case, the antigen is incubated with antibody of interest conjugated to a labeled compound (e.g., ³H or ¹²⁵I) in the presence of increasing amounts of an unlabeled second antibody.

### Physical Variants

The invention also encompasses polypeptide sequences having substantial amino acid sequence similarity and/or identity with an amino acid sequence described herein. Amino acid sequence similarity, or sequence identity, is determined by optimizing residue matches. This changes when considering conservative substitutions as matches. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine. See also Needleham, et al. (1970) J. Mol. Biol. 48:443-453; Sankoff, et al. (1983) Time Warps, String Edits, and Macromolecules: The Theory and Practice of Sequence Comparison Chapter One, Addison-Wesley, Reading, MA; and software packages from IntelliGenetics, Mountain View, CA; and the University of Wisconsin Genetics Computer Group, Madison, WI.

The invention encompasses, but is not limited to, polypeptide sequences that are functionally related to a polypeptide encoded by a specific sequence identifier of the present application. Functionally related polypeptides include any polypeptide sharing a functional characteristic with a binding composition (e.g., the ability to selectively and/or specifically bind TGF Beta 1 and not bind TGF Beta 2 and/or 3). Such functionally related polypeptides include, without limitation, additions or substitutions of amino acid residues within the amino acid sequence encoded by the sequences described herein; particularly, those that result in a silent change, thus producing a functionally equivalent polypeptide. Amino acid substitutions may be made based on similarity in polarity, charge, solubility, hydrophobicity, hydrophilicity, and/or the amphiphatic nature of the residues involved.

For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

Furthermore, non-classical amino acids or chemical amino acid analogs may be substituted or added into a polypeptide sequence. Non-classical amino acids include, e.g., without limitation, D-isomers of the common amino acids; 2,4-diaminobutyric acid; a-amino isobutyric acid, 4-aminobutyric acid, Abu, 2-amino butyric acid, g-Abu, e-Ahx, 6-amino hexanoic acid, Aid, 2-amino isobutyric acid, 3-amino propionic acid, ornithine, norleucine, norvaline, hydroxyproline, sarcosine, citrulline, homocitrulline, cystic acid, t-butylglycine, t-butylalanine, phenylglycine, cyclohexylalanine, b-alanine, fluoro-amino acids, designer amino acids such as b-methyl amino acids, Ca-methyl amino acids, Na-methyl amino acids, and amino acid analogs in general. Furthermore, the amino acid can be either dextrorotary (D) or levorotary (L).

Additions of peptide moieties to facilitate handling are familiar and routine art techniques. Moreover, a binding composition (including any fragment thereof, and specifically an epitope) can be combined with parts of the constant domain of an immunoglobulin e.g., (IgA, IgE, IgG, IgM) portions thereof (CH 1, CH2, CH3), and any combination thereof including both entire domains and portions thereof), resulting in a chimeric polypeptide. Such fusion proteins can facilitate purification and often are useful to increase the *in vivo* half-life of the protein. For example, this has been demonstrated for chimeric proteins comprising the first two domains of a human CD4 polypeptide and various domains of the constant regions of the heavy or light chains of mammalian immunoglobulins (EP 394,827; Traunecker, et al., 1988 Nature 331:84-6). Fusion proteins with disulfide-linked dimeric structures (due to the IgG domain) can also be more efficient in binding and neutralizing other molecules than a monomeric secreted protein or sole protein fragment (Fountoulakis, et al. 1995 J. Biochem.15 270:3958-64). Enhanced delivery of an antigen across an epithelial barrier to the immune system has been demonstrated for antigens (e.g., insulin) conjugated to an FcyR binding partner such as IgG or Fc fragments (see, e.g., WO 96/22024 and WO 99/104813).

Additionally, a fusion protein can comprise various portions of the constant region of an immunoglobulin molecule together with a human protein (or part thereof). In many cases, the Fc part in a fusion protein is beneficial in therapy and diagnosis, and thus, can result in, e.g., improved pharmacokinetic properties. Alternatively, deleting the Fc part after the fusion protein has been expressed, detected, and purified, may be desired. For example, the Fc portion may hinder therapy and/or diagnosis if the fusion protein is used as an immunogen for immunizations. In drug discovery for example, human proteins have been fused with Fc portions for the purpose of high-throughput screening assays to identify antagonists.

Furthermore, new constructs may be made by combining similar functional domains from other proteins. For example, protein-binding or other segments may be "swapped" between different new fusion polypeptides or fragments. Thus, new chimeric polypeptides exhibiting new combinations of specificities will result from the functional linkage of protein-binding specificities and other functional domains. Additionally, fusion constructions may be generated through the techniques of gene-shuffling, motif-shuffling, exon shuffling, and/or codon shuffling.

**"Substantially pure"** refers to nucleic acid, protein, or polypeptide that are removed from their natural environment and are isolated and/or separated from other contaminating proteins, nucleic acids, and other biologicals. Purity, or **"isolation"** may be assayed by standard methods, and will ordinarily be at least about 50% pure, more ordinarily at least about 60% pure, generally at least about 70% pure, more generally at least about 80% pure, often at least about 85% pure, more often at least about 90% pure, preferably at least about 95% pure, more preferably at least about 98% pure, and in most preferred embodiments, at least 99% pure. Similar concepts apply, e.g., to binding compositions, e.g., such as antibodies of the invention. For example, it may be desirable to purify a polypeptide from recombinant cell proteins or polypeptides.

**"Solubility"** of a protein or polypeptide is reflected by sedimentation measured in Svedberg units, which are a measure of the sedimentation velocity of a molecule under particular conditions. The determination of the sedimentation velocity was classically performed in an analytical ultracentrifuge, but is typically now performed in a standard ultracentrifuge (see, Freifelder 1982 Physical Biochemistry (2d Ed.) W.H. Freeman & Co., San Francisco, CA; and Cantor and Schimmel (1980) Biophysical Chemistry parts 1-3, W.H. Freeman & Co., San Francisco, CA). As a crude determination, a sample containing a putatively soluble polypeptide is spun in a standard full sized ultracentrifuge at about 50K rpm for about 10 minutes, and soluble molecules will remain in the supernatant. A soluble particle or polypeptide will typically be less than about 30S, more typically less than about 15S, usually less than about 10S, more usually less than about 6S, and, in particular embodiments, preferably less than about 4S, and more preferably less than about 3S. Solubility of a polypeptide or fragment depends upon the environment and the polypeptide. Many parameters affect polypeptide solubility, including temperature, electrolyte environment, size and molecular characteristics of the polypeptide, and nature of the solvent. Typically, the temperature at which the polypeptide is used ranges from about 4° C to about 65° C. Usually the temperature at use is greater than about 18° C and more usually greater than about 22° C. For diagnostic purposes, the temperature will usually be about room temperature or warmer, but less than the denaturation temperature of components in the assay. For therapeutic purposes, the temperature will usually be body temperature, typically about 37° C for humans, though under certain situations the temperature may be raised or lowered in situ or *in vitro.* The size and structure of the polypeptide should generally be in a substantially stable state, and usually not in a denatured state. The polypeptide may be associated with other polypeptides in a quaternary structure, e.g., to confer solubility, or associated with lipids or detergents in a manner that approximates natural lipid bilayer interactions.

The solvent will usually be a biologically compatible buffer, of a type used for preservation of biological activities, and will usually approximate a physiological solvent. Usually the solvent will have a neutral pH, typically between about 5 and 10, and preferably about 7.5. On some occasions, a detergent will be added, typically a mild non-denaturing one, e.g., CHS (cholesteryl hemisuccinate) or CHAPS (3-[3-cholamidopropyl)-dimethylammonio]-1-propane sulfonate), or a low enough concentration as to avoid significant disruption of structural or physiological properties of the protein. In a preferred embodiment, the solubility of a binding composition of the invention (pH 5.0-6.0 and 150mM NaCl) is greater than 10, 15, 20, 25, 30, 35, or 40 mg/mL; in a more preferred embodiment, greater than 41, 42, 43, 44, 45, 46, 47, 48, 49, 50 mg/mL; in a still more preferred embodiment, greater than 51, 52, 53, 54, 55, 56, 57, 58, 59 or 60 mg/mL, in an even more preferable embodiment greater than 65, 70, 75, 80, 85, 90, 95, 100, 105, 110, 115, 120, 125, 130, 135, 140, or 150 mg/mL (or in a range between any two such values, which range may or may not include either or both end points).

### Variants

Also disclosed are polypeptides that comprise, or alternatively consist of, an amino acid sequence that is at least: 80%, 85%, 90%, 95%, 96%, 97%, 98%, 99% identical to, e.g., a polypeptide sequence of the invention (or fragments thereof). Determining if a particular sequence exhibits identity to a binding composition sequence can be accomplished using art-known methods.

Typically, in such a sequence comparison, one sequence acts as a reference sequence, to which test sequences are compared. When using a sequence comparison algorithm, test and reference sequences are input into a computer, subsequent coordinates are designated, if necessary, and sequence algorithm program parameters are designated. The sequence comparison algorithm then calculates the percentage sequence identity for a test sequence(s) relative to the reference sequence, based on the parameters of a designated program.

Optimal alignment of sequences for comparison can be conducted, e.g., by the local homology algorithm of Smith and Waterman (1981) Adv. Appl. Math. 2:482, by the homology alignment algorithm of Needlman and Wunsch (1970) J. Mol. Biol. 48:443, by the search for similarity method of Pearson and Lipman (1988) Proc. Nat'l Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), the LASERGENE bioinformatics computing suite, which is produced by DNASTAR (Madison, Wisconsin), by the multiple sequences alignment methods developed by Notredame, et al. (e.g., 3Dcoffee or Tcoffee in, e.g., Nucleic Acids Res. 2004:32(Web Server issue):W37-40; Nucleic Acids Res. 2003 Jul 1;31(13):3503-6; or Pharmacogenomics. 2002 Jan;3(1):131-4)).or by visual inspection (see generally, Ausubel, et al. supra). Other methods for comparing nucleotide or amino acid sequences by determining optimal alignment are well-known (see, e.g., Peruski and Peruski, The Internet and the New Biology: Tools for Genumic and Molecular Research (ASM Press, Inc. 1997); Wu, et al. (eds.), "Information Superhighway and Computer Databases of Nucleic Acids and Proteins," in Methods in Gene Biotechnology, pages 123-151 (CRC Press, Inc. 1997); or Bishop (end.), Guide to Human Genome .Computing, 2nd Edition (Academic Press, Inc. 1998)).

A polypeptide exhibiting or having at least about, e.g., 95% "sequence identity" to another amino acid sequence may include, e.g., up to five amino acid alterations per each 100 amino acid stretch of the test amino acid sequence. In other words, a first amino acid sequence that is at least 95% identical to a second amino acid sequence, can have up to 5% of its total number of amino acid residues different from the second sequence, e.g., by insertion, deletion, or substitution of an amino acid residue. Alterations in amino residues of a polypeptide sequence may occur, e.g., at the amino or carboxy terminal positions or anywhere between these terminal positions, interspersed either individually among residues in the sequence or in one or more contiguous amino residue sections, portions, or fragments within the sequence. As a practical matter, whether any particular polypeptide sequence exhibits at least about: 80%, 85%, 90%, 95%, 96%, 97%, 98%, or 99% similarity to another sequence, for example, such as shown in a Table herein can be determined using art-known methods.

Variants disclosed herein may contain alterations in the coding regions, noncoding regions, or both. Moreover, variants in which 1, 2, 3, 4, 5, 6, 7, 8, 9, or 10 amino acids are substituted, deleted, or added in any combination are also disclosed. Non-naturally occurring variants may be produced by mutagenesis techniques or by direct synthesis using known methods of protein engineering and recombinant DNA technology. Such variants may be generated to improve or alter the characteristics of a binding composition polypeptide (or fragment thereof). For instance, one or more amino acids can be deleted from the N-terminus or C-terminus of a secreted polypeptide of the invention (or fragment thereof) without a substantial loss of biological function. For example, Ron, et al. 1993 J. Biol. Chem. 268:2984-8, reported variant KGF proteins having heparin binding activity even after deleting 3, 8, or 27 amino-terminal amino acid residues. For example, antigenicity and/or immunogenicity can be retained (e.g., the ability of a deletion variant to induce and/or to bind antibodies that recognize a mature form of a polypeptide) when less than the majority of the residues of the secreted form are removed from the N-terminus or C-terminus. Whether a polypeptide lacking N- or C-terminal residues of a protein retains such activities can readily be determined by routine methods described herein or art-known. Thus, the invention also encompasses, e.g., polypeptide variants that show biological activity such as, e.g., immunogenicity, or antigenicity. Such variants include, e.g., deletions, insertions, inversions, repeats, and substitutions selected so as have little effect on activity using general rules known in the art. For example, teachings on making phenotypically silent amino acid substitutions are provided, e.g., by Bowie, et al. (1990) Science 247: 1306-1310.

Besides using conservative amino acid substitutions, other variants of the invention include, e.g., but are restricted to, e.g., (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (e.g., polyethylene glycol), or (iv) fusion of the polypeptide with additional amino acids, such as, e.g., an IgG Fc fusion region peptide, or leader or secretory sequence, or a sequence facilitating purification. All such variants would be within the scope of those skilled in the art of molecular biology given Applicants' teachings herein, e.g., specifying unique polynucleotide and polypeptide sequences.

For example, polypeptide variants containing amino acid substitutions of charged amino acids with other charged or neutral amino acids may produce polypeptides with improved characteristics e.g., such as less aggregation. Aggregation of pharmaceutical formulations both reduces activity and increases clearance due to the aggregate's immunogenic activity (Pinckard, et al. (1967) Clin. Exp. Immunol. 2:331-340; Robbins, et al. (1987) Diabetes 36:838-845; Cleland, et al. (1993) Crit. Rev. Therapeutic Drug Carrier Systems 10:307-377). In a preferred embodiment, a binding composition of the invention formulated in an appropriate pH/buffer system (as described herein or art known) exhibits no significant aggregation following incubation for at least 1, 2, 3, 4, 5, 6, 7, 8, or 9 months under temperature conditions in the range of about 1-10°C, more preferably 2-8°C, even more preferably 3-7°C, and even more preferably 5-6°C.

Also disclosed is a composition that comprises an amino acid sequence of the invention containing at least one amino acid substitution, but not more than 50 amino acid substitutions, even more preferably, not more than 40 amino acid substitutions, still more preferably, not more than 30 amino acid substitutions, and still even more preferably, not more than 20 amino acid substitutions, nor more than 15 amino acid substitutions. Also disclosed is a peptide or polypeptide that has an amino acid sequence that comprises an amino acid sequence of the invention, which contains at least: one, but not more than: 10, 9, 8, 7, 6, 5, 4, 3, 2, or 1 amino acid substitutions. The number of additions, substitutions, and/or deletions in an polypeptide sequence of the invention or fragments thereof may be at least: 1, 2, 3, 4, 5, 6, 7, 8, 9, 10, 11, 12, 13, 14, 15, 16, 17, 18, 19, 20, 21, 22, 23, 24, 25, 10-50, or 50-150; wherein conservative amino acid substitutions are more preferable than non-conservative substitutions.

### Therapeutic Uses

This invention also provides reagents with useful therapeutic value. Therapeutic binding compositions of the invention include, e.g., without limitation, antibody-binding compositions of the invention (including fragments, analogs and derivatives thereof as described herein) and nucleic acid molecules encoding them (including fragments, analogs and derivatives thereof and anti-idiotypic antibodies as descried herein). Such an antibody can be used to modulate, treat, inhibit, ameliorate, or prevent diseases, disorders, or conditions associated with aberrant expression and/or activity of TGF Beta 1 (or fragment thereof), including, e.g., without limitation, any one or more of the diseases, disorders, syndromes or conditions described herein. The treatment, amelioration, and/or prevention of diseases, disorders, or conditions associated with aberrant expression and/or activity of TGF Beta 1 include, e.g., without limitation, ameliorating symptoms associated with those diseases, disorders, or conditions.

For example, a disease or disorder associated with abnormal expression or abnormal signaling by TGF Beta 1 is a target for an antagonist of TGF Beta 1 such as a binding composition of the invention. The invention encompasses binding composition-based therapies that involve administering a binding composition to an animal, preferably a mammal, most preferably a primate (e.g., a human), to modulate, treat, inhibit, effect, or ameliorate one or more of the disclosed diseases, disorders, or conditions described herein. For example, while not being bound by theory, antibodies specific for human TGF Beta have been shown to be effective in animal models for the treatment of diseases where the TGF receptor (TGFR) is over expressed. Antiserum against TGF Beta have been shown to be effective in the treatment of glomerulonephritis (Border, et al. 1990 Nature 346:371-4); and lung fibrosis (Giri, et al. 1993 Thorax 48:959-66). Consequently, new binding compositions of the invention with improved characteristics would also be effective in the amelioration of conditions, states, or diseases, such as, e.g., fibrotic diseases and conditions associated with TGF Beta 1.

Recombinant and/or isolated binding compositions of the invention, such as, e.g., antibodies, can be purified and administered to a subject for treatment. These reagents can be combined for use with additional active or inert ingredients, e.g., in conventional pharmaceutically acceptable carriers or diluents, e.g., immunogenic adjuvants, along with physiologically innocuous stabilizers and excipients. These combinations can be sterile filtered and placed into dosage forms as by lyophilization in dosage vials or storage in stabilized aqueous preparations. This invention also contemplates use of antibodies or binding fragments thereof, including forms that are not complement binding.

Another therapeutic approach included within the invention involves direct administration of reagents, formulations, or compositions by any conventional administration techniques (such as, e.g., without limit, local injection, inhalation, or systemic administration) to a subject. The invention also provides a pharmaceutical pack or kit comprising one or more containers filled with one or more of the ingredients of the compositions of the invention and instructions such as, e.g., for disposal (typically, in a form prescribed by a governmental agency regulating the manufacture, use or sale of pharmaceuticals or biological products). Methods for administration include intravenous, intraperitoneal, or intramuscular administration, transdermal diffusion, and others. Pharmaceutically acceptable carriers will include water, saline, buffers, and other compounds described, e.g., in the Merck Index, Merck & Co., Rahway, NJ.

Other abnormal developmental conditions encompassed herein are known in cell types shown to possess TGF Beta 1 mRNA by northern blot analysis (see, e.g., Berkow (ed.) The Merck Manual of Diagnosis and Therapy, Merck & Co., Rahway, N.J.; Thorn et al. Harrison's Principles of Internal Medicine, McGraw-Hill, N.Y.; and Rich (ed.) Clinical Immunology; Principles and Practice, Mosby, St. Louis (cur. ed.); and below). Developmental or functional abnormalities, (e.g., of the neuronal, immune, or hematopoetic system) cause significant medical abnormalities and conditions which may be susceptible to prevention or treatment using compositions provided herein.

Another therapeutic approach included within the invention involves direct administration of reagents, formulations, or compositions by any conventional administration techniques (such as, e.g., without limit, local injection, inhalation, or systemic administration) to a subject. The reagents, formulations, or compositions encompassed may be targeted by any method described herein or art-known. The actual dosage of reagent, formulation, or composition that modulates a disease, disorder, condition, syndrome, etc., depends on many factors, including the size and health of an organism, however one of one of ordinary skill in the art can use the following teachings describing methods and techniques for determining clinical dosages (see, e.g., Spilker (1984) Guide to Clinical Studies and Developing Protocols, Raven Press Books, Ltd., New York, pp. 7-13, 54-60; Spilker (1991) Guide to Clinical Trials, Raven Press, Ltd., New York, pp. 93-101; Craig and Stitzel (eds. 1986) Modem Pharmacology, 2d ed., Little, Brown and Co., Boston, pp. 127-33; Speight (ed. 1987) Avery's Drug Treatment: Principles and Practice of Clinical Pharmacology and Therapeutics, 3d ed., Williams and Wilkins, Baltimore, pp. 50-56; Tallarida, et al. (1988) Principles in General Pharmacology, Springer-Verlag, New York, pp. 18-20; and U.S. Pat. Nos. 4,657,760; 5,206,344; or 5,225,212.). Generally, in the range of about between 0.5 fg/ml and 500ug/ml inclusive final concentration is administered per day to a human adult in any pharmaceutically acceptable carrier. Furthermore, animal experiments provide reliable guidance for the determination of effective doses for human therapy. Interspecies scaling of effective doses can be performed following art known principles (e.g., see, Mordenti and Chappell (1989) "The Use of Interspecies Scaling in Toxicokinetics," in Toxicokinetics and New Drug Development; Yacobi, et al. (eds.) Pergamon Press, NY).

Effective doses can also be extrapolated using dose-response curves derived from in vitro or animal-model test systems. For example, for antibodies a dosage is typically 0.1 mg/kg to 100 mg/kg of a recipient's body weight. Preferably, a dosage is between 0.1 mg/kg and 20 mg/kg of a recipient's body weight, more preferably 1 mg/kg to 10 mg/kg of a recipient's body weight. Generally, homo-specific antibodies have a longer half-life than hetero-specific antibodies, (e.g., human antibodies last longer within a human host than antibodies from another species, e.g., such as a mouse, probably, due to the immune response of the host to the foreign composition). Thus, lower dosage of human antibodies and less frequent administration is often possible if the antibodies are administered to a human subject. Furthermore, the dosage and frequency of administration of antibodies of the invention may be reduced by enhancing uptake and tissue penetration (e.g., into the brain) by using modifications such as, e.g., lipidation.

The invention also provides a pharmaceutical pack or kit comprising one or more containers filled with one or more of the ingredients of the compositions of the invention and instructions such as, e.g., for disposal (typically, in a form prescribed by a governmental agency regulating the manufacture, use or sale of pharmaceuticals or biological products).

The quantities of reagents necessary for effective treatment will depend upon many different factors, including means of administration, target site, physiological state of the patient, and other medicaments administered. Thus, treatment dosages should be titrated to optimize safety and efficacy. Typically, dosages used in vitro may provide useful guidance in the amounts useful for in situ administration of these reagents. Animal testing of effective doses for treatment of particular disorders will provide further predictive indication of human dosage. Various considerations are described, e.g., in Gilman, et al. (eds.) (1990) Goodman and Gilman's: The Pharmacological Bases of Therapeutics (8th ed.) Pergamon Press; and (1990) Remington's Pharmaceutical Sciences (17th ed.) Mack Publishing Co., Easton, PA. Methods for administration are discussed therein and below, e.g., for oral, intravenous, intraperitoneal, or intramuscular administration, transdermal diffusion, and others. Pharmaceutically acceptable carriers will include water, saline, buffers, and other compounds described, e.g., in the Merck Index, Merck & Co., Rahway, NJ. Dosage ranges would ordinarily be expected to be in amounts lower than 1 mM concentrations, typically less than about 10 µM concentrations, usually less than about 100 nM, preferably less than about 10 pM (picomolar), and most preferably less than about 1 fM (femtomolar), with an appropriate carrier. Slow release formulations, or a slow release apparatus will often be utilized for continuous administration.

Binding compositions may be administered directly to the host to be treated or, depending on the size of the compounds, it may be desirable to conjugate them to carrier proteins such as ovalbumin or serum albumin before their administration. Therapeutic formulations may be administered in any conventional dosage formulation. While it is possible for the active ingredient to be administered alone, it is preferable to present it as a pharmaceutical formulation. Formulations typically comprise at least one active ingredient, as defined herein, together with one or more acceptable carriers thereof. Each carrier should be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by any methods well known in the art of pharmacy. See, e.g., Gilman, et al. (eds.) (1990) Goodman and Gilman's: The Pharmacological Bases of Therapeutics (8th ed.) Pergamon Press; and (1990) Remington's Pharmaceutical Sciences (17th ed.) Mack Publishing Co., Easton, PA; Avis, et al. (eds.) (1993) Pharmaceutical Dosage Forms: Parenteral Medications Dekker, NY; Lieberman, et al. (eds.) (1990) Pharmaceutical Dosage Forms: Tablets Dekker, NY; and Lieberman, et al. (eds.) (1990) Pharmaceutical Dosage Forms: Disperse Systems Dekker, NY. The treatment of this invention may be combined with or used in association with other therapeutic agents such as, e.g., ACE inhibitors.

The invention also provides a pharmaceutical composition. Such a composition comprises, e.g., a therapeutically effective amount of a composition of the invention in a pharmaceutically acceptable carrier. As used herein, the term "pharmaceutically acceptable carrier" means a carrier approved by a federal regulatory agency of the United States of America, or a regulatory/administrative agency of a state government of the United States or a carrier that is listed in the U.S. Pharmacopoeia or other pharmacopoeia; which is generally recognized by those in the art for use in an animal, e.g., a mammal, and, more particularly, in a primate, e.g., a human primate.

The term "carrier" as used herein refers to a diluent, adjuvant, excipient, or vehicle that is administered with a composition of the invention. A pharmaceutical carrier typically can be a sterile liquid, such as water or oils, (including those of petroleum, animal, vegetable, or synthetic origin, e.g., such as peanut oil, soybean oil, mineral oil, sesame oil and the like). Typically, sterile water is a preferred carrier when a pharmaceutical composition is administered intravenously. Saline solutions and aqueous dextrose and glycerol solutions can also be employed as liquid carriers, particularly for injectable solutions.

Suitable pharmaceutical excipients include, e.g., without limit, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. A composition of the invention, if desired, can also contain minor amounts of wetting or emulsifying agents, or pH buffering agents.

A composition of the invention can be in a solution, suspension, emulsion, tablet, pill, capsule, powder, sustained-release formulation, etc., or it can be formulated as a suppository (with traditional binders, and/or carriers, e.g., such as triglycerides). Additional examples of suitable pharmaceutical carriers are described in the current edition of Remington's Pharmaceutical Sciences by E.W. Martin. Such formulations will contain a therapeutically effective amount of a composition of the invention, preferably in purified form, together with a suitable amount of carrier to provide for proper administration to a subject. Traditionally, a formulation will suit the mode of administration.

In a preferred embodiment, a composition is formulated in accordance with routine procedures as a pharmaceutical composition adapted for intravenous administration to, e.g., a human. Typically, compositions for intravenous administration are solutions in sterile isotonic aqueous buffer. Where necessary, the composition may also include, e.g., a solubilizing agent and a local anesthetic such as lidocaine to promote comfort at the injection site. Generally, ingredients are supplied either separately or mixed in unit dosage form, e.g., as a dry lyophilized powder or water free concentrate in a hermetically sealed container (such as an ampoule or sachet indicating the quantity of active agent). Where a composition is to be administered by infusion, it can be dispensed using an infusion bottle containing sterile pharmaceutical grade water or saline. Where a composition is administered by injection, an ampoule of sterile water for injection or saline can be provided so that the ingredients may be mixed before administration.

Compositions of the invention can be formulated as neutral or salt forms. Pharmaceutically acceptable salts include, e.g., without limit, anionic salts (such as those derived from hydrochloric, phosphoric, acetic, oxalic, tartaric acids, etc.,) and cationic salts, (e.g., such as those derived from sodium, potassium, ammonium, calcium, ferric hydroxides, isopropylamine, triethylamine, 2-ethylamino ethanol, histidine, procaine, etc).

### Fibrotic Diseases, Disorders, or Conditions

A binding composition, e.g., such as an antibody, is useful in the treatment of conditions associated with fibrotic diseases. Accumulation of components of the extracellular matrix (ECM) or the replacement of normal cellular material with ECM components in a wide variety of cells, tissues, and organs can result in disease-producing fibrosis. Progressive fibrosis can be fatal, leading to end-organ failure in multiple organs, such as, for example the Kidney. Both, preclinical and clinical data indicate that TGF-Beta 1 is a major contributor to matrix protein deposition in interstitial fibrosis, and is involved in the initiation and progression of a number of associated fibrotic disease states, including renal fibrosis — which is common to all forms of chronic renal disease (CRD). The extent of renal fibrosis positively correlates with progression to chronic renal failure (CRF, also known as end-stage renal disease (ESRD)), which can result in chronic dialysis or renal transplantation, and death. TGF-Beta 1 is the cytokine most consistently linked both experimentally and by association in human and animal studies with such fibrotic processes.

The profound effects of TGF-Beta 1 on the ECM including its role in the stimulation of the synthesis and the inhibition of degradation of extracellular matrix components have been the subject of numerous reviews (see, e.g., Rocco & Ziyadeh 1991 in Contemporary Issues in Nephrology v.23, "Hormones, autocoids and the kidney" Ed. Jay Stein, Churchill Livingston, New York pp.391-410; Roberts, et al. 1988 Rec. Prog. Hormone Res. 44:157-97). TGF-Beta 1 can induce accumulation of ECM in multiple and cooperative ways, for example, TGF-Beta 1 stimulates mRNA expression and protein production of key ECM components including type I collagen, type IV collagen, fibronectin, and laminin (Sharma & Ziyadeh 1997 Semin Nephrol 17:80-92). At the same time, TGF-Beta 1 impedes degradation of ECM by inhibiting production of proteases (such as, e.g., plasminogen activator, collagenase, elastase, and stromelysin) that digest the matrix and by activating inhibitors of those proteases such as, e.g., tissue inhibitors of metalloproteinases and plasminogen activator inhibitor 1 (Sharma & Ziyadeh 1995 Kidney Int 51:S34-6). TGF-Beta 1 also up-regulates integrins and cell surface receptors for ECM, thereby enhancing the ability of cells to interact with specific ECM proteins (Heino, et al. 1989 J Biol Chem 264:380-8). Additionally, TGF-Beta 1 has a potent chemotactic property to attract ECM cells such as fibroblasts and phagocytic cells (Reibman, et al. 1991 PNAS 88:6805-9). Moreover, TGF-Beta 1 has a peculiar ability to induce its own expression potentially amplifying aberrant fibrotic processes (Kim, et al. 1990 Mol Cell Biol 10:1492-7).

TGF-Beta 1 is implicated in the following diseases, syndromes, and/or conditions:
- ***Kidney disease*** ― such as, e.g., chronic renal disease (CRD); chronic renal failure (CRF); end stage renal disease (ESRD); glomerulonephritis (GN), including, e.g., mesangial proliferative GN, mesangiocapillary GN, membranous GN, focal and segmental GN, immune GN, and crescentic GN; glomerulosclerosis; nephrosclerosis, membranous nephropathy, immunoglobulin A (IgA) nephropathy; renal interstitial fibrosis; focal segmental glomerular sclerosis, renal fibrosis in transplant patients receiving cyclosporin; chronic renal transplant rejection; HIV-associated nephropathy;
   renal cell hypertrophy; tubulointerstitial fibrosis accompanying chronic kidney diseases,
   including that resulting from urinary tract obstruction or analgesic associated nephropathy or following an episode of acute renal failure, such as acute renal failure resulting from renal ischemia (Spurgeon et al. Am. J Physiol Renal Physiol 288:F568-F577, 2005); renal thrombotic microangiopathy such as associated with e.g., glomerular endothelial cell injury or other microvascular endothelial cell injuries, such as associated with preeclampsia, endotoxemia, and radiation exposure; renal vasculitis; focal necrotizing glomerulonephritis; diabetic nephropathy [TGF Beta 1 is associated with CRF through complex and diverse events that impact the majority of cells of the kidney (Bottinger, 2002, J. Am. Soc. Nephrol. 13, 2600). These events ultimately result in both tubulointerstitial fibrosis and glomerulosclerosis leading to loss of nephron function and ultimately chronic renal failure. Of the three TGF-Beta isoforms, TGF-Beta 1 appears to predominant in mediating the progression of chronic renal disease, not only as being the most predominantly expressed isoform, but also because both TGF-Beta 2 and -Beta 3 appear to mediate their effects through up-regulation of TGF-Beta 1 expression (Yu, 2003 Kid. Int. 64:844). Both *in vitro* and *in vivo* studies implicate TGF-Beta 1 in the pathogenesis of diabetic kidney disease including complications associated with type 1 or type 2 diabetes mellitus such as, e.g., glomerulosclerosis and tubulointerstitial fibrosis (Ziyadeh 1998 Cur. Pract. Med. 1:87-9). Antiserum against TGF beta is effective in the treatment of glomerulonephritis (Border et al. 1990 Nature 346:371-4)]; fibrotic renal disease [TGF-Beta 1 mediates renal cellular hypertrophy, another characteristic of diabetic nephropathy by interfering with normal regulation of the cell cycle by inducing cyclin-dependent kinase inhibitors such as p27Kip1 and p21Cip1 (Wolf & Ziyadeh 1999 Kidney Int 56:393-405). These inhibitors also are increased by high glucose and the diabetic state (Wolf, et al. 2001 Am J Pathol;158:1091-1100; Wolf, et al. 1999 Diabetologia 42:1425-32; Wolf, et al. 1997 Am J Physiol 273:F348-56). They suppress the activity of cyclin-dependent kinases, predominantly cyclin-dependent kinase 2/cyclin E kinase (Liu & Preisig 1999 Am J Physiol 277:F186-94), thus inhibiting the phosphorylation of retinoblastoma protein and arresting a cell in the late G1 phase. The cell enters a period of protein synthesis without DNA replication and undergoes hypertrophy. Thus, TGF-Beta 1 causes changes at the cellular level that translate into the pathophysiologic features of diabetic nephropathy.]; experimental diabetic nephropathy; hypertensive nephrosclerosis [Since the structure and filtration properties of the glomerulus are largely determined by the extracellular matrix composition of the mesangium and glomerular membrane, it is not surprising that TGF-Beta 1 has profound effects on the kidney. TGF-Beta 1 mediates pathologic changes of diabetic kidney disease (Ziyadeh 1998 Cur Prac Med 1:87-89). The accumulation of mesangial matrix in proliferative glomerulonephritis (Border, et al. 1990 Kidney Int. 37:689-695) and diabetic nephropathy (Mauer, et al. (1984) J. Clin. Invest. 74:1143-1155) are clear and dominant pathological features of the diseases. TGF-Beta 1 levels are elevated in human diabetic glomerulosclerosis (advanced neuropathy) (Yamamoto, et al. 1993 Proc. Natl. Acad. Sci. 90:1814-1818). TGF-Beta 1 has also been found to be an important mediator in the genesis of renal fibrosis in a number of animal models (Phan, et al. 1990 Kidney Int. 37:426; Okuda, et al. 1990 J. Clin. Invest. 86:453). Suppression of experimentally induced glomerulonephritis in rats has been demonstrated by using antiserum against TGF-Beta (Border, et al. 1990 Nature 346:371) and by an extracellular matrix protein, decorin, which can bind TGF-Beta 1 (Border, et al. 1992 Nature 360:361-363; see, also, e.g., Border & Noble 1994 N. Engl. J. Med. 331:1286-92; Border, et al. 1989 Semin. Nephrol. 9:307-17; Han, et al. 2000 Am J Physiol Renal Physiol 278:F628-34; and Ziyadeh, et al. 2000 PNAS 97:8015-20)]. Consequently, a binding composition of the invention would be useful in for the treatment, amelioration, modulation, diagnosis, and/or inhibition of a disease, disorder, syndrome, and/or condition as described above.
- ***Liver disease*** ― such as, e.g., cirrhosis; hepatic fibrosis; [Hepatic fibrosis is a common response to hepatocellular necrosis or injury, which may be induced by a wide variety of agents, e.g., any process disturbing hepatic homeostasis (especially inflammation, toxic injury, or altered hepatic blood flow) and infections of the liver (viral, bacterial, fungal, and parasitic). Numerous storage disorders resulting from inborn errors of metabolism are often associated with fibrosis, including lipid abnormalities (Gaucher's disease); glycogen storage diseases (especially types III, IV, VI, IX, and X); 1-antitrypsin deficiency; storage of exogenous substances, as seen in iron-overload syndromes (hemochromatosis) and copper storage diseases (Wilson's disease); accumulation of toxic metabolites (as in tyrosinemia, fructosemia, and galactosemia); and peroxisomal disorders (Zellweger syndrome). Numerous chemicals and drugs cause fibrosis, especially alcohol, methotrexate, isoniazid, oxyphenisatin, methyldopa, chlorpromazine, tolbutamide, and amiodarone. Disturbances of hepatic circulation (e.g., chronic heart failure, Budd-Chiari syndrome, veno-occlusive disease, portal vein thrombosis) and chronic obstruction to bile flow can lead to fibrosis. Lastly, congenital hepatic fibrosis is an autosomal recessive malformation.

The majority of people worldwide have chronic viral hepatitis, or steatohepatitis associated with either alcohol or obesity, but other fibroritc-inducing insults include, for example, parasitic disease (e.g., schistosomiasis), autoimmune attack on hepatocytes or biliary epithelium, neonatal liver disease, metabolic disorders including Wilson's hemochromatosis and a variety of storage diseases, chronic inflammatory conditions (e.g. sarcoidosis), drug toxicity (e.g. methotrexate or hypervitaminosis A), and vascular derangements, either congenital or acquired.

Hepatic stellate cells (HSC) are an important cellular source of ECM in liver fibrosis (Li & Friedman 1999 J. Gastroenterol. Hepatol. 14:618-33). HSC reside in the perisinusoidal space of Disse, which separates hepatocytes from the sinusoidal endothelium. Liver insults, as described, activate normally quiescent HSC leading to their subsequent proliferation and activation. Activated HSC undergo a subsequent phenotypic transdifferentiation to contractile myofibroblasts (MFB), which express smooth muscle actin and an excess of ECM molecules seen in liver fibrosis. The transdifferentiation of HSC into MFB is due to over expression of TGF-β1 in its role as a key regulator of the liver's fibrotic response to stress and injury (Gressner, et al. 2002 Front Biosci. 7:d793-807). TGF-β1 is the strongest known inducer of fibrogenesis in the effector cells of hepatic fibrosis (Schuppan, et al. 2003 Cell Death Differ. 10 Supl 1:S59-67). Accordingly, tissue and serum levels of active TGF-β1 are elevated in liver fibrosis and overexpression of TGF-β1 in transgenic mice and application of exogenous TGF-β1 have been shown to induce organ fibrosis (Kanzler, et al. 1999 Am. J. Physiol. 276:G1059-68; Sanderson, et al. 1995 PNAS 2572-76). Further, experimental fibrosis can be inhibited by anti-TGF-β1 treatments, e.g. with neutralizing antibodies or soluble TGF receptors (George, et al. 1999 PNAS 12719-24; Qi, et al. 1999 PNAS 2345-49). The observed TGF-β1 expression of activated HSC/MFB, the potency of TGF-β1 to upregulate ECM expression, and the expression of TGF receptors on HSC has led to a widely accepted model in which the persistent auto-/paracrine stimulation of activated HSC/MFB by TGF-β1 is the key fibrogenic response in promoting organ fibrosis. Consequently, the reduction in TGF-β1 is predicted to reduce liver fibrogenesis (Wu & Zern 2000 J. Gastroenterol. 35:665-72)];
- ***Lung disease*** ― such as, e.g.: pulmonary fibrosis (Giri et al. Thorax 48, 959-966, 1993); idiopathic pulmonary fibrosis; adult respiratory distress syndrome; cryptogenic organizing pneumonia, bronchiolitis obliterans (BO), bronchiolitis obliterans (transplant associated); drug induced lung disease; irradiation induced lung disease; localized fibrosis around airways in asthma and emphysema; hypersensitivity pneumonitis; cryptogenic organizing pneumonia (COP); chronic obstructive pulmonary disease (COPD); acute interstitial pneumonitis (AIP); asbestosis; interstitial pulmonary fibrosis associated with autoimmune disorders, such as systemic lupus erythematosus and scleroderma, chemical contact, or allergies; asthma; newborn chronic lung disease (CLD) [Dysregulated expression of TGF-β1 plays an important role in pathogenesis of aberrant airway development in a number of chronic lung diseases including asthma, pulmonary fibrosis, and newborn chronic lung disease (CLD) (Holgate, et al. 2001 Int Arch Allergy Immunol 124 1-3:253-8; Khalil, et al. 2001 Thorax 56 12:907-15; Lecart, et al. 2000 Biol Neonate 77 4:217-23; Pulleyn, et al., 2001 Hum Genet 109 6:623-7; Sagara, et al. 2002 J Allergy Clin Immunol 110 2:249-54; Sheppard, 2001 Chest 120 1 Suppl:49S-53S; Strieter, 2001 Chest 120 1 Supp:77S-85S; Toti, et al. 1997 Pediatr Pulmonol 24 1:22-8). Overexpression of active TGF-β1 along alveolar surfaces of mice leads to a vigorous fibrotic response, and inhibition of TGF-β1 by antibodies or decoys abrogates bleomycin-induced fibrosis (Kelly, et al. 2003 Cur. Pharm. Des. 9:3949). Moreover, microarray analysis of whole lung mRNA shows that most of the known TGF-β1-inducible genes are upregulated during experimentally-induced fibrosis (Kaminski, et al. 2000 PNAS 97:1778-83)];
- ***Cardiovascular disease*** ― atherosclerosis; [TGF-β is associated with atherosclerosis (see, e.g., T.A. McCaffrey: 2000 "TGF-βs and TGF-β Receptors in Atherosclerosis" Cytokine and Growth Factor Reviews 11:103-114)]; hypertrophic heart growth (Schultz, et al. 2002 J Clin Invest. 109 (6): 787-96; Brand & Schneider 1995 J. Mol. Cell Cardiol. 27:518); progressive systemic sclerosis [paralleling the increase in TGF-β1 levels in the aging vasculature are marked increases in fibronectin levels (Li et al. 1999). These changes undoubtedly contribute to the shift from an elastic to a fibrotic/stiff vessel]; mechanically-induced microvascular fibrosis; sarcoidosis; ventricular fibrosis; radiation-induced ischemic heart disease [a significantly higher risk of death due to ischemic heart disease has been reported for patients treated with radiation for Hodgkin's disease and breast cancer. Certain cytokines and growth factors, such as TGF-β1, may stimulate radiation-induced endothelial proliferation, fibroblast proliferation, collagen deposition, and fibrosis leading to advanced lesions of atherosclerosis]; arterial injury (Wolf, et al. 1994 J. Clin. Invest. 93, 1172-8); chronic venous insufficiency (CVI); restenotic lesion after percutaneous transluminal coronary angioplasty (PTCA) or stenting; Hermansky-Pudlak syndrome; polymyositis; scleroderma; dermatomyositis; eosinophilic fascitis; morphea, or those associated with the occurrence of Raynaud's syndrome; perivascular fibrosis of intramural coronary vasculature of non-infarcted myocardium; injury-induced hyperplasias such as, e.g., restenosis; atherosclerosis fibrosis with collagen vascular disease [TGF-β1 may be a factor in the progressive thickening of the arterial wall which results from the proliferation of smooth muscle cells and deposition of extracellular matrix in the artery after balloon angioplasty. The diameter of the restenosed artery may be reduced 90% by this thickening, and since most of the reduction in diameter is due to extracellular matrix rather than smooth muscle cell bodies, it may be possible to open these vessels to 50% simply by reducing extensive extracellular matrix deposition. In uninjured pig arteries transfected in vivo with a TGF-β1 gene, TGF-β1 gene expression was associated with both extracellular matrix synthesis and hyperplasia (Nabel, et al. 1993 PNAS 90:10759-63)]; histiocytosis X (eosinophilic granuloma);
- ***Soft Tissue Fibroses*** ― (Border & Ruoslahti 1992 J Clin Inv 90:1-7; Border & Noble 1994 N Engl J Med 331:1286-91); erectile dysfunction (Moreland, R. 1998 Int J Impot Res 10:113-20); rheumatoid arthritis (Wahl, et al 1993 J. Exp. Medicine 177, 225-30);
- ***Wound Healing*** ― dermal scarring; burn injury; hypertrophic scarring; keloid formation; conjunctival scarring (Cordeiro MF, 2003 Clin Sci (Lond). 104(2): 181-7) [Treatment of fetal wounds with different concentrations of TGF-β1 caused marked scarring of these wounds, showing a direct involvement of TGF-β1 in cutaneous scarring (Sullivan, et al. 1995 J Pediatr Surg 30:198-203). Neutralizing TGF-β1 antibodies injected into the margins of healing wounds in rats have been shown to inhibit scarring without interfering with the rate of wound healing or the tensile strength of the wound. At the same time, there was reduced angiogenesis, reduced number of macrophages and monocytes in the wound, and a reduced amount of disorganized collagen fiber deposition in the scar tissue (Shah, et al. 1992 Lancet 339:213-4; Shah et al. 1994 J. Cell Science 107:1137-57; Shah et al. 1995 J Cell Sci. 108:985-1002). Reduced scarring response occurs in mouse wounds topically treated with antisense TGF-β1 (Choi, et al. 1996 Immunol Cell Biol 74:144-50). Topical application of a synthetic TGF-β1 antagonist reduced scarring in porcine bum and excisional wounds as well as in rabbit skin excisions (Huang, et al. 2002 FASEB J 16:1269-70; Werner & Grose 2003 Physiol Rev 83(3): 835-70)];
- ***Inflammatory Disease*** ― inflammatory bowel disease (IBD) such as, e.g., Crohn disease (CD) and ulcerative colitis (UC) [intestinal inflammation is dependent on the balance between inflammatory cytokines, especially IFN-γ, and TGF-β1 activity (Strober, et al. 1997 Immunol. Today 18:61-4; Neurath, et al. 1996 J. Exp. Med. 183, 2605-16; Ludviksson, et al. 1997 J. Immunol. 159; Boirivant, et al. 1998 J. Exp. Med. 188, 1929-39; Powrie, et al. 1996 J. Exp. Med. 183, 2669-74). Immunologically mediated tissue damage in the gut is associated with increased production of proinflammatory cytokines, which activate the transcription factor NF kappa-B in a variety of different cell types.]; rheumatoid arthritis (RA); synovial hyperplasia [TGF-β1 is elevated in human rheumatoid arthritis synovial fluid (Lotz, et al. 1990 J. Immunol. 144:4189-94). Excessive TGF-β1 forms painful bony joint outgrowths called osteophytes (van Beuningen, et al. 1994 Lab. Invest. 71:279-90), synovial hyperplasia, and inflammation in RA (Hamilton, et al. 1991 PNAS 88:7180-4). Inhibition of endogenous TGF-β1 in a murine arthritis model results in prevention of osteophyte formation and impairs cartilage repair, suggesting its role in these pathologic events (Scharstuhl, et al. 2002 Immunol. 169:507-14)];
- ***Cellular Proliferative Diseases** ―* Numerous data demonstrate that TGF-β1 not only has transforming potential, but can also drive malignant progression, invasion, and metastasis both *in vitro* and *in vivo* (Derynck, et al. 2001 Nat. Genet 29:117-29; Cui, et al. 1996 Cell 86:531-42). Examples of hyperproliferative states, diseases, disorders, syndromes, and/or conditions include, e.g., without limitation, a neoplasm of the colon, abdomen, bone, breast, digestive system, liver, pancreas, peritoneum, endocrine system (e.g., an adrenal gland, a parathyroid gland, the pituitary, the testicles, the ovary, the thymus, or the thyroid), eye, head, neck, nervous system (central or peripheral), the lymphatic system, pelvis, skin, spleen, thorax, and urogenital system. Similarly, other hyperproliferative conditions include, e.g., without limit hypergammaglobulinemia, lymphoproliferative conditions, paraproteinemias, purpura, sarcoidosis, Hamartoma, Sezary Syndrome, Waldenstron's Macroglobulinemia, Gaucher's Disease syndrome, histiocytosis, and other hyperproliferative states.

Despite the tumor suppressor activity of TGF-β1, tumor cells often show increased production of this growth factor (Derynck, et al. 1987 Cancer Res. 47:707-12; Dickson, et al. 1987 PNAS 84:837-41), and considerable evidence documents its tumor-promoting role through its effects on tumor cell invasion and changes in the tumor microenvironment. It is now clear that TGF-β1 can act as both a tumor suppressor and as a significant stimulator of tumor progression, invasion, and metastasis, At early stages of tumorigenesis, such as when the tumor is still benign, TGF-β acts directly on the cancer cell to suppress tumor outgrowth. As the tumor progresses, however, genetic and/or biochemical changes allow TGF-β1 to stimulate tumor progression by its pleiotropic activities on both the cancer cell per se and on non-malignant stromal cell types of the tumor. The stimulation of invasion and metastasis by TGF-β1 might be of greater clinical consequence than its tumor-suppressive role, as the majority of human tumors retain a functional TGF-β signaling pathway (Akhurst & Derynck 2001 Trends Cell Biol. 11:S44-51).

There is a substantial body of evidence that excess TGF-β1 production in cell proliferative diseases is associated with poor prognosis (Tsushima et al. 1996 Gastroenterology 110:375-82; Adler et al. 1999 J. Urol. 161:182-7). There are several types of cancer where TGF-β1 produced by the tumor may be deleterious. MATLyLu rat prostate cancer cells (Steiner & Barrack 1992 Mol. Endocrinol 6:15-25) and MCF-7 human breast cancer cells (Arteaga, et al. 1993 Cell Growth and Differ. 4:193-201) became more tumorigenic and metastatic after transfection with a vector expressing mouse TGF-β1. TGF-β1 is associated with angiogenesis, metastasis, and poor prognosis in human prostate and advanced gastric cancer (Wikstrom, P., et al. (1998) Prostate 37:19-29; Saito, H. et al. (1999) Cancer 86: 1455-62). In breast cancer, poor prognosis is associated with elevated TGF-β1 (Dickson, et al. 1987 PNAS 84:837-41; Kasid, et al. 1987; Cancer Res. 47:5733-8; Daly, et al. 1990 J. Cell Biochem. 43:199-211; Barrett-Lee, et al. 1990 Br. J Cancer 61:612-7; King, et al. 1989 J. Steroid Biochem. 34:133-8; Welch, et al. 1990 PNAS 87:7678-82; Walker, et al. 1992 Eur. J. Cancer 238:641-4) and induction of TGF-β1 by tamoxifen treatment (Butta, et al. 1992 Cancer Res. 52:4261-4) is associated with failure of tamoxifen treatment for breast cancer (Thompson, et al. 1991 Br. J. Cancer 63:609-14). Anti-TGF-β1 antibodies inhibit the growth of MDA-231 human breast cancer cells in athymic mice (Arteaga, et al. 1993 J. Clin. Invest. 92:2569-76), a treatment that is correlated with an increase in spleen natural killer cell activity. CHO cells transfected with latent TGF-β1 also show decreased NK activity and increased tumor growth in nude mice (Wallick, et al. 1990 J. Exp. Med. 172:1777-84). Thus, TGF-β1 secreted by breast tumors may cause an endocrine immune suppression. High plasma concentrations of TGF-β1 indicate poor prognosis for patients with advanced breast cancer (Anscher, et al. 1993 N. Engl. J. Med. 328:1592-8). Patients with high circulating TGF-β1 before high dose chemotherapy and autologous bone marrow transplantation are at high risk for hepatic veno-occlusive disease (15-50% of all patients with a mortality rate up to 50%) and idiopathic interstitial pneumonitis (40-60% of all patients). The implications of these findings are that elevated plasma levels of TGF-β1 can identify at risk patients and that TGF-β1 reduction could decrease morbidity and mortality of these treatments in breast cancer patients.

Many malignant cells secrete TGF-β1 suggesting that TGF-β1 production may provide an escape mechanism of tumor cells from host immunosurveillance. Establishment of a leukocyte sub-population with disrupted TGF-β1 signaling in the tumor-bearing host offers a potential means for immunotherapy of cancer. A transgenic animal model with disrupted TGF-β1 signaling in T cells is capable of eradicating a normally lethal TGF-β1 over expressing lymphoma tumor, EL4 (Gorelik & Flavell, 2001 Nature Medicine 7(10): 1118-22). Down regulation of TGF-β1 secretion in tumor cells results in restoration of immunogenicity in the host, while T-cell insensitivity to TGF-β1 results in accelerated differentiation and autoimmunity, elements of which may be required to combat self-antigen-expressing tumors in a tolerized host. The immunosuppressive effects of TGF-β1 have also been implicated in a subpopulation of HIV patients with lower than predicted immune response based on their CD4/CD8 T cell counts (Garba, et al. 2002 J. Immunology 168:2247-54). A TGF-β1 neutralizing antibody was capable of reversing the effect in culture, indicating that other TGF-β1 antibody inhibitors may have utility in reversing the immune suppression present in this subset of HIV patients.

The dual tumor suppression/tumor promotion roles of TGF-β1 are clearly elucidated in a transgenic system over expressing TGF-β1 in keratinocytes. While the transgenics were more resistant to formation of benign skin lesions, the rate of metastatic conversion in the transgenics was dramatically increased (Cui, et al 1996 Cell 86(4): 531-42). The production of TGF-β1 by malignant cells in primary tumors increases with advancing stages of tumor progression. Studies in many of the major epithelial cancers suggest that the increased production of TGF-β1 by human cancers occurs as a relatively late event during tumor progression. Further, tumor-associated TGF-β1 provides tumor cells with a selective advantage and promotes tumor progression. The effects of TGF-β1 on cell/cell and cell/stroma interactions result in a greater propensity for invasion and metastasis. Tumor-associated TGF-β1 may allow tumor cells to escape from immune surveillance since it is a potent inhibitor of the clonal expansion of activated lymphocytes, as seen for example, in patients with aggressive brain or breast tumors (Arteaga, et al. 1993 J. Clin. Invest. 92:2569-76). TGF-β1 inhibits the production of angiostatin. Cancer therapeutic modalities such as radiation therapy and chemotherapy induce production of activated TGF-β1 in the tumor, thereby selecting for the outgrowth of malignant cells resistant to TGF-β1's growth inhibitory effects. Thus, these anticancer treatments increase the risk and hasten the development of tumor malignancy. Agents preventing TGF-β1-mediated signal transduction might be a very effective therapeutic strategy in such situations. Resistance of tumor cells to TGF-β1 negates much of the cytotoxic effects of radiation therapy and chemotherapy and the treatment-dependent activation of TGF-β1 in the stroma may even be detrimental as it can make the microenvironment more conducive to tumor progression and contributes to tissue damage leading to fibrosis. Prostate cancer cells express high levels of TGF-β1, which seems to enhance prostate cancer growth and metastasis by stimulating angiogenesis and by inhibiting immune responses directed against tumor cells. Prostate cancer cells frequently lose their TGF-beta receptors and acquire resistance to the anti-proliferative and pro-apoptotic effects of TGF-β1. Accordingly, high expression of TGF-β1 and loss of TGF-beta receptor expression have been associated with a particularly bad prognosis in human prostate cancer patients (Wikstrom, et al. 2000 Scand J Urol Nephrol. 34(2): 85-94). In hepatocellular carcinoma (HCC), the induction of TGF-β1 expression with concomitant loss of TGF-β1 receptor expression in liver foci and nodules makes altered hepatocytes escape from TGF-β1 induced apoptosis, thereby endowing the altered hepatocytes with growth advantage during hepatocarcinogenesis (Lim 2003 Mech Ageing Dev. 124(5): 697-708). In head and neck squamous cell carcinomas, TGF-β1 overexpression at early stages provides a tumor promoting microenvironment (Lu, et al. 2004 Cancer Res 64(13): 4405-10); a binding composition can be used to modulate, ameliorate, treat, prevent, and/or diagnose a hyperproliferative disease, condition, disorder, or syndrome (such as, e.g., a neoplasm) via direct or indirect interactions. For example, such as by preventing the inhibition of the proliferation of cells that, in turn, modulate a hyperproliferative state (e.g., TGF Beta 1 inhibits the proliferation and functional differentiation of T lymphocytes, lymphokines-activated killer cells, NK cells, neutrophils, macrophages, and B cells (Letterio & Roberts 1998 "Regulation of the immune response by TGF Beta" Ann Rev Immunol 16:137-61); or by increasing an immune response (e.g., by increasing the antigenicity of a protein involved in a hyperproliferative condition); or by causing the proliferation, differentiation, or mobilization of a specific cell type (e.g., a T-cell). A desired effect using a composition of the invention may also be accomplished either by, e.g., enhancing an existing immune response, or by initiating a new immune response. Alternatively, the desired result may be affected either by, e.g., diminishing or blocking an existing immune response, or by preventing the initiation of a new immune response. Local administration to an abnormally proliferating cell may be achieved by any art known method or technique discussed herein including, e.g., without limit to transfection, electroporation, microinjection of cells, or in vehicles (such as a liposome, lipofectin, or a naked polynucleotide). By "cell proliferative condition" is meant any human or animal disease, syndrome, disorder, condition, or state, affecting any cell, tissue, any site or any combination of organs, tissues, or body parts, which is characterized by a single or multiple local abnormal proliferation of cells, groups of cells, or tissues, whether benign or malignant.];
- ***Neuronal Disease*** ― neural scarring (Logan et al. 1994 Eur. J. Neurosci. 6:355-63); cerebrovascular abnormalities; Alzheimer's disease (Masliah, et al. 2001 Neurochemistry International 39:393-400) [TGF-β1 mRNA levels in the midfrontal gyrus correlate positively with the relative degree of cerebrovascular amyloid deposition in that brain region, suggesting a possible role for TGF-β1 in human cerebrovascular abnormalities. Transgenic mice overexpressing TGF-β1 in astrocytes develop Alzheimer disease (AD)-like cerebrovascular abnormalities, including perivascular astrocytosis, microvascular basement membrane thickening, and accumulation of thioflavin S-positive amyloid in the absence of parenchymal degeneration. Chronic overproduction of TGF-β1 in the brain results in structural and functional impairments reminist;ent of those in AD cases with amyloid angiopathy (Buckwalter, et al. 2002 Ann N Y Acad Sci. 977:87-95]; proliferative retinopathy (Chaturvedi, et al. 2002 Diabetes Care 25:2320-7)); other eye diseases associated with a fibroproliferative condition associated with TGF-β1 overproduction include retinal reattachment surgery accompanying proliferative vitreoretinopathy; cataract extraction with intraocular lens implantation; and post glaucoma drainage surgery;
- ***Hemopoietic States*/*****Conditions―*** [The naturally occurring balance between endogenous stimulators and inhibitors of angiogenesis is one in which inhibitory influences typically predominate (see, e.g., Rastinejad, et al., Cell 56345-355 (1989)). When neovascularization occurs under normal physiological conditions, such as wound healing, organ regeneration, embryonic development, and female reproductive processes, angiogenesis is stringently regulated, and delimited spatially and temporally. In pathological angiogenesis such as, e.g., during solid tumor formation, these regulatory controls fail and unregulated angiogenesis can become pathologic by sustaining progression of many neoplastic and non-neoplastic diseases. A number of serious diseases are dominated by abnormal neovascularization (including, e.g., solid tumor growth and metastases, arthritis, some types of eye conditions, and psoriasis; see, e.g., reviews by Moses, et al., 1991 Biotech. 9630-634; Folkman, et al., 1995 N. Engl. J. Med., 333:1757-63; Auerbach, et al., 1985 J. Microvasc. Res. 29:401-11; Folkman, "Advances in Cancer Research," Eds. Klein and Weinhouse, Academic Press, New York, pp. 175-203 (1985); Patz, 1982 Am. J. Opthalmol. 94:715-43; and Folkman, et al., 1983 Science 221:719-25. Tumor angiogenesis is crucial for tumor growth and invasion, as blood vessels deliver nutrients and oxygen to the tumor cells and allow them to intravasate the blood system, which leads to metastasis. TGF-Beta 1 acts as a potent inducer of angiogenesis in several assays (Roberts, et al. 1986 PNAS 83:4167-71; Madri, et al. 1988 J. Cell Biol. 106:1375-84; Yang & Moses 1990 J. Cell Biol. 111:731-74; Gajdusek, et al. 1993 J. Cell. Physiol. 157:133-44; Choi & Ballermann 1995 J. Biol. Chem. 270:21144-50), and mouse models defective in TGF- signaling components indicate the importance of TGF-1 in normal vascular development. Moreover, TGF beta 1 and its receptors ALK-5 and ALK-1, are implied in the vascular maturation phase of angiogenesis (see, e.g., Bull Acad Natl Med. 2000; 184 (3):537-44). In a number of pathological conditions, angiogenesis contributes to a disease-state, e.g., for example, significant data have accumulated suggesting that solid tumor formation is dependent on angiogenesis (see, e.g., Folkman & Klagsbrun 1987 Science 235:442-7). In another embodiment of the invention, administration of a binding composition provides for the treatment, amelioration, modulation, diagnosis, and/or inhibition of a disease, disorder, syndrome, and/or condition associated with neovascularization or hematopoeitic cell expansion. Malignant and metastatic conditions that can be effected in a desired fashion using a binding composition include, e.g., without limitation, a malignancy, solid tumor, and a cancer as described herein or as otherwise known in the art (for a review of such disorders, syndromes, etc. see, e.g., Fishman, et al., Medicine, Current Ed., J. B. Lippincott Co., Philadelphia 2002). For example, cancers that may be so affected using a composition of the invention includes, e.g., without limit a solid tumor, including e.g., prostate, lung, breast, ovarian, stomach, pancreas, larynx, esophagus, testes, liver, parotid, biliary tract, colon, rectum, cervix, uterus, endometrium, kidney, bladder, thyroid cancer; primary tumors and metastases; melanomas; glioblastoma; Kaposi's sarcoma; leiomyosarcoma; non-small cell lung cancer; colorectal cancer; advanced malignancies; and blood born tumors such as e.g., leukemia;
- ***Infections* —** sepsis; [the course of Leishmanial infection in mice is drastically altered by TGF-β1. Genetically resistant mice became susceptible to Leishmanial infection upon administration of TGF-β1. TGF-β1 exacerbated the disease, whereas TGF-β1 antibodies halted the disease progression (Barral-Netto, et al. 1992 Science 257:545-7)];.
- ***Hair loss*** — alopecia; hair loss [a neutralizing anti-TGF-β1 antibody reversed the androgen-elicited growth inhibition of keratinocytes in a dose-dependent manner suggesting that androgen-inducible TGF-ß1 derived from balding dermal papilla cells mediates hair growth suppression in androgenetic alopecia and thus may play a role in human pattern baldness (Inui, et al. 2002 FASEB J. 16(14): 1967-9)]; thus, the invention
provides a method of ameliorating, modulating, treating, preventing, and/or diagnosing an angiogenesis-related disease and/or disorder, comprising administering to a subject in need thereof a beneficially effective amount of a binding composition.

The broad scope of this invention is best understood with reference to the following examples, which are not intended to limit the invention to specific embodiments.

### EXAMPLES

### General Methods

Many of the standard methods described herein are described or referenced, e.g., in Sambrook, et al. (2001) Molecular Cloning, A Laboratory Manual Cold Spring Harbor Laboratory, Cold Spring Harbor Press, NY; and its associated web site: (www.MolecularCloning.com); Ausubel, et al., Biology Greene Publishing Associates, Brooklyn, NY; or Ausubel, et al. (1987 and Supplements) Current Protocols in Molecular Biology Wiley/Greene, NY; Bartlett & Stirling (2003) PCR Protocols: Methods in Molecular Biology Vol. 226 Humana Press, NJ. Methods for protein purification include such methods as ammonium sulfate precipitation, column chromatography, electrophoresis, centrifugation, crystallization, and others; see, e.g., Ausubel, et al. (1987 and periodic supplements); Deutscher (1990) "Guide to Protein Purification," Methods in Enzymology vol. 182, and other volumes in this series: Coligan, et al. (1995 and supplements) Current Protocols in Protein Science John Wiley and Sons, New York, NY; Matsudaira (ed.) (1993) A Practical Guide to Protein and Peptide Purification for Microsequencing, Academic Press, San Diego, CA; and manufacturer's literature on use of protein purification products, e.g., Pharmacia, Piscataway, NJ, or Bio-Rad, Richmond, CA. Combination with recombinant techniques allows fusion to appropriate segments (epitope tags), e.g., to a FLAG sequence or an equivalent which can be fused, e.g., via a protease-removable sequence. See, e.g., Hochuli (1989) Chemische Industrie 12:69-70; Hochuli (1990) "Purification of Recombinant Proteins with Metal Chelate Absorbent" in Setlow (ed.) Genetic Engineering, Principle and Methods 12:87-98, Plenum Press, NY; and Crowe, et al. (1992) QIAexpress: The High Level Expression and Protein Purification System QUIAGEN, Inc., Chatsworth, CA.

Standard immunological techniques are described, e.g., in Hertzenberg, et al. (eds.) 5th ed. 1996 Weir's Handbook of Experimental Immunology vols. 1-4, Blackwell Science; Bierer, et al., Eds. (2004) Current Protocols in Immunology Wiley/Greene, NY; and Methods in Enzymology volumes: 70, 73, 74, 84, 92, 93, 108, 116, 121, 132, 150, 162, and 163 Elsevier, USA.

### Example 1: Construction and Screening of Fab Fragments Using CDRs and Human Frameworks

Standard approaches to characterizing and synthesizing antibody variable region CDR libraries of single mutations are used (see, e.g., Wu et al, 1998 PNAS 95:6037-42). Libraries are constructed in bacteriophage M13 expression vectors containing antibody light chain and heavy chain genes composed of human constant region and variable region framework sequences described herein together with CDR sequences of the invention. In some cases, the target CDR is first deleted prior to annealing the nucleotides. Codon based mutagenesis for oligonucleotide synthesis to yield CDR sequences of the invention is employed.

Libraries are initially screened by capture lift to identify the highest affinity variants. The capture lift procedure (Watkins, 2002 Methods Mol. Biol. 178:187-93) is art known and described in WO/0164751 and US2002/0098189. Subsequently, desired clones are further characterized by titration on immobilized antigen in an ELISA and a cell proliferation potency assay as described herein. Following such screening, dissociation constants (K_{d}), association rates (Kₒₙ) and dissociation rates (K_{off}), are determined for a clone of interest.

To identify potential antibody binding compositions comprising embedding donor CDRs of the invention, libraries of synthetic CDRs are inserted into a deletion template as described herein or art known. Standard mutagenesis techniques (Kunkel, 1985 PNAS 82:488-92) are employed to replace a particular CDR using a pool of mutagenic oligonucleotides. Typically, CDR positioning within a framework is accomplished using the system as defined by Kabat with the exception of CDRH1, which is the sum of Kabat and Chothia definitions. Mutagenic oligonucleotides are annealed to an uridinylated phage template in which the corresponding CDR is deleted.

Annealing is accomplished by incubating a reaction at 85°C for 5 minutes followed by slow cooling to 20°C over the course of 45 minutes. Annealed samples are placed on ice, T4 DNA polymerase and T4 DNA ligase are added to generate double stranded DNA, and the reaction is incubated for 5 minutes at 4°C followed by 90 minutes at 37°C. The reaction is phenol extracted, ethanol precipitated and the resulting DNA electroporated into DH10B cells. XLOLR cells are added to the reaction to allow phage amplification before the libraries are plated. Phage stocks are prepared by the addition of 8 ml of growth medium to the plates followed by incubation at 4°C for a minimum of 4 hours. The phage-containing medium is harvested and clarified by centrifugation and sodium azide (0.02%) is added as a preservative.

Initial screening of the libraries is done by plaque lifts as described in Watkins, et al 1998 Anal Biochem 256: 169-77; and Watkins, 2002 Methods Mol. Biol., 178:187-93. Filters containing expressed Fabs from individual plaques bound to immobilized anti-human kappa antibody are sequentially incubated with biotinylated TGF Beta-1 (bio-TGF Beta-1), Neutravidin-alkaline phosphatase (NA-AP), with brief washes in between. Clones of interest are sequenced and further characterized by ELISA. The ELISA generally used Costar 3366 microtiter plates coated overnight at 4°C with 0.4 ug/ml TGF Beta-1, TGF-Beta-2 or TGF-Beta 3. Plates are subsequently washed 2X times prior to the addition of 100 uL of blocking solution (10 mg/ml BSA in wash buffer) into each well. Dilutions of Fabs are incubated in the coated wells for 1.5 h at 22°C. After washing, an anti-human kappa-alkaline phosphatase conjugate is added and incubated for 1 hour at 22°C. A colometric substrate is added after extensive washing and absorbance is at A560 is measured to identify positive clones.

### Assaying Fabs by ELISA

In one non-limiting example, an ELISA is employed that uses Costar 3366 microtiter plates coated overnight at 4°C with 0.4 ug/ml TGF Beta-1, TGF-Beta-2, or TGF-Beta-3 (TGF-Beta-1 (R&D Systems, Cat # 240-B/CF, 239 ug/ml), TGF-Beta-2 (RDI, Cat # RDI-1035, 50 ug/ml) and TGF-Beta-3 (RDI, Cat # RDI-1036/CF, 50 ug/ml) diluted to 0.4 ug/mL in coating buffer). The plate is subsequently washed (2X) prior to the addition of 100 uL of Blocking solution (10 mg/ml BSA in wash buffer) into each well. Dilutions of Fabs of the invention are incubated in the coated wells (1.5hr at 22°C). After washing, an anti-human kappa-alkaline phosphatase conjugate is added and incubated (1 hour at 22°C). A colometric substrate is added after extensive washing and absorbance is measured at A560.

In another example, binding compositions of the invention are tested in a competitive ELISA assay. Typically, a solution phase assay is performed in which a compound that might compete with an antigen for binding to a binding composition, such as an antibody, is combined first with the antibody in solution phase, then the degree of binding of the antibody with the antigen is subsequently measured.

### Materials:

Carbonate coating buffer consists of 50 mM sodium carbonate pH 9.6. Antigens are TGF-Beta 1 (R&D Systems, Cat # 240-B/CF, 239 ug/ml), TGF-Beta 2 (RDI, Cat # RDI-1035, 50 ug/ml) and TGF-Beta 3 (RDI, Cat # RDI-1036/CF, 50 ug/ml) diluted to 0.4 ug/mL in coating buffer. Wash buffer consists of 0.02 M Tris pH 7.4, 0.15 M NaCl, 0.1% Tween 20 and blocking solution of 10 mg/ml BSA (Sigma A-4503) dissolved in wash buffer. Proteins used as positive controls are mouse-anti-human TGF-Beta 1, 2, or 3 (R&D Systems, cat# 1D11), mouse-anti-human TGF-Beta 2 (R&D Systems, cat# BAF302) and mouse-anti-human TGF-Beta 3 (R&D Systems, cat# BAF243), which are diluted to 1ug/ml in block buffer. The detection antibody conjugate used is anti-mouse kappa - peroxidase conjugate (Southern Biotech, cat# 1050-05), at a working concentration of 1:2000 in blocking solution. The substrate used for the color reaction is O-phenylenediamine (OPD) tablets (Sigma cat# P-6912), which is dissolved in substrate buffer: 0.1 M Na₂HPO₄, pH to 5.0 with 0.05 M citric Acid. The OPD substrate working solution (i.e., the volume for one 196-well-plate) is freshly made prior to each plate development by dissolving 1 x 5mg OPD tablet in 12.5 mL of substrate buffer followed by the addition of 5 ul of 30% H₂O₂.

### Protocol:

A single 96 well plate is coated with antigen (TGF-Beta 1, 2, or 3 at 0.4 ug/ml and dispense 50 uL per well) and then sealed with adhesive tape before storage (16-20 hours at 4°C). The plate is subsequently washed (2X) in wash buffer (described above) before adding 100 uL of blocking solution (10 mg/ml BSA in wash buffer) into each well. After incubation (approximately 1 hour at 22°C), the plate is washed (2X) with wash buffer. Then, 100 uL of either sample (diluted in buffer) or control (diluted in PBS) is added to each well and incubated (1.5 hours at 22°C). After incubation, the plate is washed (6X) with wash buffer before adding 100 uL per well of either anti-mouse kappa-peroxidase conjugate (diluted to 1:2000 in Blocking solution) or SA-HRP (diluted 1:10,000 in blocking solution). The test samples are left to incubate (1 hour at 22°C) before adding 100 uL of OPD substrate to each well. After color development (approximately 10 minutes), the 96-well plate is measured at an absorbance of 490nm.

Successful results under such conditions are Fab embodiments that produce an absorbance greater than 1.6 units at 490 nm with TGF-Beta 1 but show significantly lower values with TGF-Beta 2 and TGF-Beta 3 thus demonstrating specific and/or selective binding for TGF-Beta 1.

### Assaying mAbs in a Cell-Based Assay

To test the ability of a binding composition of the invention to neutralize TGF beta bioactivity and to neutralize a particular TGF Beta isoform, one can adapt the HT-2 cell proliferation assay of Tsang, et al., (1995 Cytokine 7:389-97). The HT-2 cell proliferation assay is utilized to determine the *in vitro* potency of Fab and mAb compositions. Briefly, HT-2 cells proliferate in the presence of IL-4 but undergo apoptosis in the presence of TGF-Beta. The TGF-Beta induced cell death is prevented by the addition of a TGF-Beta 1 neutralizing Fab or mAb.

The human cell line HT-2 proliferates in response to IL-4 but the IL-4-induced-proliferation is inhibited by TGF-Beta 1, TGF-Beta 2, or TGF-Beta 3. Consequently, a binding composition that is specific and/or selective for TGF-Beta 1 is neutralizing if it prevents the normal inhibitory effect that TGF-Beta 1 has on IL-4-induced HT-2 cells. Accordingly, IL-4-induced cell proliferation should proceed unconstrained if sufficient TGF Beta 1-specific binding composition is added to a mixture of HT-2 cells containing TGF Beta 1. Consequently, the dose response neutralizing capability of binding compositions of the invention is assessed using the HT-2 assay in the presence of particular TGF isoforms and the IL-4 proliferation signal. The degree of cell proliferation is assessed using a commercial colorimetric cell proliferation measure (e.g., CellTiter 96® AQueous One Solution Cell Proliferation Assay from Promega).

HT-2 cells are maintained in RPMI 1640 supplemented with 10% FBS, penicillin/streptomycin at 100 U/ml and 100 ug/ml respectively, 50 uM betamercaptoethanol and 10 ng/ml human IL-2 (R&D Systems). Cells are centrifuged at 1000 RPM in a Jouan CR422 centrifuge and re-suspended in PBS. After two washes with PBS, cells are finally re-suspended at 0.15 x 10⁶ cells/ml in Assay Media which consists of phenol red-free RPMI 1640 supplemented with 2% FBS, penicillin/streptomycin at 100 U/ml and 100 ug/ml respectively and 50 uM beta- mercaptoethanol. To each well of a 96 well plate is added 50 ul of cells in Assay Media. Before adding test mAbs to cell bioassay, varying concentrations of mAb are pre-incubated with 300 pg/ml TGF-Beta 1, TGF-Beta 2, or TGF-Beta 3 (in Assay Media). Following a 30 min incubation, 50 ul of the TGF-Beta /Fab mixture is added to the cells, followed immediately thereafter by 50 ul of Assay media containing 45 ng/ml murine IL-4 (15 ng/ml final). After an incubation of 20-48hr, 35 ul of CellTiter 96 Aqueous solution (Promega Corp) is added. After a further 2 to 3 hr incubation (37°C in a humidified, 5% CO2 atmosphere), the assay is quantitated by analysis on an ELISA plate reader at 490 nM using the CellTiter 96® colorimetric assay (the quantity of formazan product generated —and as measured by the amount of 490nm absorbance— is directly proportional to the number of living cells in culture). Modal data are shown below in Table 2.

**Table 2 HT-2 In Vitro Cell Bioassay: An HT -2 cell proliferation assay is utilized to determine in vitro potency of Fab and mAb binding compositions of the invention. HT-2 cells proliferate in the presence of IL-4 but undergo apoptosis in the presence of TGF-Beta. A TGF-Beta induced cell death is prevented by the addition of a TGF-Beta neutralizing compositon (such as, e.g., a FaB or mAb of the invention). In comparison to the murine IgG1 FAb #2471, binding compositions of the invention show at least a greater than 100-fold or more improvement in neutralization potency in preventing TGF -Beta induced cell death. The IC50's for mAb compositions range from approximately 0.1-1.0 ng/ml (the IC50 of mAb 2471 is approximately 0.1 µg/ml).**

| **Anti-TGF-β1 mAb Binding Compositions** | **Fold Improvement in IC50 relative to mAb2471** |
|---|---|
| 21D1 | 106.8 + 16.1 |
| DM4 | 391.0 + 5.2 |
| DM7 | 140.0 + 29.2 |
| C27 | 401.0 + 132.3 |

### Measuring Kinetic Constants for Fabs

A KinExA 3000 instrument (Sapidyne Inst. Inc.) is used to measure binding kinetics. Briefly, an antigen is covalently coupled to alzactone beads and the binding of a free Fab binding composition of the invention to the beads is detected on the instrument. To measure Kd, individual tubes containing 20 pM of Fab (200 pM for the mAb) with decreasing serially diluted antigen (0-250nM), is incubated for 1-6 days at 25°C in PBS containing 1% BSA, 0.02% azide and 0.0 1 % Tween20. After the incubation, free Fab in each equilibrated sample is determined on the KinExA 3000 according to the manufacturer's instructions. K_{d} values are determined using KinExA 3000 software.

To measure kₒₙ, individual Fabs at 2nM are mixed with 0-240nM of antigen using the injection method according to the manufacturer's instructions, and the unbound Fab is detected. The resulting data is used to calculate the kₒₙ with KinExA 3000 software. The k_{off} is calculated by using the formula K_{d} = k_{off}/kₒₙ.

### Measuring Kinetic Constants for Mabs

Alternate methods of measuring kinetic constants are known, for example: affinity of a binding composition for human TGF-Beta 1 (R&D Systems, Cat # 240-B/CF), TGF-Beta 2 (RDI, Cat # RDI-1035) and TGF-Beta 3 (RDI, Cat # RDI-1036/CF) is measured using a BIAcore® 2000 instrument. The BIAcore® utilizes the optical properties of surface plasmon resonance to detect alteration in protein concentration of interacting molecules within a dextran biosensor matrix. Except as noted, all reagents and materials are purchased from BIAcore® AB (Upsala, Sweden). All measurements are performed at room temperature. Samples are dissolved in HBS-EP buffer (150mM sodium chloride, 3mM EDTA, 0.01% (w/v) surfactant P-20, and 10mM HEPES, pH7.4). Recombinant Protein A is immobilized on all four flow cells of a CM4 sensor chip at a level of 400-450 response units (RUs) using an amine coupling kit.

Binding is evaluated using multiple analytical cycles. Each cycle is performed at a flow rate of 50µL/minute and consists of the following steps: injection of 12µL of an antibody binding composition at 0.5 µg/mL, injection of 250µL of TGF-Beta 1 (starting at 5nM and using two-fold serial dilutions to 0.13nM for each cycle, with two injections for each concentration) followed by either a short (5 minutes) or long (120 minutes) delay for dissociation, and regeneration using two injections of 50µL of 10mM glycine hydrochloride, pH1.5. Association and dissociation rates for each cycle are determined by fitting of the biosensor data using to a simple association model using ClampXP (Center for Biomolecular Interaction Analysis, Univ. of Utah) to extract the kₒₙ and k_{off} rate constants; the equilibrium binding constant Kd is calculated using the relationship K_{d} = k_{off}/kₒₙ. Modal data for invention compositions are in Table 3 shown below.

**Table 3 Binding affinity and kinetic measurements for Fabs and mAbs of the invention. Equilibrium (K_{d}) and kinetic (kₒₙ) binding parameters are determined using Kinexa (k_{off} is calculated from K_{d} and kₒₙ). Equilibrium and kinetic binding properties of mAbs are determined using Biacore. The equilibrium binding constant K_{d} is calculated from the determined kₒₙ and k_{off}. Comparison is to murine IgG1 Fab #2471. Due to the very slow dissociation, the k_{off} for 21D1 and DM7 is the upper limit detectable , and are likely much slower, and therefore, the K_{d} values calculated are also upper limits. Values are the average of repeat measurements (n = 3-4).**

| | Fab Binding Data (Kincxa) | | | Mab Binding data (Biacore) | | |
|---|---|---|---|---|---|---|
| | *kₒₙ* (M⁻¹ s⁻¹) (× 10⁻⁶) | *k_{off}* (sec⁻¹), calc, (× 10⁶) | K_{d} (pM) | *kₒₙ* (M⁻¹ s⁻¹) (× 10⁻⁷) | *k_{off}* (sec⁻¹), (× 10⁵) | K_{d} (pM) (calc) |
| 2471 | 1.7 | 664 | 406 | nd | nd | nd |
| 21D1 | 4.0 | 3.4 | 0.9 | 1.3±0.1 | <0.6±0.6 | < 0.5 ±0.5 |
| DM4 | 4.2 | 5.1 | 1.2 | 1.6 ±0.3 | 1.4 ±0.9 | 0.9 ±0.4 |
| DM7 | 4.5 | 2.3 | 0.5 | 13±0.3 | <0.7±0.4 | < 0.5 ± 0.4 |
| C27 | 4.1 | 17 | 4.2 | 1.3±0.1 | 0.8±0.5 | 0.6±0.4 |
| 23A3 | 4.8 | 19 | 4.0 | 1.9±0.4 | 10±0.8 | 0.8 ±0.6 |

### Determination of Mab Specificity

BIAcore methods are used to determine the ability of invention mAb compositions to bind other entities, specifically the latent form of TGF-Beta 1 or TGF-Beta 3. All measurements are performed at room temperature. Samples are dissolved in HBS-EP buffer (150mM sodium chloride, 3mM EDTA, 0.01% (w/v) surfactant P-20, and 10mM HEPES, pH7.4). Recombinant Protein A is immobilized on all four flow cells of a CM4 sensor chip at a level of 400-450 response units (RUs) using an amine coupling kit.

Binding is evaluated using multiple analytical cycles. Each cycle is performed at a flow rate of 100µL/minute consisting of the following steps: injection of 15µL of an antibody binding composition at 1 µg/mL, injection of 250µL of either 5 nM TGF-Beta 1, 5 nM latent TGF-Beta 1, or 5 nM TGF-Beta 3 followed by a short delay (5 min) for dissociation, and regeneration using two injections of 50µL of 10mM glycine hydrochloride, pH1.5. The amount of signal after capturing first the Mab, and then the ligand, are determined using the instrument control software. Since the signal is proportional to the mass of protein captured, the stoichiometry of the captured ligand is readily calculable (Table 4).

**Table 4 Binding of TGF-Beta 1, latent-TGF-Beta 1 and TGF-Beta 3 to Mabs (tested at 5 nM ligand).**

| | Stoichiometry of ligand binding | | |
|---|---|---|---|
| Mab | TGF-β1 | Latent TGF-β1 | TGF-β3 |
| 21D1 | 1.29 | 0.05 | 0.16 |
| DM4 | 1.55 | 0.03 | ND |
| DM7 | 1,23 | 0.04 | 0.54 |
| C27 | 1.74 | 0.08 | ND |
| 23A3 | 1.63 | 0.07 | 1.26 |

### Specificity for TGF-Beta 1

Affinities of binding composition mAbs for TGF-β3 are determined using BIAcore methods. All measurements are performed at room temperature. Samples are dissolved in HBS-EP buffer (150mM sodium chloride, 3mM EDTA, 0.01% (w/v) surfactant P-20, and 10mM HEPES, pH7.4). Recombinant Protein A is immobilized on four flow cells of a CM4 sensor chip at a level of 400-450 response units (RUs) using an amine coupling kit. Binding is evaluated over multiple analytical cycles. Each cycle is performed at a flow rate of 100µL/minute consisting of the following steps: injection of 15µL of an antibody binding composition at 0.5 µg/mL, injection of 250µL of TGF-β3 (starting at 5nM and using two-fold serial dilutions to 0.13nM for each cycle, with two injections for each concentration) followed by a short (5 minutes) delay for dissociation, and regeneration using two injections of 50µL of 10mM glycine hydrochloride, pH1.5.

Affinities are determined based upon the equilibrium signal as reached at varying TGF-β3 concentrations by measuring the average signal during the last 10 seconds of the TGF-β3 injections, and then fitting the resulting signals at all of the TGF-β3 concentrations to a simple binding equilibrium model in SCRUBBER (Center for Biomolecular Interaction Analysis, Univ. of Utah). Model data determined for tested human mab composition of the invention of K_{d} and specificity ―calculated by dividing the K_{d} for TGF-β3 binding by the K_{d} for TGF-β1 binding (herein)— is shown below in Table 5.

**Table 5 Affinity and relative specificity of binding composition tested mAbs for TGF-Beta 3 binding. Errors, where shown, represent the standard deviation from multiple repeat measurements (n =3).**

| Mab | K_{d} (TGF-β3), nM | Specificity (K_{d,β3}/K_{d,β1}) |
|---|---|---|
| 21D1 | 4.90 | 9800 |
| DM4 | 0.53 ± 0.01 | 621 |
| DM7 | 1.16 ± 0.18 | 2320 |
| C27 | 2.20 | 3670 |
| 23A3 | 0.66 ± 0.04 | 1050 |

### Example 2: Hepatic Fibrosis Bile Duct Ligation In Vivo Model

A bile duct ligation model is utilized in a manner similar to that reported by Arias et al (BMC Gastroenterology 3(29), 2003) to evaluate in vivo efficacy of anti-TGF-β1 therapy. Briefly, male Sprague Dawley rats (250-300 g) are anesthetized with isoflurane (2-3%) inhalation for effect. The abdomen is shaved and scrubbed with betadine and 70% ethyl alcohol. Under sterile conditions, a midline incision (∼4cm) is made and the common bile duct is isolated and ligated with 6-0 surgical silk in two positions, approximately 1 cm apart and then transected between ligatures. The abdominal wall is closed with 4-0 silk suture and the skin stapled together. Administration of an anti-TGF-β1 composition and isotype mouse control mAb (IgG) are commenced on the day of surgery and every 7 days thereafter. At 4 and 12 days post-surgery, rats are euthanized and serum liver enzymes, complete blood count, and liver histology (trichrome and H&E stains) are processed to determine effect of treatment.

### Example 3: Lung Fibrosis In Vivo Model

A number of models are available in evaluating the in vivo efficacy of anti-TGF-beta compositions on lung fibrosis. For example, a bleomycin model applied in the manner reported by Pittet, et al (JCI 107, 1537-1544, (2001) is used to assess amelioration in an anti-TGF-beta approach. Another model is the respiratory reovirus 1/L model (see, e.g., Bellum et al., Am. J. Pathol, 150, 2243; or London et al, Clin. Immunol. 103, 284; and London et al, Exp.Mol.Pathol, 72, 24-36). Briefly, using mice, on day 1, Reovirus I/L, i.n. 1 x 10⁷ pfu (30 ul total) is applied via the nostril followed on days 3, 7, 12 with varying concentrations of anti-TGF-beta binding composition of the invention or an isotype control mAb as described herein or art known. Animals are monitored during the course of treatment for signs of respiratory distress, weight loss, and mortality. On day 14 after initiation of treatment, animals are euthanased and lung samples prepared for histopathological examination (H/E) to assess the development and/or progression of lung disease (with hydroxyproline content analyzed for measurement of fibrosis).

### Example 4: Anti-Thy1.1 Glomerulonephritis In Vivo Model

The rat anti-Thy1.1 model is a well-established model of mesangioproliferative glomerulonephritis (see, e.g., Morita, et al., 1998 Am J Kidney Dis 31:559-73; Bagchus, et al., 1986 Lab. Invest. 55:680-7 and Yamamoto & Wilson 1987 Kidney Int. 32:514-25) in which injection of an antibody directed against the Thy antigen located on the surface of mesangial cells induces mesangiolysis followed by a phase of over compensatory proliferation of mesangial cells resulting in elevated levels of urinary protein (proteinuria). The anti-Thy1.1 nephritis model resembles human IgA nephritis or Henoch-Schonlein purpura in many aspects (O'Donoghue, et al., 1991 J Clin Invest 88:1522-30) and it has been used to test potentially therapeutic approaches to kidney disease by determining the ability of test compositions to effect dose-related decreases in proteinuria (see, e.g., Burg, et al., 1997 Lab Invest 76:505-16; Johnson, et al., 1995 Kidney Int 47:62-9).

To test binding compositions of the invention in such a model, individually marked, male Sprague Dawley rats (200-260 grams; approximately 9 weeks of age) are acclimated for five days pre-treatment with free access to food and water on a standard diet. A pre-urinary protein determination is made at pre-treatment day-5. Rats are given an individual identification by marking on the tail with a colored marker as well as ear tagged prior to being bled by retro-orbital, and randomized into 5 groups based on body weight at day l.

The study is performed blinded to the treatment groups and unblinded at the end of the study. Groups recieve either 1.25 mg of anti-Thy 1.1 mAb or PBS as a control injection via the penile vein on day 0. Binding compositions are prepared and purified under standard conditions or as described herein. The control mouse IgG1 mAb (11513) is protein A purified material resuspended in PBS pH7.2 and is purchased from Harlan Bioproducts for Science, Indianapolis, IN 46229-0176.

Mouse anti-Thy 1.1 is produced in 2 x 10L cultures of mouse hybridoma. Conditioned media is combined, concentrated to 18X and subsequently purified. Approximately, 764mLs of the concentrated harvest supernatant is mixed with 1.5M Glycine/3.0M NaCl pH 8.9 and applied to a virgin, 137ml Protein A Sepharose column that is pre-equilibrated in 1.5M Glycine/3.0M NaCl pH 8.9. The Protein A column is then ished with 1.5M Glycine/3.0M NaCl pH 8.9. The column is eluted with 100 mM Citric Acid pH 3.0. Selected fractions of eluate that correspond to IgG are pooled, adjusted to pH 7.4 with 1M NaOH, and applied to a 318ml Pharmacia Superdex 200 column equilibrated in PBS, pH 7.4sodium chloride, . The peak corresponding in size to IgG is pooled, aliquoted and stored at -20°C.

One hour after anti-Thy 1.1 mAb administration, animals are dosed subcutaneously with isotype or anti-TGF-Beta 1 antibody compositions of the invention. Antibodies are again dosed on day 7, animals are tested in the following four treatment groups:
1) Shams; PBS injection
2) Anti-thy 1.1 with Isotype control antibody at ∼ 12.5 mg/kg or 2.5 mg/dose
3) Anti-thy 1.1 with DM4 antibody at ~5 mg/kg, or I mg/dose
4) Anti-thy 1.1 with DM7 antibody at ~12.5 mg/kg, or 2.5 mg/dose
5) Anti-thy 1.1 with C27 antibody at ~12.5 mg/kg, or 2.5 mg/dose

Rats are placed into metabolic cages for a 24 hr time period on days -5 and 13. On day 14, rats are sacrificed by CO₂ and bled via cardiac puncture to obtain blood for analysis. The left kidney is fixed with 4% Paraformaldehyde in PBS and stored in 70% ethanol for later histological analysis. If any rat becomes moribund, it is sacrificed (CO₂) and processed for urinary protein and blood urea nitrogen concentrations. Urinary protein and blood urea nitrogen (BUN) concentrations are analyzed on a HITACHI 911 automatic analyzer with controls from Biorad according to the manufacturers instructions.

Model data in Table 6 below show the binding compositions of the invention have a significant ability to attenuate renal damage *in vivo* and to reduce the elevated protoneuria associated with anti-Thy 1.1 mAb induced renal damage.

**Table 6. Rats are injected i.v. with 2.5mg/kg of α-Thy1.1 mAb followed 30 min later with 1mg of Herceptin for the 21D1 and DM4 studies. A second dose of anti-TGF-Beta 1 and control mAb is administered on day 7, and animals are euthanized on day 14. Both the 21D1 and DM4 mAb binding compositions decrease urinary protein levels (proteinuria) in a dose dependent manner.**

| | | | | | | | |
|---|---|---|---|---|---|---|---|
| | | | | | | | |

| | | | Day 14 Urine Protein (mg/24 hrs) | | | % of Control IgG | |
|---|---|---|---|---|---|---|---|
| Thy 1.1 Ab | Ab group | Study No. | Dose(mg) | AVG. | SE. | AVG. | SE. |
| | | | Sham | 7.0 | 3.0 | | |
| 2.5 mg/kg | 21-D1 | HS4606 | 0 | 73.4 | 9.5 | 100.0 | 14.3 |
| i.v. | | | 0.1 | 65.7 | 7.3 | 88.5 | 11.1 |
| | | | 0.5 | 35.2 | 6.2 | 42.5 | 9.3 |
| | | | 1 | 29.1 | 3.8 | 33.3 | 5.8 |
| | | | | | | | |
| | | | Sham | 9.9 | 2.3 | | |
| 2.5 mg/kg | DM4 | HS4607 | 0 | 67.2 | 6.5 | 100.0 | 11.3 |
| i.v. | | | 0.1 | 54.7 | 6.2 | 78.1 | 10.8 |
| | | | 0.5 | 49.7 | 6.2 | 69.4 | 10.7 |
| | | | 1 | 28.9 | 4.7 | 33.2 | 8.2 |

### EXAMPLE 5: Epitope Mapping of TGF-β1 Binding Compositions

A combination of H/Dex and chemical labeling are used to map epitopes of TGF-β1 binding compositions of the invention such as, for example, antibodies. As both H/D exchange and chemical modification depend on solvent accessibility to amino acid residues, changes in solvent accessibility after formation of a binding composition:TGF Beta 1 complex can be used to identify residues involved in antibody binding. Subsequent to H/D exchange or chemical modification, proteolytic digestion into peptide cleavage fragments of the formerly bound antigen permits molecular weight comparisons between fragments (using LC/MS) to determine which amino acid residues are blocked from H/D exchange or chemical modification after binding complex formation.

*Proteins Surface Chemical Labelling* 15 µg aliquots of I mg/mL TGF-β1 in 4 mM HCl solution are transferred into plastic vials, to which 180 µg of either a control or a TGF-β1 antibody composition of the invention is added (TGF-β1/antibody = ½ in molar ratio). Phosphate buffered saline (PBS) is added into each vial to a final volume of 150 µL and the solutions are allowed to incubate (to permit formation of a binding complex) at ambient temperature for at least 10 min before protein surface labeling. For chemical labeling, 7.5 µL of a 5 mg/mL acetic acid hydroxylsuccinimide ester (AHSE) solution is added in each complex vial and the mixtures are then incubated at ambient temperature (AHSE/antibody = 200/1 in molar ratio). At varying times (e.g., 10, 20, and 60 min), 50 µL of the mixture solution is quenched by mixing with 50 µL of 1 mg/mL K in 0.1 M tris buffer, pH 8.0. The solution is directly analyzed by LC/MS (as described herein). The remaining solution of each sample is treated with 3-5 µL of 50 mg/mL DTT solution at 37°C (10-15 min) to reduce the disulfide bonds of mature TGF-β1.

The reduced protein solution is subsequently treated with 3 µL of 0.1 mg/mL chymotrypsin solution at 37°C for 2-3 hours, and then treated with 1 µL of 0.25 mg/mL Glu-C solution at 37°C for another 2-3 hours. This reaction is quenched by adding 0.5 µL of glacial acetic acid, and then analyzed by LC/MS, using a Waters 2795 HPLC and Micromass LTC Premier Mass spectrometer. The HPLC used a Zorbax, SB C18, 2.1×50 mm, at ambient temperature, and proteins and peptides are eluted with an acetonitrile gradient in 0.15% formic acid; a 14 minute run time is used for the intact protein, and a 75 minute run time is used for proteolytic digests.

For lysine (K) residues on the TGF-β1 surface or either within or structurally near the epitope, acetylation of the K amino group is blocked (either partially or completely) after a test composition binds TGF-β1. Comparing the extent of acetylation between a peptide from a complexed (TGF-β1 + antibody that binds TGF-β1) or uncomplexed (TGF-β1 + control antibody that does not bind TGF-β1) sample allows one to identify amino acid residues blocked from acetylation by the formation of the binding complex. One model of acetylation data obtained under such an LC/MS analysis is shown below in Table 7.

**Table 7. Mole of Acetylated Amino Group per Mole Peptide Obtained by LC/MS for Epitope Mapping**

| **Ac. Time** | **Complex** | Mole of Acetylation/Mole peptide | | | | | | | | | | | |
|---|---|---|---|---|---|---|---|---|---|---|---|---|---|
| | | **1-6** | **9-21** | **22-30** | **22-32** | **31-39** | **33-43** | **51-62** | **53-62** | **66-90** | **91-112** | **9.1-99** | **100-112** |
| 10 min | **TGF β1+Cont.** | 0.97 | 0.10 | 0.07 | 0.94 | 0.74 | 0.22 | 0.64 | 0.60 | 0.00 | 1.09 | 1.28 | 0.03 |
| | **TGF β1+21D1** | 0.95 | 0.09 | 0.07 | 0.95 | 0.78 | 0.26 | 0.50 | 0.45 | 0.00 | 0.49 | 0.71 | 0.02 |
| | **TGFβ1+DM4** * | 0.91 | 0.10 | 0.04 | 0.88 | 0.56 | 0.05 | 0.31 | 0.29 | 0.00 | 0.35 | 0.59 | 0.02 |
| | **TGFβ1+DM4** # | 0.91 | 0.07 | 0.06 | 0.92 | 0.62 | 0.13 | 0.30 | 0.28 | 0.00 | 0.39 | 0.66 | 0.02 |
| 20 min | **TGFβ1+Cont.** | 1.00 | 0.25 | 0.14 | 0.99 | 0.89 | 0.35 | 0.82 | 0.79 | 0.00 | 1.32 | 1.57 | 0.05 |
| | **TGFβ1+21D1** | 1.00 | 0.27 | 0.15 | 1.00 | 0.95 | 0.42 | 0.76 | 0.71 | 0.00 | 1.01 | 0.96 | 0.04 |
| | **TGFβ1+DM4** * | 1.00 | 0.23 | 0.06 | 0.95 | 0.76 | 0.09 | 0.52 | 0.51 | 0.00 | 0.55 | 0.81 | 0.03 |
| | **TGFβ1+DM4** # | 1.00 | 0.21 | 0.12 | 0.97 | 0.85 | 0.22 | 0.59 | 0.58 | 0.00 | 0.63 | 0.88 | 0.04 |
| 60 min | **TGFβ1+Cont.** | 0.97 | 0.67 | 0.30 | 1.03 | 1.03 | 0.56 | 0.87 | 0.82 | 0.00 | 1.68 | 1.85 | 0.10 |
| | **TGFβ1+21D1** | 0.97 | 0.79 | 0.36 | 1.08 | 1.21 | 0.73 | 0.93 | 0.92 | 0.00 | 1.24 | 1.39 | 0.08 |
| | **TGFβ1+DM4** * | 0.91 | 0.80 | 0.16 | 0.99 | 0.95 | 0.26 | 0.85 | 0.80 | 0.00 | 0.91 | 1.13 | 0.08 |
| | **TGFβ1+DM4** # | 0.99 | 0.66 | 0.36 | 1.06 | 1.10 | 0.51 | 0.93 | 0.91 | 0.00 | 1.14 | 1.25 | 0.09 |

| | | | | | | | | | | | | | |
|---|---|---|---|---|---|---|---|---|---|---|---|---|---|
| * and # indicates the two different lots of the DM4 antibody are characterized separately. | | | | | | | | | | | | | |

Given such model data, differences between the TGF- β1:antibody complexes and controls are discernable for several TGF-β1 peptide fragments, especially as shown for short periods of acetylation (e.g., 10 min). Fragments encompassing residues 31-39, 33-43, 53-62, and 91-112 demonstrate such discernable differences. Both fragments 31-39 and 33-43 comprise the K37 residue. Fragment 22-32, which comprises K26 & K31, shows no significant difference over controls, thus the acetylation differences of fragment 31-39 are attributable to blocking K37 from acetylation after formation of the antigen: antibody complex.

Fragments 53-62 and 91-112 exhibit persistent differences over the tested range and for each antibody tested. Fragment 53-62, shows decreased differences in the upper range of acetylation (60 min), however, under lower AHSE/antibody ratio conditions, such differences remain unchanged throughout the range. Not being bound by theory, one interpretation of such data is that K60 is not directly involved in antigen:antibody binding but that its proximity to the binding complex is sufficiently close to block accessibility of AHSE to the K60 residue, thus blocking acetylation. Alternatively, however, K60 can comprise the epitope defined by the tested antibody.

Fragment 91-112 shows acetylation differences throughout the tested range, suggesting at least one of three lysine residues of this fragment (K95, K97, or K110) participates in the binding composition:TGF Beta 1 complex. To identify the lysine residue(s) involved, the chymotryptic digest is further treated with Glu-C producing two additional fragments - 91-99 and 100-112. The later (fragment 100-112) contains K110, however, it shows no significant acetylation difference suggesting it is inaccessible either to solvent or to chemical modification.

The former (fragment 91-99) containing K95 and K97 is further tested using uncomplexed TGF-β1 treated with AHSE and MS/MS analysis to determine elution times of the singly-acetylated species, and to quantitate the extent of K95 or K97 acetylation (model data under such conditions are shown below in Table 8). Such data show that acetylation of K97 remains unchanged with or without complex formation, however, presence of an antibody composition of the invention significantly affects K95 acetylation indicating that K95 is directly involved in binding complex formation.

**Table 8. Acetylation of K95 & K97**

| **Acetylation Time** | **TGFb1 & Antibody Complex** | **Acetylation (%)** | |
|---|---|---|---|
| | | **K97** | **K95** |
| 10 min | TGF-β1+Control | 54 | 73 |
| | TGF-β1+LA307-21D1 | 51 | 20 |
| | TGF-β1+LA307-DM4* | 44 | 16 |
| | TGF-β1+LA307-DM4# | 46 | 18 |
| 20 min | TGF-β1+Control | 71 | 86 |
| | TGF-β1+LA307-21D1 | 66 | 27 |
| | TGF-β1+LA307-DM4* | 59 | 22 |
| | TGF-β1+LA307-DM4# | 63 | 25 |
| 60 min | TGF-β1 + Control | 87 | 95 |
| | TCF-β1+LA307-21D1 | 86 | 53 |
| | TGF-β1+LA307-DM4* | 75 | 38 |
| | TGF-β1+LA307-DM4# | 82 | 42 |

| | | | |
|---|---|---|---|
| * and # indicate that two different lots of the DM4 antibody are characterized ***H*/*D exchange.*** | | | |

In epitope mapping, the technique of deuterium/hydrogen (H/D) exchange resembles protein surface labeling/MS, however, H/D exchange is not residue-specific, and thus can detect changes in any amino acid residue. In comparing a binding composition:TGF Beta 1 complex to an uncomplexed mature TGF-β1 protein, the molecular weight of the complexed protein is about 20 Da (or 30Da at 100% D₂O) lower than uncomplexed TGF-β1 thus, by calculating D₂O weight differences between complexed and uncomplexed TGF-β1, it can be shown that approximately thirty amino acid residues in the mature TGF-β1 dimer may participate in forming an invention binding complex.

120 µg aliquots (~140 or 280 µL) of antibody solutions are buffer exchanged into PBS by successive concentration and dilution using a Microcon (30 kD) ultrafiltration protein concentrator (Millipore). After two successive concentrations and dilutions with PBS, the antibody solutions are concentrated, removed, adjusted to a final volume 70 µL with PBS. Then, 10 µL of 1 mg/mL TGF-β1 in 4 mM HCl solution and 2 µL of 1 M tris buffer, pH 8.0, is added into each antibody vial to form a TGF-β1:antibody complex. A TGF-β1 control sample is prepared by mixing 70 µL of 1xPBS, 20 µL of TGF-β1 1 in 4 mM HCl solution, and 2 µL of 1 M tris buffer, pH 8.0. Subsequently, 9 µL of TGF-β1 or the TGF-β1 antibody complex is transferred into a micro plastic vial, and then 21 µL of 100% D₂O is added to form a 70% D₂O solution. The solution is incubated at ambient temperature for 10 min and then at 0°C for 1 min.

After incubation, H/D exchange is quenched and the protein digested by adding 15 µL of 1 % formic acid solution (at 0°C) and 4 µL of 2 mg/mL pepsin solution (at 0°C), and then incubating at 0°C for 5 min. The digest is immediately injected onto the column manually for LC/MS analysis (as described above, except that the tubing and HPLC column are immersed in an ice-water bath).

Mature TGF-β1 resists pepsin digestion at low pH (∼2.5) and low temperature (0°C) due to disulfide bond formation. As a result, few cleavage peptides are produced and most of TGF-β1 is still intact despite longer digestion times and higher enzyme:protein concentrations. Identifiable TGF-β1 proteolytic fragments are typically generated at C-terminal and middle regions of the protein (e.g., fragments 58-64 or 61-64). Model data for the change in mass (delta mass) after D/H exchange using such fragments is shown below in Table 9. The delta mass for fragment 61-64 is approximately zero while the delta mass for fragment 58-64 is about 1 Da suggesting that the region protected from deuterium exchange -after complex formation with a binding composition of the invention- comprises amino acid residues 58-61.

**Table 9. Delta Masses of the Identified Peptic Peptide of TGF-Beta 1 After D/H Exchange**

| **Delta Mass (100 % D₂O)*** | **Peptic Peptide of TGF-β1** | | | | | |
|---|---|---|---|---|---|---|
| | **61-64** | **59-64** | **58-64** | **58-61** | **91-104** | **90-1114** |
| **Average for DM4 (n=3)** | 0.04 | -0.87 | -1.02 | -0.30 | -1.85 | -1.87 |
| **SD for DM4 (n=3)** | 0.01 | 0.11 | 0.21 | 0.05 | 0.30 | 0.30 |
| **for 21D1 (n=1)** | 0.01 | -0.34 | -0.99 | -0.18 | -1.90 | -1.51 |

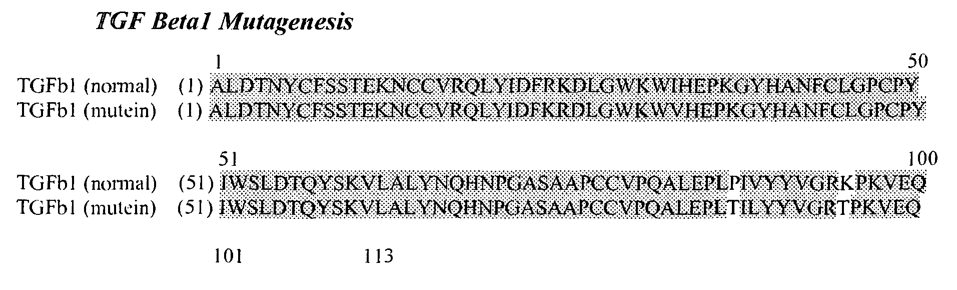

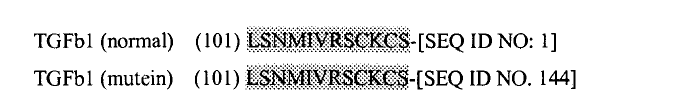

To further define epitopes for binding compositions of the invention, traditional mutagenesis techniques are used to identify TGF Beta 1 residues critical in forming binding complexes with compositions of the invention. The crystal structure of the TGF-Beta 3/TGF-Beta RII complex (2002 Nat Struct Biol. 3:203-8) is used as a model to define significant TGF-Beta 1 protein mutagenesis sites -R25K, K26R, V33I, P87T, V89L, and K95T (shown above).

Ability to bind the TGF Beta 1 mutein is tested using binding compositions specific for TGF Beta isoforms and commercially available mAbs (1D11 and 240; R&D Systems), which prevent binding of TGF Beta 1 to its cognate receptor (TGF-Beta RII). Testing is carried out in a laser-induced desorption/ionization time-of-flight mass spectrometer analysis. Test mAbs, such as, e.g., mAb 3821 and 2471, which specifically bind TGF Beta 1 (disclosed in International Publication No. WO2005/010049) and controls, such as, e.g., mAb 1D11, which binds all three TGF Beta isoforms, are mixed (providing an opportunity to complex with the TGF Beta 1 protein (either mutein or wild-type)), immobilized on detecting chips, lazed, and subsequently analyzed using standardized software under manufactures conditions (Ciphergen Diagnostics).

Model results show that a distinct subset of amino acid residues at the binding interface of TGF Beta I/TGF-Beta RII differ from the other TGF-beta isoforms (TGF Beta 2, and TGF Beta 3. Test mAb #2471 (with specific binding affinity for TGF Beta 1) binds wild type TGF-Beta 1 at a five-fold greater rate than the TGF Beta 1 mutein, while mAbs 3821, and 1D11 bind the TGF-Beta 1 mutein at a rate that is two and one-half fold less then wild type TGF Beta 1.

### SEQUENCE LISTING

SEQ ID NO: I is homo sapiens mature, TGF Beta 1 amino acid sequence.
SEQ ID NOs: 2 and 141 are artificial VHCDR1 amino acid sequence.
SEQ ID NOs: 3 and 142 are artificial VHCDR2 amino acid sequence.
SEQ ID NOs: 4 and 143 are artiticial VHCDR3 amino acid sequence.
SEQ ID NOs: 5 and 138 are artificial VLCDR1 amino acid sequence.
SEQ ID NOs: 6 and 139 are artificial VLCDR2 amino acid sequence.
SEQ ID NOs: 7 and 140 are artificial VLCDR3 amino acid sequence.
SEQ ID NO: 8 is homo sapiens HCVR FR1 framework amino acid sequence.
SEQ ID NO: 9 is homo sapiens HCVR FR2 framework amino acid sequence.
SEQ ID NO: 10 is homo sapiens HCVR FR3 framework amino acid sequence.
SEQ ID NO: 11 is homo sapiens HCVR FR4 framework amino acid sequence.
SEQ ID NO: 12 is homo sapiens LCVR FR1 framework amino acid sequence.
SEQ ID NOs: 13-36 are homo sapiens LCVR FR2 framework amino acid sequences.
SEQ ID NO: 37 is homo sapiens LCVR FR3 framework amino acid sequence.
SEQ ID NO: 38 is homo sapiens LCVR FR4 framework amino acid sequence.
SEQ ID NO: 39 is homo sapiens IgG1 heavy chain constant region amino acid sequence.
SEQ ID NO: 40 is homo sapiens IgG4 heavy chain constant region amino acid sequence.
SEQ ID NO: 41 is homo sapiens kappa chain constant region amino acid sequence.
SEQ ID NOs: 42-86 are artificial light chain variable region amino acid sequences.
SEQ ID NOs: 87-125 are artificial HCVR amino acid sequences.

## Claims

1. An antibody or an antigen-binding fragment thereof which neutralizes human TGF-β1 and has a K_{d} of less than 40pM for human TGF-β1, comprising a heavy chain variable region and a light chain variable region selected from the group consisting of:
i) a heavy chain variable region comprising the amino acid sequence shown in SEQ ID NO: 90 and a light chain variable region comprising the amino acid sequence shown in SEQ ID NO: 43;
ii) a heavy chain variable region comprising the amino acid sequence shown in SEQ ID NO: 92 and a light chain variable region comprising the amino acid sequence shown in SEQ ID NO: 42;
iii) a heavy chain variable region comprising the amino acid sequence shown in SEQ ID NO: 107 and a light chain variable region comprising the amino acid sequence shown in SEQ ID NO: 57; and
iv) a heavy chain variable region comprising the amino acid sequence shown in SEQ ID NO: 119 and a light chain variable region comprising the amino acid sequence shown in SEQ ID NO: 73.

2. An antibody or an antigen-binding fragment thereof according to claim 1, comprising a heavy chain variable region having the amino acid sequence shown in SEQ ID NO: 90 and a light chain variable region having the amino acid sequence shown in SEQ ID NO: 43.

3. The antibody or antigen-binding fragment thereof of claim 1 or claim 2, further comprising an IgG4 constant region having the amino acid sequence shown in SEQ ID NO: 40 and a kappa chain constant region having the amino acid sequence shown in SEQ ID NO: 41.

4. A pharmaceutical composition, comprising an antibody or an antigen-binding fragment thereof according to any one of claims 1 to 3 and a pharmaceutically acceptable carrier.

5. An antibody or an antigen-binding fragment thereof according to any one of claims 1 to 3 for use as a medicament.

6. An antibody or an antigen-binding fragment thereof according to any one of claims 1 to 3 for use in the treatment of a fibrotic disease in a human subject.

7. An antibody or an antigen-binding fragment thereof according to any one of claims 1 to 3 for use in the treatment of a chronic renal disease in a human subject.

8. An antibody or an antigen-binding fragment thereof according to any one of claims 1 to 3 for use in combination therapy for treating a fibrotic disease in a human subject, wherein said antibody or antigen-binding fragment thereof is administered in combination with an ACE inhibitor.

9. An antibody or an antigen-binding fragment thereof according to any one of claims 1 to 3 for use in combination therapy for treating a chronic renal disease in a human subject, wherein said antibody or antigen-binding fragment thereof is to be administered in combination with an ACE inhibitor.

10. An antibody or antigen-binding fragment thereof according to any one of claims 1 to 3 for use in the treatment of cancer.

## Patentansprüche

1. Antikörper oder antigenbindendes Fragment hiervon, der bzw. das humanen TGF-β1 neutralisiert und einen K_{d}-Wert von weniger als 40 pM für humanen TGF-β1 aufweist, umfassend eine variable Region einer schweren Kette und eine variable Region einer leichten Kette, die aus der Gruppe ausgewählt sind, die aus den folgenden besteht:
i) variable Region einer schweren Kette, die die in SEQ ID Nr. 90 dargestellte Aminosäuresequenz umfasst, und variable Region einer leichten Kette, die die in SEQ ID Nr. 43 dargestellte Aminosäuresequenz umfasst;
ii) variable Region einer schweren Kette, die die in SEQ ID Nr. 92 dargestellte Aminosäuresequenz umfasst, und variable Region einer leichten Kette, die die in SEQ ID Nr. 42 dargestellte Aminosäuresequenz umfasst;
iii) variable Region einer schweren Kette, die die in SEQ ID Nr. 107 dargestellte Aminosäuresequenz umfasst, und variable Region einer leichten Kette, die die in SEQ ID Nr. 57 dargestellte Aminosäuresequenz umfasst; und
iv) variable Region einer schweren Kette, die die in SEQ ID Nr. 119 dargestellte Aminosäuresequenz umfasst, und variable Region einer leichten Kette, die die in SEQ ID Nr. 73 dargestellte Aminosäuresequenz umfasst.

2. Antikörper oder antigenbindendes Fragment hiervon nach Anspruch 1, umfassend eine variable Region einer schweren Kette mit der in SEQ ID Nr. 90 dargestellten Aminosäuresequenz und eine variable Region einer leichten Kette mit der in SEQ ID Nr. 43 dargestellten Aminosäuresequenz.

3. Antikörper oder antigenbindendes Fragment hiervon nach Anspruch 1 oder Anspruch 2, der bzw. das des weiteren eine konstante Region von IgG4 mit der in SEQ ID Nr. 40 dargestellten Aminosäuresequenz und eine konstante Region einer Kappa-Kette mit der in SEQ ID Nr. 41 dargestellten Aminosäuresequenz umfasst.

4. Pharmazeutische Zusammensetzung, die einen Antikörper oder ein antigenbindendes Fragment hiervon nach einem der Ansprüche 1 bis 3 und einen pharmazeutisch akzeptablen Träger umfasst.

5. Antikörper oder antigenbindendes Fragment hiervon nach einem der Ansprüche 1 bis 3 zur Verwendung als Medikament.

6. Antikörper oder antigenbindendes Fragment hiervon nach einem der Ansprüche 1 bis 3 zur Verwendung bei der Behandlung einer fibrotischen Erkrankung bei einem menschlichen Patienten.

7. Antikörper oder antigenbindendes Fragment hiervon nach einem der Ansprüche 1 bis 3 zur Verwendung bei der Behandlung einer chronischen Nierenerkrankung bei einem menschlichen Patienten.

8. Antikörper oder antigenbindendes Fragment hiervon nach einem der Ansprüche 1 bis 3 zur Verwendung in einer Kombinationstherapie zur Behandlung einer fibrotischen Erkrankung bei einem menschlichen Patienten, wobei der Antikörper oder das antigenbindende Fragment hiervon in Kombination mit einem ACE-Inhibitor verabreicht wird.

9. Antikörper oder antigenbindendes Fragment hiervon nach einem der Ansprüche 1 bis 3 zur Verwendung in einer Kombinationstherapie zur Behandlung einer chronischen Nierenerkrankung bei einem menschlichen Patienten, wobei der Antikörper oder das antigenbindende Fragment hiervon in Kombination mit einem ACE-Inhibitor zu verabreichen ist.

10. Antikörper oder antigenbindendes Fragment hiervon nach einem der Ansprüche 1 bis 3 zur Verwendung bei der Behandlung von Krebs.

## Revendications

1. Anticorps ou fragment de celui-ci se liant à l'antigène qui neutralise le TGF-β1 humain et a une Kd de moins de 40 pM pour le TGF-β1 humain, comprenant une région variable de chaîne lourde et une région variable de chaîne légère choisies dans le groupe constitué par :
i) une région variable de chaîne lourde comprenant la séquence d'acides aminés présentée dans SEQ ID NO : 90 et une région variable de chaîne légère comprenant la séquence d'acides aminés présentée dans SEQ ID NO : 43.
ii) une région variable de chaîne lourde comprenant la séquence d'acides aminés présentée dans SEQ ID NO : 92 et une région variable de chaîne légère comprenant la séquence d'acides aminés présentée dans SEQ ID NO : 42.
iii) une région variable de chaîne lourde comprenant la séquence d'acides aminés présentée dans SEQ ID NO : 107 et une région variable de chaîne légère comprenant la séquence d'acides aminés présentée dans SEQ ID NO : 57 ; et
iv) une région variable de chaîne lourde comprenant la séquence d'acides aminés présentée dans SEQ ID NO : 119 et une région variable de chaîne légère comprenant la séquence d'acides aminés présentée dans SEQ ID NO : 73.

2. Anticorps ou fragment de celui-ci se liant à l'antigène selon la revendication 1, comprenant une région variable de chaîne lourde ayant la séquence d'acides aminés présentée dans SEQ ID NO : 90 et une région variable de chaîne légère ayant la séquence d'acides aminés présentée dans SEQ ID NO : 43.

3. Anticorps ou fragment de celui-ci se liant à l'antigène selon la revendication 1 ou la revendication 2, comprenant en outre une région constante d'lgG4 ayant la séquence d'acides aminés présentée dans SEQ ID NO : 40 et une région constante de chaîne kappa ayant la séquence d'acides aminés présentée dans SEQ ID NO : 41

4. Composition pharmaceutique, comprenant un anticorps ou un fragment de celui-ci se liant à l'antigène selon l'une quelconque des revendications 1 à 3 et un support pharmaceutiquement acceptable.

5. Anticorps ou fragment de celui-ci se liant à l'antigène selon l'une quelconque des revendications 1 à 3 pour une utilisation en tant que médicament.

6. Anticorps ou fragment de celui-ci se liant à l'antigène selon l'une quelconque des revendications 1 à 3 pour une utilisation dans le traitement d'une maladie fibrotique chez un sujet humain.

7. Anticorps ou fragment de celui-ci se liant à l'antigène selon l'une quelconque des revendications 1 à 3 pour une utilisation dans le traitement d'une maladie rénale chronique chez un sujet humain.

8. Anticorps ou fragment de celui-ci se liant à l'antigène selon l'une quelconque des revendications 1 à 3 pour une utilisation dans une thérapie en combinaison destinée à traiter une maladie fibrotique chez un sujet humain, dans laquelle ledit anticorps ou fragment de celui-ci se liant à l'antigène est administré en combinaison avec un inhibiteur de l'ECA.

9. Anticorps ou fragment de celui-ci se liant à l'antigène selon l'une quelconque des revendications 1 à 3 pour une utilisation dans une thérapie en combinaison destinée à traiter une maladie rénale chronique chez un sujet humain, dans laquelle ledit anticorps ou fragment de celui-ci se liant à l'antigène doit être administré en association avec un inhibiteur de l'ECA.

10. Anticorps ou fragment de celui-ci se liant à l'antigène selon l'une quelconque des revendications 1 à 3 pour une utilisation dans le traitement du cancer.


### SEQUENCE LISTING

<110> Eli Lilly and company
<120> Binding compositions; Related Reagents
<130> X-16598
<160> 135
<170> PatentIn version 3.3
<210> 1
   <211> 112
   <212> PRT
   <213> Primate
<400> 1
   Ala Leu Asp Thr Asn Tyr Cys Phe Ser Ser Thr Glu Lys Asn Cys Cys
   1 5 10 15
   Val Arg Gln Leu Tyr Ile Asp Phe Arg Lys Asp Leu Gly Trp Lys Trp
   20 25 30
   Ile His Glu Pro Lys Gly Tyr His Ala Asn Phe Cys Leu Gly Pro Cys
   35 40 45
   Pro Tyr Ile Trp Ser Leu Asp Thr Gln Tyr Ser Lys Val Leu Ala Leu
   50 55 60
   Tyr Asn Gln His Asn Pro Gly Ala Ser Ala Ala Pro Cys Cys Val Pro
   65 70 75 80
   Gln Ala Leu Glu Pro Leu Pro Ile Val Tyr Tyr Val Gly Arg Lys Pro
   85 90 95
   Lys Val Glu Gln Leu Ser Asn Met Ile Val Arg Ser Cys Lys Cys Ser
   100 105 110
<210> 2
   <211> 10
   <212> PRT
   <213> Primate
<220>
   <221> MISC_FEATURE
   <222> (3)..(3)
   <223> xaa at position 3 = Thr or Asp
<220>
   <221> MISC_FEATURE
   <222> (5)..(5)
   <223> xaa at position 5 = Thr, Glu, or Phe
<220>
   <221> MISC_FEATURE
   <222> (9)..(9)
   <223> Xaa at position 9 = Met, Ile, Leu, or Val
<220>
   <221> MISC_FEATURE
   <222> (10)..(10)
   <223> Xaa at position 10 = His, val, or Ala
<400> 2
   Gly Tyr Xaa Phe Xaa Asp Tyr Asn Xaa Xaa
   1 5 10
<210> 3
   <211> 17
   <212> PRT
   <213> Primate
<220>
   <221> MISC_FEATURE
   <222> (1)..(1)
   <223> Xaa at position 1 = Tyr, Gln, or Ser
<220>
   <221> MISC_FEATURE
   <222> (2)..(2)
   <223> xaa at position 2 = Ile or Val
<220>
   <221> MISC_FEATURE
   <222> (8)..(8)
   <223> Xaa at position 8 = Asp or Glu
<220>
   <221> MISC_FEATURE
   <222> (11) .. (11)
   <223> xaa at position 11 = Tyr, Thr, His, or Leu
<220>
   <221> MISC_FEATURE
   <222> (13)..(13)
   <223> xaa at position 13 = Gln, Lys, Pro, or Ser
<220>
   <221> MISC_FEATURE
   <222> (15)..(15)
   <223> Xaa at position 15 = Phe or Tyr
<400> 3
   Xaa Xaa Tyr Pro Tyr Asp Gly Xaa Thr Gly Xaa Asn Xaa Lys Xaa Lys
   1               5                   10                  15
   Ser
<210> 4
   <211> 7
   <212> PRT
   <213> Primate
   <220>
   <221> MISC_FEATURE
   <222> (4)..(4)
   <223> xaa at position 4 = Trp or Ala
<220>
   <221> MISC_FEATURE
   <222> (5)..(5)
   <223> xaa at position 5 = Phe or Leu
<220>
   <221> MISC_FEATURE
   <222> (6)..(6)
   <223> Xaa at position 6 = Ala, Glu, or Tyr
<400> 4
   Gly Tyr Arg Xaa Xaa Xaa Tyr
   1               5
<210> 5
   <211> 10
   <212> PRT
   <213> Primate
<220>
   <221> MISC_FEATURE
   <222> (1)..(1)
   <223> Xaa at postion 1 = Arg, Tyr, Glu, or Gln
<220>
   <221> MISC_FEATURE
   <222> (3)..(3)
   <223> Xaa at postion 3 = Ser or Thr
<220>
   <221> MISC_FEATURE
   <222> (4)..(4)
   <223> Xaa at postion 4 = Ser, val, or Ala
<220>
   <221> MISC_FEATURE
   <222> (5)..(5)
   <223> Xaa at postion 5 = Ser or Leu
<220>
   <221> MISC_FEATURE
   <222> (7)..(7)
   <223> Xaa at postion 7 = ser, Pro, Leu, or Tyr
<400> 5
   Xaa Ala Xaa Xaa Xaa Val Xaa Tyr Met His
   1               5                   10
<210> 6
   <211> 7
   <212> PRT
   <213> Primate
   <220>
   <221> MISC_FEATURE
   <222> (5)..(5)
   <223> Xaa at position 5 = Leu, Asn, or Pro <220>

   <221> MISC_FEATURE
   <222> (7)..(7)
   <223> xaa at position 7 = Ser, Lys, Tyr, Leu, Met, Phe, Glu, Gln, Arg, or His
<400> 6
   Ala Thr Ser Asn Xaa Ala Xaa
   1 5
<210> 7
   <211> 9
   <212> PRT
   <213> Primate
<220>
   <221> MISC_FEATURE
   <222> (1)..(1)
   <223> xaa at position 1 = Gln or Ser
<220>
   <221> MISC_FEATURE
   <222> (5)..(5)
   <223> Xaa at position 5 = Leu, Asp, or Pro
   <220>
   <221> MISC_FEATURE
   <222> (6)..(6)
   <223> Xaa at position 6 = Asn or Arg
   <220>
   <221> MISC_FEATURE
   <222> (7)..(7)
   <223> xaa at position 7 = Pro, Phe, Tyr, or Arg
<400> 7
   Xaa Gln Trp Asp Xaa Xaa Xaa Pro Ala
   1 5
<210> 8
   <211> 25
   <212> PRT
   <213> Primate
<400> 8
<210> 9
   <211> 14
   <212> PRT
   <213> Primate
<400> 9
   Trp Val Arg Gln Ala Pro Gly Gln Gly Leu Glu Trp Met Gly
   1 5 10
<210> 10
   <211> 30
   <212> PRT
   <213> Primate
<400> 10
   Thr Met Thr Thr Asp Thr Ser Thr Ser Thr Ala Tyr Met Glu Leu Arg
   1 5 10 15
   Ser Leu Arg Ser Asp Asp Thr Ala Val Tyr Tyr Cys Ala Arg
   20 25 30
<210> 11
   <211> 11
   <212> PRT
   <213> Primate
<400> 11
   Trp Gly Gln Gly Thr Leu Val Thr Val Ser Ser
   1 5 10
<210> 12
   <211> 23
   <212> PRT
   <213> Primate
<400> 12
   Asp Ile Gln Met Thr Gln Ser Pro Ser Ser Leu Ser Ala Ser Val Gly
   1 5 10 15
   Asp Arg Val Thr Ile Thr Cys
   20
<210> 13
   <211> 15
   <212> PRT
   <213> Primate
<400> 13
   Trp Tyr Gln Gln Lys Pro Gly Lys Ala Pro Lys Pro Leu Ile Tyr
   1 5 10 15
<210> 14
   <211> 15
   <212> PRT
   <213> Primate
<400> 14
   Trp Tyr Gln Gln Lys Pro Gly Lys Ala Pro Lys Leu Leu Ile Tyr
   1               5                   10                  15
<210> 15
   <211> 15
   <212> PRT
   <213> Primate
<400> 15
   Trp Tyr Gln Gln Lys Pro Gly Lys Ala Pro Lys Ser Leu Ile Tyr
   1               5                   10                  15
<210> 16
   <211> 15
   <212> PRT
   <213> Primate
<400> 16
   Trp Tyr Gln Gln Lys Pro Glu Lys Ala Pro Lys Pro Leu Ile Tyr
   1               5                   10                  15
<210> 17
   <211> 15
   <212> PRT
   <213> Primate
<400> 17
   Trp Tyr Gln Gln Lys Pro Glu Lys Ala Pro Lys Leu Leu Ile Tyr
   1               5                   10                  15
<210> 18
   <211> 15
   <212> PRT
   <213> Primate
<400> 18
   Trp Tyr Gln Gln Lys Pro Glu Lys Ala Pro Lys Ser Leu Ile Tyr
   1               5                   10                  15
<210> 19
   <211> 15
   <212> PRT
   <213> Primate
<400> 19
   Trp Phe Gln Gln Lys Pro Gly Lys Ala Pro Lys Pro Leu Ile Tyr
   1 5 10 15
<210> 20
   <211> 15
   <212> PRT
   <213> Primate
<400> 20
   Trp Phe Gln Gln Lys Pro Gly Lys Ala Pro Lys Leu Leu Ile Tyr
   1 5 10 15
<210> 21
   <211> 15
   <212> PRT
   <213> Primate
<400> 21
   Trp Phe Gln Gln Lys Pro Gly Lys Ala Pro Lys Ser Leu Ile Tyr
   1 5 10 15
<210> 22
   <211> 15
   <212> PRT
   <213> Primate
<400> 22
   Trp Phe Gln Gln Lys Pro Glu Lys Ala Pro Lys Pro Leu Ile Tyr
   1 5 10 15
<210> 23
   <211> 15
   <212> PRT
   <213> Primate
<400> 23
   Trp Phe Gln Gln Lys Pro Glu Lys Ala Pro Lys Leu Leu Ile Tyr
   1 5 10 15
<210> 24
   <211> 15
   <212> PRT
   <213> Primate
<400> 24
   Trp Phe Gln Gln Lys Pro Glu Lys Ala Pro Lys Ser Leu Ile Tyr
   1 5 10 15
<210> 25
   <211> 15
   <212> PRT
   <213> Primate
<400> 25
   Trp Tyr Gln Gln Lys Pro Gly Lys Ala Pro Lys Pro Trp Ile Tyr
   1 5 10 15
<210> 26
   <211> 15
   <212> PRT
   <213> Primate
<400> 26
   Trp Tyr Gln Gln Lys Pro Gly Lys Ala Pro Lys Leu Trp Ile Tyr
   1 5 10 15
<210> 27
   <211> 15
   <212> PRT
   <213> Primate
<400> 27
   Trp Tyr Gln Gln Lys Pro Gly Lys Ala Pro Lys Ser Trp Ile Tyr
   1 5 10 15
<210> 28
   <211> 15
   <212> PRT
   <213> Primate
<400> 28
   Trp Tyr Gln Gln Lys Pro Glu Lys Ala Pro Lys Pro Trp Ile Tyr
   1 5 10 15
<210> 29
   <211> 15
   <212> PRT
   <213> Primate
<400> 29
   Trp Tyr Gln Gln Lys Pro Glu Lys Ala Pro Lys Leu Trp Ile Tyr
   1 5 10 15
<210> 30
   <211> 15
   <212> PRT
   <213> Primate
<400> 30
   Trp Tyr Gln Gln Lys Pro Glu Lys Ala Pro Lys Ser Trp Ile Tyr
   1 5 10 15
<210> 31
   <211> 15
   <212> PRT
   <213> Primate
<400> 31
   Trp Phe Gln Gln Lys Pro Gly Lys Ala Pro Lys Pro Trp Ile Tyr
   1 5 10 15
<210> 32
   <211> 15
   <212> PRT
   <213> Primate
<400> 32
   Trp Phe Gln Gln Lys Pro Gly Lys Ala Pro Lys Leu Trp Ile Tyr
   1 5 10 15
<210> 33
   <211> 15
   <212> PRT
   <213> Primate
<400> 33
   Trp Phe Gln Gln Lys Pro Gly Lys Ala Pro Lys Ser Trp Ile Tyr
   1 5 10 15
<210> 34
   <211> 15
   <212> PRT
   <213> Primate
<400> 34
   Trp Phe Gln Gln Lys Pro Glu Lys Ala Pro Lys Pro Trp Ile Tyr
   1 5 10 15
<210> 35
   <211> 15
   <212> PRT
   <213> Primate
<400> 35
   Trp Phe Gln Gln Lys Pro Glu Lys Ala Pro Lys Leu Trp Ile Tyr
   1 5 10 15
<210> 36
   <211> 15
   <212> PRT
   <213> Primate
<400> 36
   Trp Phe Gln Gln Lys Pro Glu Lys Ala Pro Lys Ser Trp Ile Tyr
   1 5 10 15
<210> 37
   <211> 32
   <212> PRT
   <213> Primate
<400> 37
   Gly Val Pro Ser Arg Phe Ser Gly Ser Gly Ser Gly Thr Asp Phe Thr
   1 5 10 15
   Leu Thr Ile Ser Ser Leu Gln Pro Glu Asp Phe Ala Thr Tyr Tyr Cys
   20 25 30
<210> 38
   <211> 10
   <212> PRT
   <213> Primate
<400> 38
   Phe Gly Gln Gly Thr Lys Leu Glu Ile Lys
   1 5 10
<210> 39
   <211> 328
   <212> PRT
   <213> Primate
<400> 39
   Ser Thr Lys Gly Pro Ser Val Phe Pro Leu Ala Pro Ser Ser Lys Ser
   1               5                   10                  15
   Thr Ser Gly Gly Thr Ala Ala Leu Gly Cys Leu Val Lys Asp Tyr Phe
               20                  25                  30
   Pro Glu Pro Val Thr Val Ser Trp Asn Ser Gly Ala Leu Thr Ser Gly
           35                  40                  45
   Val His Thr Phe Pro Ala Val Leu Gln Ser Ser Gly Leu Tyr Ser Leu
       50                  55                  60
   Ser Ser Val Val Thr Val Pro Ser Ser Ser Leu Gly Thr Gln Thr Tyr
   65                  70                  75                  80
   Ile Cys Asn Val Asn His Lys Pro Ser Asn Thr Lys Val Asp Lys Lys
                   85                  90                  95
   Val Glu Pro Lys Ser Cys Asp Lys Thr His Thr Cys Pro Pro Cys Pro
               100                 105                 110
   Ala Pro Glu Leu Leu Gly Gly Pro Ser Val Phe Leu Phe Pro Pro Lys
           115                 120                 125
   Pro Lys Asp Thr Leu Met Ile Ser Arg Thr Pro Glu Val Thr Cys Val
       130                 135                 140
   Val Val Asp Val Ser His Glu Asp Pro Glu Val Lys Phe Asn Trp Tyr
   145                 150                 155                 160
   Val Asp Gly Val Glu Val His Asn Ala Lys Thr Lys Pro Arg Glu Glu
                   165                 170                 175
   Gln Tyr Asn Ser Thr Tyr Arg Val Val Ser Val Leu Thr Val Leu His
               180                 185                 190
   Gln Asp Trp Leu Asn Gly Lys Glu Tyr Lys Cys Lys Val Ser Asn Lys
           195                 200                 205
   Ala Leu Pro Ala Pro Ile Glu Lys Thr Ile Ser Lys Ala Lys Gly Gln
       210                 215                 220
   Pro Arg Glu Pro Gln Val Tyr Thr Leu Pro Pro Ser Arg Asp Glu Leu
   225                 230                 235                 240
   Thr Lys Asn Gln Val Ser Leu Thr Cys Leu Val Lys Gly Phe Tyr Pro
                   245                 250                 255
   Ser Asp Ile Ala Val Glu Trp Glu Ser Asn Gly Gln Pro Glu Asn Asn
               260                 265                 270
   Tyr Lys Thr Thr Pro Pro Val Leu Asp Ser Asp Gly Ser Phe Phe Leu
           275                 280                 285
   Tyr Ser Lys Leu Thr Val Asp Lys Ser Arg Trp Gln Gln Gly Asn Val
       290                 295                 300
   Phe Ser Cys Ser Val Met His Glu Ala Leu His Asn His Tyr Thr Gln
   305                 310                 315                 320
   Lys Ser Leu Ser Leu Ser Pro Gly
                   325
<210> 40
   <211> 326
   <212> PRT
   <213> Primate
<400> 40
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Arg Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu Gly
   325
<210> 41
   <211> 107
   <212> PRT
   <213> Primate
<400> 41
<210> 42
   <211> 106
   <212> PRT
   <213> Primate
<400> 42
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Ser Tyr Met
               20                  25                  30
   His Trp Tyr Gln Gln Lys Pro Gly Lys Ala Pro Lys Pro Leu Ile Tyr
           35                  40                  45
   Ala Thr Ser Asn Leu Ala Lys Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 43
   <211> 106
   <212> PRT
   <213> Primate
<400> 43
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Ser Tyr Met
               20                  25                  30
   His Trp Tyr Gln Gln Lys Pro Gly Lys Ala Pro Lys Pro Leu Ile Tyr
           35                  40                  45
   Ala Thr Ser Asn Leu Ala Tyr Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
   100 105
<210> 44
   <211> 106
   <212> PRT
   <213> Primate
<400> 44
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Pro Leu Ile Tyr
   35 40 45
   Ala Thr Ser Asn Leu Ala Leu Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 45
   <211> 106
   <212> PRT
   <213> Primate
<400> 45
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Leu Val Ser Tyr Met
   20 25 30
   His Trp Tyr Gln Gln Lys Pro Gly Lys Ala Pro Lys Pro Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 46
   <211> 106
   <212> PRT
   <213> Primate
<400> 46
   Asp Ile Gln Met Thr Gln Ser Pro Ser Ser Leu Ser Ala Ser Val Gly
   1               5                   10                  15
   Asp Arg Val Thr Ile Thr Cys Tyr Ala Ser Ser Ser Val Ser Tyr Met
               20                  25                  30
   His Trp Tyr Gln Gln Lys Pro Gly Lys Ala Pro Lys Pro Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 47
   <211> 106
   <212> PRT
   <213> Primate
<400> 47
   Asp Ile Gln Met Thr Gln Ser Pro Ser Ser Leu Ser Ala Ser Val Gly
   1               5                   10                  15
   Asp Arg Val Thr Ile Thr Cys Glu Ala Ser Ser Ser Val Ser Tyr Met
               20                  25                  30
   His Trp Tyr Gln Gln Lys Pro Gly Lys Ala Pro Lys Pro Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 48
   <211> 106
   <212> PRT
   <213> Primate
<400> 48
   Asp Ile Gln Met Thr Gln Ser Pro Ser Ser Leu Ser Ala Ser Val Gly
   1 5 10 15
   Asp Arg Val Thr Ile Thr Cys Gln Ala Ser Ser Ser Val Ser Tyr Met
   20 25 30
   His Trp Tyr Gln Gln Lys Pro Gly Lys Ala Pro Lys Pro Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 49
   <211> 106
   <212> PRT
   <213> Primate
<400> 49
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Pro Leu Ile Tyr
   35 40 45
   Ala Thr Ser Asn Asn Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 50
   <211> 106
   <212> PRT
   <213> Primate
<400> 50
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Pro Leu Ile Tyr
   35 40 45
   Ala Thr Ser Asn Leu Ala Met Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 51
   <211> 106
   <212> PRT
   <213> Primate
<400> 51
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Ser Tyr Met
               20                  25                  30
   His Trp Tyr Gln Gln Lys Pro Gly Lys Ala Pro Lys Pro Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 52
   <211> 106
   <212> PRT
   <213> Primate
<400> 52
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Pro Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Phe Pro Ala
   85 90 95
   Phe Gly Gln Gly Thr Lys Leu Glu Ile Lys
   100 105
<210> 53
   <211> 106
   <212> PRT
   <213> Primate
<400> 53
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Pro Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Asp Asn Pro Pro Ala
   85 90 95
   Phe Gly Gln Gly Thr Lys Leu Glu Ile Lys
   100 105
<210> 54
   <211> 106
   <212> PRT
   <213> Primate
<400> 54
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Ser Tyr Met
               20                  25                  30
   His Trp Tyr Gln Gln Lys Pro Gly Lys Ala Pro Lys Pro Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Arg Pro Pro Ala
                   85                  90                  95
   Phe Gly Gln Gly Thr Lys Leu Glu Ile Lys
               100                 105
<210> 55
   <211> 106
   <212> PRT
   <213> Primate
<400> 55
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Ser Tyr Met
               20                  25                  30
   His Trp Tyr Gln Gln Lys Pro Gly Lys Ala Pro Lys Pro Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Ser Gln Trp Asp Leu Asn Pro Pro Ala
                   85                  90                  95
   Phe Gly Gln Gly Thr Lys Leu Glu Ile Lys
               100                 105
<210> 56
   <211> 106
   <212> PRT
   <213> Primate
<400> 56
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Pro Tyr Met
               20                  25                  30
   His Trp Tyr Gln Gln Lys Pro Gly Lys Ala Pro Lys Leu Leu Ile Tyr
   35 40 45
   Ala Thr Ser Asn Leu Ala Tyr Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 57
   <211> 106
   <212> PRT
   <213> Primate
<400> 57
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Pro Tyr Met
   20 25 30
   His Trp Tyr Gln Gln Lys Pro Gly Lys Ala Pro Lys Leu Leu Ile Tyr
   35 40 45
   Ala Thr Ser Asn Pro Ala Tyr Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 58
   <211> 106
   <212> PRT
   <213> Primate
<400> 58
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Pro Tyr Met
               20                  25                  30
   His Trp Tyr Gln Gln Lys Pro Gly Lys Ala Pro Lys Leu Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 59
   <211> 106
   <212> PRT
   <213> Primate
<400> 59
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Pro Tyr Met
               20                  25                  30
   His Trp Tyr Gln Gln Lys Pro Gly Lys Ala Pro Lys Leu Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 60
   <211> 106
   <212> PRT
   <213> Primate
<400> 60
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Pro Tyr Met
               20                  25                  30
   His Trp Tyr Gln Gln Lys Pro Gly Lys Ala Pro Lys Leu Leu Ile Tyr
           35                  40                  45
   Ala Thr Ser Asn Leu Ala Lys Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 61
   <211> 106
   <212> PRT
   <213> Primate
<400> 61
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Pro Tyr Met
   20 25 30
   His Trp Tyr Gln Gln Lys Pro Gly Lys Ala Pro Lys Leu Leu Ile Tyr
   35 40 45
   Ala Thr Ser Asn Leu Ala Phe Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 62
   <211> 106
   <212> PRT
   <213> Primate
<400> 62
   Asp Ile Gln Met Thr Gln Ser Pro Ser Ser Leu Ser Ala Ser Val Gly
   1 5 10 15
   Asp Arg Val Thr Ile Thr Cys Arg Ala Ser Val Ser Val Ser Tyr Met
   20 25 30
   His Trp Tyr Gln Gln Lys Pro Gly Lys Ala Pro Lys Leu Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 63
   <211> 106
   <212> PRT
   <213> Primate
<400> 63
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Leu Tyr Met
   20 25 30
   His Trp Tyr Gln Gln Lys Pro Gly Lys Ala Pro Lys Leu Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 64
   <211> 106
   <212> PRT
   <213> Primate
<400> 64
   Asp Ile Gln Met Thr Gln Ser Pro Ser Ser Leu Ser Ala Ser Val Gly
   1               5                   10                  15
   Asp Arg Val Thr Ile Thr Cys Arg Ala Thr Ser Ser Val Leu Tyr Met
               20                  25                  30
   His Trp Tyr Gln Gln Lys Pro Gly Lys Ala Pro Lys Leu Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 65
   <211> 106
   <212> PRT
   <213> Primate
<400> 65
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Tyr Tyr Met
               20                  25                  30
   His Trp Tyr Gln Gln Lys Pro Gly Lys Ala Pro Lys Leu Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 66
   <211> 106
   <212> PRT
   <213> Primate
<400> 66
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Leu Leu Ile Tyr
   35 40 45
   Ala Thr Ser Asn Leu Ala Glu Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 67
   <211> 106
   <212> PRT
   <213> Primate
<400> 67
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Leu Leu Ile Tyr
   35 40 45
   Ala Thr Ser Asn Leu Ala Gln Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 68
   <211> 106
   <212> PRT
   <213> Primate
<400> 68
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Leu Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Tyr Pro Ala
   85 90 95
   Phe Gly Gln Gly Thr Lys Leu Glu Ile Lys
   100 105
<210> 69
   <211> 106
   <212> PRT
   <213> Primate
<400> 69
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Leu Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 70
   <211> 106
   <212> PRT
   <213> Primate
<400> 70
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Pro Tyr Met
               20                  25                  30
   His Trp Tyr Gln Gln Lys Pro Glu Lys Ala Pro Lys Ser Leu Ile Tyr
           35                  40                  45
   Ala Thr Ser Asn Leu Ala Tyr Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 71
   <211> 106
   <212> PRT
   <213> Primate
<400> 71
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Pro Tyr Met
   20 25 30
   His Trp Tyr Gln Gln Lys Pro Glu Lys Ala Pro Lys Ser Leu Ile Tyr
   35 40 45
   Ala Thr Ser Asn Leu Ala Leu Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 72
   <211> 106
   <212> PRT
   <213> Primate
<400> 72
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Pro Tyr Met
   20 25 30
   His Trp Tyr Gln Gln Lys Pro Glu Lys Ala Pro Lys Ser Leu Ile Tyr
   35 40 45
   Ala Thr Ser Asn Leu Ala Lys Gly Val Pro Ser Arg Phe Ser Gly Ser
   50 55 60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 73
   <211> 106
   <212> PRT
   <213> Primate
<400> 73
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Pro Tyr Met
               20                  25                  30
   His Trp Tyr Gln Gln Lys Pro Glu Lys Ala Pro Lys Ser Leu Ile Tyr
           35                  40                  45
   Ala Thr Ser Asn Leu Ala Phe Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 74
   <211> 106
   <212> PRT
   <213> Primate
<400> 74
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ala Ser Val Pro Tyr Met
               20                  25                  30
   His Trp Tyr Gln Gln Lys Pro Glu Lys Ala Pro Lys Ser Leu Ile Tyr
           35                  40                  45
   Ala Thr Ser Asn Leu Ala Lys Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 75
   <211> 106
   <212> PRT
   <213> Primate
<400> 75
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ala Ser Val Pro Tyr Met
   20 25 30
   His Trp Tyr Gln Gln Lys Pro Glu Lys Ala Pro Lys Ser Leu Ile Tyr
   35 40 45
   Ala Thr Ser Asn Leu Ala Tyr Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 76
   <211> 106
   <212> PRT
   <213> Primate
<400> 76
   Asp Ile Gln Met Thr Gln Ser Pro Ser Ser Leu Ser Ala Ser Val Gly
   1 5 10 15
   Asp Arg Val Thr Ile Thr Cys Arg Ala Arg Ser Ser Val Ser Tyr Met
   20 25 30
   His Trp Tyr Gln Gln Lys Pro Glu Lys Ala Pro Lys Ser Leu Ile Tyr
   35 40 45
   Ala Thr Ser Asn Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 77
   <211> 106
   <212> PRT
   <213> Primate
<400> 77
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Ser Tyr Met
               20                  25                  30
   His Trp Tyr Gln Gln Lys Pro Glu Lys Ala Pro Lys Ser Leu Ile Tyr
           35                  40                  45
   Ala Thr Ser Asn Leu Ala Arg Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 78
   <211> 106
   <212> PRT
   <213> Primate
<400> 78
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Ser Tyr Met
               20                  25                  30
   His Trp Tyr Gln Gln Lys Pro Glu Lys Ala Pro Lys Ser Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Arg Pro Ala
                   85                  90                  95
   Phe Gly Gln Gly Thr Lys Leu Glu Ile Lys
               100                 105
<210> 79
   <211> 106
   <212> PRT
   <213> Primate
<400> 79
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Ser Tyr Met
               20                  25                  30
   His Trp Tyr Gln Gln Lys Pro Glu Lys Ala Pro Lys Ser Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Pro Asn Pro Pro Ala
                   85                  90                  95
   Phe Gly Gln Gly Thr Lys Leu Glu Ile Lys
               100                 105
<210> 80
   <211> 106
   <212> PRT
   <213> Primate
<400> 80
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Ser Tyr Met
               20                  25                  30
   His Trp Tyr Gln Gln Lys Pro Glu Lys Ala Pro Lys Ser Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 81
   <211> 106
   <212> PRT
   <213> Primate
<400> 81
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Pro Tyr Met
               20                  25                  30
   His Trp Phe Gln Gln Lys Pro Glu Lys Ala Pro Lys Ser Leu Ile Tyr
           35                  40                  45
   Ala Thr Ser Asn Leu Ala Phe Gly Val Pro Ser Arg Phe Ser Gly Ser
       50                  55                  60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 82
   <211> 106
   <212> PRT
   <213> Primate
<400> 82
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Pro Tyr Met
   20 25 30
   His Trp Phe Gln Gln Lys Pro Glu Lys Ala Pro Lys Ser Leu Ile Tyr
   35 40 45
   Ala Thr Ser Asn Leu Ala Tyr Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 83
   <211> 106
   <212> PRT
   <213> Primate
<400> 83
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Pro Tyr Met
   20 25 30
   His Trp Phe Gln Gln Lys Pro Glu Lys Ala Pro Lys Ser Leu Ile Tyr
   35 40 45
   Ala Thr Ser Asn Leu Ala Leu Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 84
   <211> 106
   <212> PRT
   <213> Primate
<400> 84
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Pro Tyr Met
   20 25 30
   His Trp Phe Gln Gln Lys Pro Glu Lys Ala Pro Lys Ser Leu Ile Tyr
   35 40 45
   Ala Thr Ser Asn Leu Ala His Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 85
   <211> 106
   <212> PRT
   <213> Primate
<400> 85
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Pro Tyr Met
   20 25 30
   His Trp Phe Gln Gln Lys Pro Glu Lys Ala Pro Lys Ser Leu Ile Tyr
   35 40 45
   Ala Thr Ser Asn Leu Ala Lys Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys
   100 105
<210> 86
   <211> 106
   <212> PRT
   <213> Primate
<400> 86
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Ser Ser Val Pro Tyr Met
               20                  25                  30
   His Trp Phe Gln Gln Lys Pro Glu Lys Ala Pro Lys Ser Leu Ile Tyr
           35                  40                  45
   Ala Thr Ser Asn Pro Ala Tyr Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys
               100                 105
<210> 87
   <211> 116
   <212> PRT
   <213> Primate
<400> 87
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Glu Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Leu Ala Tyr Trp Gly Gln Gly Thr Leu Val
   100 105 110
   Thr Val Ser Ser
   115
<210> 88
   <211> 116
   <212> PRT
   <213> Primate
<400> 88
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Asp Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 89
   <211> 115
   <212> PRT
   <213> Primate
<400> 89
   Val Gln Leu Val Gln Ser Gly Ala Glu Val Lys Lys Pro Gly Ala Ser
   1 5 10 15
   Val Lys Val Ser Cys Lys Ala Ser Gly Tyr Asp Phe Thr Asp Tyr Asn
   20 25 30
   Ile His Trp Val Arg Gln Ala Pro Gly Gln Gly Leu Glu Trp Met Gly
   35 40 45
   Tyr Ile Tyr Pro Tyr Asp Gly Glu Thr Gly Tyr Asn Gln Lys Phe Lys
   50 55 60
   Ser Arg Val Thr Met Thr Thr Asp Thr Ser Thr Ser Thr Ala Tyr Met
   65 70 75 80
   Glu Leu Arg Ser Leu Arg Ser Asp Asp Thr Ala Val Tyr Tyr Cys Ala
   85 90 95
   Arg Gly Tyr Arg Trp Phe Ala Tyr Trp Gly Gln Gly Thr Leu Val Thr
   100 105 110
   Val Ser Ser
   115
<210> 90
   <211> 116
   <212> PRT
   <213> Primate
<400> 90
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Asp Phe Thr Asp Tyr
               20                  25                  30
   Asn Ile His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Leu Ala Tyr Trp Gly Gln Gly Thr Leu Val
               100                 105                 110
   Thr Val Ser Ser
           115
<210> 91
   <211> 116
   <212> PRT
   <213> Primate
<400> 91
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Asp Phe Glu Asp Tyr
               20                  25                  30
   Asn Met His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Phe Ala Tyr Trp Gly Gln Gly Thr Leu Val
               100                 105                 110
   Thr Val Ser Ser
   115
<210> 92
   <211> 116
   <212> PRT
   <213> Primate
<400> 92
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Asp Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Leu Tyr Tyr Trp Gly Gln Gly Thr Leu Val
   100 105 110
   Thr Val Ser Ser
   115
<210> 93
   <211> 116
   <212> PRT
   <213> Primate
<400> 93
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Glu Asp Tyr
   20 25 30
   Asn Ile His Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 94
   <211> 116
   <212> PRT
   <213> Primate
<400> 94
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Asp Phe Thr Asp Tyr
               20                  25                  30
   Asn Leu His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Leu Ala Tyr Trp Gly Gln Gly Thr Leu Val
               100                 105                 110
   Thr Val Ser Ser
           115
<210> 95
   <211> 116
   <212> PRT
   <213> Primate
<400> 95
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Asp Phe Thr Asp Tyr
               20                  25                  30
   Asn Leu His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Phe Ala Tyr Trp Gly Gln Gly Thr Leu Val
               100                 105                 110
   Thr Val Ser Ser
           115
<210> 96
   <211> 116
   <212> PRT
   <213> Primate
<400> 96
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Met His Trp Val Arg Gln Ala Pro Gly Gln Gly Leu Glu Trp Met
           35                  40                  45
   Gly Tyr Ile Tyr Pro Tyr Asp Gly Asp Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 97
   <211> 116
   <212> PRT
   <213> Primate
<400> 97
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Met His Trp Val Arg Gln Ala Pro Gly Gln Gly Leu Glu Trp Met
   35 40 45
   Gly Tyr Val Tyr Pro Tyr Asp Gly Asp Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 98
   <211> 116
   <212> PRT
   <213> Primate
<400> 98
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Met His Trp Val Arg Gln Ala Pro Gly Gln Gly Leu Glu Trp Met
           35                  40                  45
   Gly Gln Ile Tyr Pro Tyr Asp Gly Asp Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Phe Ala Tyr Trp Gly Gln Gly Thr Leu Val
               100                 105                 110
   Thr Val Ser Ser
           115
<210> 99
   <211> 116
   <212> PRT
   <213> Primate
<400> 99
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Phe Asp Tyr
               20                  25                  30
   Asn Met His Trp Val Arg Gln Ala Pro Gly Gln Gly Leu Glu Trp Met
           35                  40                  45
   Gly Tyr Ile Tyr Pro Tyr Asp Gly Asp Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Phe Ala Tyr Trp Gly Gln Gly Thr Leu Val
               100                 105                 110
   Thr Val Ser Ser
           115
<210> 100
   <211> 116
   <212> PRT
   <213> Primate
<400> 100
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Asp Thr Gly Thr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 101
   <211> 116
   <212> PRT
   <213> Primate
<400> 101
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Asp Thr Gly His Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 102
   <211> 116
   <212> PRT
   <213> Primate
<400> 102
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Asp Thr Gly Leu Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 103
   <211> 116
   <212> PRT
   <213> Primate
<400> 103
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Met His Trp Val Arg Gln Ala Pro Gly Gln Gly Leu Glu Trp Met
           35                  40                  45
   Gly Tyr Ile Tyr Pro Tyr Asp Gly Asp Thr Gly Tyr Asn Lys Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Phe Ala Tyr Trp Gly Gln Gly Thr Leu Val
               100                 105                 110
   Thr Val Ser Ser
           115
<210> 104
   <211> 116
   <212> PRT
   <213> Primate
<400> 104
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Met His Trp Val Arg Gln Ala Pro Gly Gln Gly Leu Glu Trp Met
           35                  40                  45
   Gly Tyr Ile Tyr Pro Tyr Asp Gly Asp Thr Gly Ile Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Phe Ala Tyr Trp Gly Gln Gly Thr Leu Val
   100 105 110
   Thr Val Ser Ser
   115
<210> 105
   <211> 116
   <212> PRT
   <213> Primate
<400> 105
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Asp Phe Thr Asp Tyr
   20 25 30
   Asn Met Val Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Ala Phe Glu Tyr Trp Gly Gln Gly Thr Leu Val
   100 105 110
   Thr Val Ser Ser
   115
<210> 106
   <211> 116
   <212> PRT
   <213> Primate
<400> 106
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Asp Phe Thr Asp Tyr
   20 25 30
   Asn Met Val Trp Val Arg Gln Ala Pro Gly Gln Gly Leu Glu Trp Met
   35 40 45
   Gly Ser Ile Tyr Pro Tyr Asp Gly Glu Thr Gly Tyr Asn Pro Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Ala Phe Glu Tyr Trp Gly Gln Gly Thr Leu Val
   100 105 110
   Thr Val Ser Ser
   115
<210> 107
   <211> 116
   <212> PRT
   <213> Primate
<400> 107
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Asp Phe Thr Asp Tyr
               20                  25                  30
   Asn Met Val Trp Val Arg Gln Ala Pro Gly Gln Gly Leu Glu Trp Met
           35                  40                  45
   Gly Ser Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Ala Phe Glu Tyr Trp Gly Gln Gly Thr Leu Val
               100                 105                 110
   Thr Val Ser Ser
           115
<210> 108
   <211> 116
   <212> PRT
   <213> Primate
<400> 108
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Asp Phe Glu Asp Tyr
               20                  25                  30
   Asn Ile His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Ala Phe Glu Tyr Trp Gly Gln Gly Thr Leu Val
               100                 105                 110
   Thr Val Ser Ser
           115
<210> 109
   <211> 116
   <212> PRT
   <213> Primate
<400> 109
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Asp Phe Thr Asp Tyr
               20                  25                  30
   Asn Met His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
   85 90 95
   Ala Arg Gly Tyr Arg Ala Phe Glu Tyr Trp Gly Gln Gly Thr Leu Val
   100 105 110
   Thr Val Ser Ser
   115
<210> 110
   <211> 116
   <212> PRT
   <213> Primate
<400> 110
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Ile His Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Ala Phe Glu Tyr Trp Gly Gln Gly Thr Leu Val
   100 105 110
   Thr Val Ser Ser
   115
<210> 111
   <211> 116
   <212> PRT
   <213> Primate
<400> 111
<210> 112
   <211> 116
   <212> PRT
   <213> Primate
<400> 112
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Asp Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Ala Phe Glu Tyr Trp Gly Gln Gly Thr Leu Val
   100 105 110
   Thr Val Ser Ser
   115
<210> 113
   <211> 116
   <212> PRT
   <213> Primate
<400> 113
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Val His Trp Val Arg Gln Ala Pro Gly Gln Gly Leu Glu Trp Met
   35 40 45
   Gly Tyr Ile Tyr Pro Tyr Asp Gly Asp Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Ala Phe Glu Tyr Trp Gly Gln Gly Thr Leu Val
   100 105 110
   Thr Val Ser Ser
   115
<210> 114
   <211> 116
   <212> PRT
   <213> Primate
<400> 114
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Met Ala Trp Val Arg Gln Ala Pro Gly Gln Gly Leu Glu Trp Met
           35                  40                  45
   Gly Tyr Ile Tyr Pro Tyr Asp Gly Asp Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Ala Phe Glu Tyr Trp Gly Gln Gly Thr Leu Val
               100                 105                 110
   Thr Val Ser Ser
           115
<210> 115
   <211> 116
   <212> PRT
   <213> Primate
<400> 115
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Met His Trp Val Arg Gln Ala Pro Gly Gln Gly Leu Glu Trp Met
           35                  40                  45
   Gly Tyr Ile Tyr Pro Tyr Asp Gly Asp Thr Gly Val Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Ala Phe Glu Tyr Trp Gly Gln Gly Thr Leu Val
               100                 105                 110
   Thr Val Ser Ser
           115
<210> 116
   <211> 116
   <212> PRT
   <213> Primate
<400> 116
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Ile His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Leu Glu Tyr Trp Gly Gln Gly Thr Leu Val
               100                 105                 110
   Thr Val Ser Ser
           115
<210> 117
   <211> 116
   <212> PRT
   <213> Primate
<400> 117
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Ile His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 118
   <211> 116
   <212> PRT
   <213> Primate
<400> 118
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Leu Ala Tyr Trp Gly Gln Gly Thr Leu Val
   100 105 110
   Thr Val Ser Ser
   115
<210> 119
   <211> 116
   <212> PRT
   <213> Primate
<400> 119
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Ile His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Leu Ala Tyr Trp Gly Gln Gly Thr Leu Val
               100                 105                 110
   Thr Val Ser Ser
           115
<210> 120
   <211> 116
   <212> PRT
   <213> Primate
<400> 120
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Met His Trp Val Arg Gln Ala Pro Gly Gln Gly Leu Glu Trp Met
           35                  40                  45
   Gly Tyr Ile Tyr Pro Tyr Asp Gly Asp Thr Gly Tyr Asn Ser Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Phe Ala Tyr Trp Gly Gln Gly Thr Leu Val
               100                 105                 110
   Thr Val Ser Ser
           115
<210> 121
   <211> 116
   <212> PRT
   <213> Primate
<400> 121
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Asp Thr Gly Tyr Asn Gln Lys Tyr
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 122
   <211> 116
   <212> PRT
   <213> Primate
<400> 122
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Asp Thr Gly Ser Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser
   115
<210> 123
   <211> 116
   <212> PRT
   <213> Primate
<400> 123
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Asp Phe Glu Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Leu Ala Tyr Trp Gly Gln Gly Thr Leu Val
   100 105 110
   Thr Val Ser Ser
   115
<210> 124
   <211> 116
   <212> PRT
   <213> Primate
<400> 124
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Glu Asp Tyr
               20                  25                  30
   Asn Met His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Gln Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Phe Ala Tyr Trp Gly Gln Gly Thr Leu Val
               100                 105                 110
   Thr Val Ser Ser
           115
<210> 125
   <211> 116
   <212> PRT
   <213> Primate
<400> 125
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Asn Ile His Trp Val Arg Gln Ala Pro Gly Gln Gly Leu Glu Trp Met
           35                  40                  45
   Gly Tyr Ile Tyr Pro Tyr Asp Gly Glu Thr Gly Tyr Asn Pro Lys Phe
       50                  55                  60
   Lys Ser Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Arg Trp Phe Ala Tyr Trp Gly Gln Gly Thr Leu Val
   100 105 110
   Thr Val Ser Ser
   115
<210> 126
   <211> 9
   <212> PRT
   <213> Primate
<400> 126
   Gln Gln Trp Asn Gly Asn Pro Pro Ala
   1 5
<210> 127
   <211> 9
   <212> PRT
   <213> Primate
<400> 127
   Gln Gln Trp Asp Ser Asn Pro Pro Ala
   1 5
<210> 128
   <211> 17
   <212> PRT
   <213> Primate
<400> 128
   Tyr Ile Tyr Pro Tyr Asn Gly Asp Thr Gly Tyr Asn Gln Lys Phe Lys
   1 5 10 15
   Ser
<210> 129
   <211> 10
   <212> PRT
   <213> Primate
<400> 129
   Gly Tyr Thr Phe Thr Asp Tyr Thr Met His
   1 5 10
<210> 130
   <211> 213
   <212> PRT
   <213> primate
<400> 130
   Asp Ile Gln Met Thr Gln Ser Pro Ser Ser Leu Ser Ala Ser Val Gly
   1               5                   10                  15
   Asp Arg Val Thr Ile Thr Cys Glu Ala Ser Ser Ser Val Ser Tyr Met
               20                  25                  30
   His Trp Tyr Gln Gln Lys Pro Gly Lys Ala Pro Lys Pro Leu Ile Tyr
           35                  40                  45
   Ala Thr Ser Asn Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Tyr Cys Gln Gln Trp Asp Leu Asn Pro Pro Ala
                   85                  90                  95
   Phe Gly Gln Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 131
   <211> 442
   <212> PRT
   <213> primate
<400> 131
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Met His Trp Val Arg Gln Ala Pro Gly Gln Gly Leu Glu Trp Met
   35 40 45
   Gly Tyr Ile Tyr Pro Tyr Asp Gly Asp Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser Ala Ser Thr Lys Gly Pro Ser Val Phe Pro Leu Ala
   115 120 125
   Pro Cys Ser Arg Ser Thr Ser Glu Ser Thr Ala Ala Leu Gly Cys Leu
   130 135 140
   Val Lys Asp Tyr Phe Pro Glu Pro Val Thr Val Ser Trp Asn Ser Gly
   145 150 155 160
   Ala Leu Thr Ser Gly Val His Thr Phe Pro Ala Val Leu Gln Ser Ser
   165 170 175
   Gly Leu Tyr Ser Leu Ser Ser Val Val Thr Val Pro Ser Ser Ser Leu
   180 185 190
   Gly Thr Lys Thr Tyr Thr Cys Asn Val Asp His Lys Pro Ser Asn Thr
   195 200 205
   Lys Val Asp Lys Arg Val Glu Ser Lys Tyr Gly Pro Pro Cys Pro Pro
   210 215 220
   Cys Pro Ala Pro Glu Phe Leu Gly Gly Pro Ser Val Phe Leu Phe Pro
   225 230 235 240
   Pro Lys Pro Lys Asp Thr Leu Met Ile Ser Arg Thr Pro Glu Val Thr
   245 250 255
   Cys Val Val Val Asp Val Ser Gln Glu Asp Pro Glu Val Gln Phe Asn
   260 265 270
   Trp Tyr Val Asp Gly Val Glu Val His Asn Ala Lys Thr Lys Pro Arg
   275 280 285
   Glu Glu Gln Phe Asn Ser Thr Tyr Arg Val Val Ser Val Leu Thr Val
   290 295 300
   Leu His Gln Asp Trp Leu Asn Gly Lys Glu Tyr Lys Cys Lys Val Ser
   305 310 315 320
   Asn Lys Gly Leu Pro Ser Ser Ile Glu Lys Thr Ile Ser Lys Ala Lys
   325 330 335
   Gly Gln Pro Arg Glu Pro Gln Val Tyr Thr Leu Pro Pro Ser Gln Glu
   340 345 350
   Glu Met Thr Lys Asn Gln Val Ser Leu Thr Cys Leu Val Lys Gly Phe
   355 360 365
   Tyr Pro Ser Asp Ile Ala Val Glu Trp Glu Ser Asn Gly Gln Pro Glu
   370 375 380
   Asn Asn Tyr Lys Thr Thr Pro Pro Val Leu Asp Ser Asp Gly Ser Phe
   385 390 395 400
   Phe Leu Tyr Ser Arg Leu Thr Val Asp Lys Ser Arg Trp Gln Glu Gly
   405 410 415
   Asn Val Phe Ser Cys Ser Val Met His Glu Ala Leu His Asn His Tyr
   420 425 430
   Thr Gln Lys Ser Leu Ser Leu Ser Leu Gly
   435 440
<210> 132
   <211> 213
   <212> PRT
   <213> primate
<400> 132
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Pro Tyr Met
   20 25 30
   His Trp Phe Gln Gln Lys Pro Gly Lys Ala Pro Lys Ser Leu Ile Tyr
   35 40 45
   Ala Thr Ser Asn Pro Ala Tyr Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
   85 90 95
   Phe Gly Gln Gly Thr Lys Leu Glu Ile Lys Arg Thr Val Ala Ala Pro
   100 105 110
   Ser Val Phe Ile Phe Pro Pro Ser Asp Glu Gln Leu Lys Ser Gly Thr
   115 120 125
   Ala Ser Val Val Cys Leu Leu Asn Asn Phe Tyr Pro Arg Glu Ala Lys
   130 135 140
   Val Gln Trp Lys Val Asp Asn Ala Leu Gln Ser Gly Asn Ser Gln Glu
   145 150 155 160
   Ser Val Thr Glu Gln Asp Ser Lys Asp Ser Thr Tyr Ser Leu Ser Ser
   165 170 175
   Thr Leu Thr Leu Ser Lys Ala Asp Tyr Glu Lys His Lys Val Tyr Ala
   180 185 190
   Cys Glu Val Thr His Gln Gly Leu Ser Ser Pro Val Thr Lys Ser Phe
   195 200 205
   Asn Arg Gly Glu Cys
   210
<210> 133
   <211> 442
   <212> PRT
   <213> primate
<400> 133
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Asn Ile His Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Phe Ala Tyr Trp Gly Gln Gly Thr Leu Val
   100 105 110
   Thr Val Ser Ser Ala Ser Thr Lys Gly Pro Ser Val Phe Pro Leu Ala
   115 120 125
   Pro Cys Ser Arg Ser Thr Ser Glu Ser Thr Ala Ala Leu Gly Cys Leu
   130 135 140
   Val Lys Asp Tyr Phe Pro Glu Pro Val Thr Val Ser Trp Asn Ser Gly
   145 150 155 160
   Ala Leu Thr Ser Gly Val His Thr Phe Pro Ala Val Leu Gln Ser Ser
   165 170 175
   Gly Leu Tyr Ser Leu Ser Ser Val Val Thr Val Pro Ser Ser Ser Leu
   180 185 190
   Gly Thr Lys Thr Tyr Thr Cys Asn Val Asp His Lys Pro Ser Asn Thr
   195 200 205
   Lys Val Asp Lys Arg Val Glu Ser Lys Tyr Gly Pro Pro Cys Pro Pro
   210 215 220
   Cys Pro Ala Pro Glu Phe Leu Gly Gly Pro Ser Val Phe Leu Phe Pro
   225 230 235 240
   Pro Lys Pro Lys Asp Thr Leu Met Ile Ser Arg Thr Pro Glu Val Thr
   245 250 255
   Cys Val Val Val Asp Val Ser Gln Glu Asp Pro Glu Val Gln Phe Asn
   260 265 270
   Trp Tyr Val Asp Gly Val Glu Val His Asn Ala Lys Thr Lys Pro Arg
   275 280 285
   Glu Glu Gln Phe Asn Ser Thr Tyr Arg Val Val Ser Val Leu Thr Val
   290 295 300
   Leu His Gln Asp Trp Leu Asn Gly Lys Glu Tyr Lys Cys Lys Val Ser
   305 310 315 320
   Asn Lys Gly Leu Pro Ser Ser Ile Glu Lys Thr Ile Ser Lys Ala Lys
   325 330 335
   Gly Gln Pro Arg Glu Pro Gln Val Tyr Thr Leu Pro Pro Ser Gln Glu
   340 345 350
   Glu Met Thr Lys Asn Gln Val Ser Leu Thr Cys Leu Val Lys Gly Phe
   355 360 365
   Tyr Pro Ser Asp Ile Ala Val Glu Trp Glu Ser Asn Gly Gln Pro Glu
   370 375 380
   Asn Asn Tyr Lys Thr Thr Pro Pro Val Leu Asp Ser Asp Gly Ser Phe
   385 390 395 400
   Phe Leu Tyr Ser Arg Leu Thr Val Asp Lys Ser Arg Trp Gln Glu Gly
   405 410 415
   Asn Val Phe Ser Cys Ser Val Met His Glu Ala Leu His Asn His Tyr
   420 425 430
   Thr Gln Lys Ser Leu Ser Leu Ser Leu Gly
   435 440
<210> 134
   <211> 442
   <212> PRT
   <213> primate
<400> 134
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Asp Phe Thr Asp Tyr
   20 25 30
   Asn Ile His Trp Val Arg Gln Ala Pro Gly Gln Gly Leu Glu Trp Met
   35 40 45
   Gly Tyr Ile Tyr Pro Tyr Asp Gly Glu Thr Gly Tyr Asn Gln Lys Phe
   50 55 60
   Lys Ser Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Tyr Arg Trp Leu Ala Tyr Trp Gly Gln Gly Thr Leu Val
   100 105 110
   Thr Val Ser Ser Ala Ser Thr Lys Gly Pro Ser Val Phe Pro Leu Ala
   115 120 125
   Pro Cys Ser Arg Ser Thr Ser Glu Ser Thr Ala Ala Leu Gly Cys Leu
   130 135 140
   Val Lys Asp Tyr Phe Pro Glu Pro Val Thr Val Ser Trp Asn Ser Gly
   145 150 155 160
   Ala Leu Thr Ser Gly Val His Thr Phe Pro Ala Val Leu Gln Ser Ser
   165 170 175
   Gly Leu Tyr Ser Leu Ser Ser Val Val Thr Val Pro Ser Ser Ser Leu
   180 185 190
   Gly Thr Lys Thr Tyr Thr Cys Asn Val Asp His Lys Pro Ser Asn Thr
   195 200 205
   Lys Val Asp Lys Arg Val Glu Ser Lys Tyr Gly Pro Pro Cys Pro Pro
   210 215 220
   Cys Pro Ala Pro Glu Phe Leu Gly Gly Pro Ser Val Phe Leu Phe Pro
   225 230 235 240
   Pro Lys Pro Lys Asp Thr Leu Met Ile Ser Arg Thr Pro Glu Val Thr
   245 250 255
   Cys Val Val Val Asp Val Ser Gln Glu Asp Pro Glu Val Gln Phe Asn
   260 265 270
   Trp Tyr Val Asp Gly Val Glu Val His Asn Ala Lys Thr Lys Pro Arg
   275 280 285
   Glu Glu Gln Phe Asn Ser Thr Tyr Arg Val Val Ser Val Leu Thr Val
   290 295 300
   Leu His Gln Asp Trp Leu Asn Gly Lys Glu Tyr Lys Cys Lys Val Ser
   305 310 315 320
   Asn Lys Gly Leu Pro Ser Ser Ile Glu Lys Thr Ile Ser Lys Ala Lys
   325 330 335
   Gly Gln Pro Arg Glu Pro Gln Val Tyr Thr Leu Pro Pro Ser Gln Glu
   340 345 350
   Glu Met Thr Lys Asn Gln Val Ser Leu Thr Cys Leu Val Lys Gly Phe
   355 360 365
   Tyr Pro Ser Asp Ile Ala Val Glu Trp Glu Ser Asn Gly Gln Pro Glu
   370 375 380
   Asn Asn Tyr Lys Thr Thr Pro Pro Val Leu Asp Ser Asp Gly Ser Phe
   385 390 395 400
   Phe Leu Tyr Ser Arg Leu Thr Val Asp Lys Ser Arg Trp Gln Glu Gly
   405 410 415
   Asn Val Phe Ser Cys Ser Val Met His Glu Ala Leu His Asn His Tyr
   420 425 430
   Thr Gln Lys Ser Leu Ser Leu Ser Leu Gly
   435 440
<210> 135
   <211> 213
   <212> PRT
   <213> primate
<400> 135
   Asp Ile Gln Met Thr Gln Ser Pro Ser Ser Leu Ser Ala Ser Val Gly
   1 5 10 15
   Asp Arg Val Thr Ile Thr Cys Arg Ala Ser Ser Ser Val Ser Tyr Met
   20 25 30
   His Trp Tyr Gln Gln Lys Pro Gly Lys Ala Pro Lys Pro Leu Ile Tyr
   35 40 45
   Ala Thr Ser Asn Leu Ala Tyr Gly Val Pro Ser Arg Phe Ser Gly Ser
   50 55 60
   Gly Ser Gly Thr Asp Phe Thr Leu Thr Ile Ser Ser Leu Gln Pro Glu
   65 70 75 80
   Asp Phe Ala Thr Tyr Tyr Cys Gln Gln Trp Asp Leu Asn Pro Pro Ala
                       85                  90                  95
   Phe Gly Gln Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210